(19) Europäisches Patentamt
European Patent Office
Office européen des brevets

(11) EP 1 087 973 B1

(12) **EUROPÄISCHE PATENTSCHRIFT**

(45) Veröffentlichungstag und Bekanntmachung des Hinweises auf die Patenterteilung:
**08.01.2003 Patentblatt 2003/02**

(21) Anmeldenummer: **99927950.8**

(22) Anmeldetag: **11.06.1999**

(51) Int Cl.[7]: **C07D 487/14**, A61K 31/55 // (C07D487/14, 249:00, 239:00), C07D235:00

(86) Internationale Anmeldenummer:
**PCT/EP99/04017**

(87) Internationale Veröffentlichungsnummer:
**WO 99/065912 (23.12.1999 Gazette 1999/51)**

(54) **IMIDAZOTRIAZOLOPYRIMIDINE ALS ARZNEIMITTEL MIT ADENOSINANTAGONISTISCHER WIRKUNG**

IMIDAZOTRIAZOLOPYRIMIDINES USED AS A DRUG HAVING AN ADENOSINE ANTAGONIST ACTIVITY

IMIDAZOTRIAZOLOPYRIMIDINES UTILISEES COMME MEDICAMENT A ACTION ANTAGONISTE DE L'ADENOSINE

(84) Benannte Vertragsstaaten:
**AT BE CH CY DE DK ES FI FR GB GR IE IT LI LU MC NL PT SE**

(30) Priorität: **16.06.1998 DE 19826843**

(43) Veröffentlichungstag der Anmeldung:
**04.04.2001 Patentblatt 2001/14**

(73) Patentinhaber: **Boehringer Ingelheim Pharma KG**
**55218 Ingelheim am Rhein (DE)**

(72) Erfinder:
- **BLECH, Stefan**
  **D-55218 Ingelheim (DE)**
- **CARTER, Adrian**
  **D-55411 Bingen (DE)**
- **GAIDA, Wolfram**
  **D-55218 Ingelheim (DE)**
- **HOFFMANN, Matthias**
  **D-55218 Ingelheim (DE)**
- **KUEFNER-MUEHL, Ulrike**
  **D-55218 Ingelheim (DE)**
- **MEADE, Christopher, John, Montague**
  **D-55411 Bingen (DE)**
- **POHL, Gerald**
  **D-55435 Gau-Algesheim (DE)**

(74) Vertreter: **Laudien, Dieter, Dr.**
**Boehringer Ingelheim GmbH,**
**B Patent**
**55216 Ingelheim am Rhein (DE)**

(56) Entgegenhaltungen:
**DE-A- 19 629 378**

- **E. TENOR ET AL.: "Synthese und Reaktivität von 7-Amino-s-triazolo(1.5-a) pyrimidonen-(5)" CHEMISCHE BERICHTE., Bd. 97, 1964, Seite 1373-1383 XP002115619 VERLAG CHEMIE GMBH. WEINHEIM., DE ISSN: 0009-2940 in der Anmeldung erwähnt**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 087 973 B1

Printed by Jouve, 75001 PARIS (FR)

## Beschreibung

Die Erfindung betrifft neue Imidazotriazolopyrimidin-Derivate, Verfahren zu ihrer Herstellung und ihre Verwendung als Arzneimittel.

Die neuen Imidazotriazolopyrimidinderivate besitzen die Struktur der allgemeinen Formel (I):

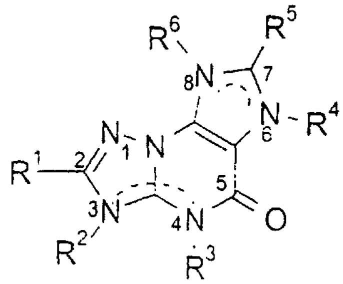

(I)

worin die gestrichelten Linien zwischen den Stickstoffatomen der zuvor genannten allgemeinen Formel I die Existenz einer Doppelbindung in einer von zwei möglichen Positionen beschreiben, sodaß die Reste $R^4$ und $R^6$ beziehungsweise $R^3$ und $R^2$ nicht gleichzeitig vorhanden sein können und worin die Reste $R^1$ bis $R^6$ die in der nachfolgenden Beschreibung genannten Bedeutungen aufweisen können.

Stand der Technik

Die ersten und einzigen beiden Vertreter der Klasse der Imidazotriazolopyrimidin-Derivate gemäß der allgemeinen Formel (I) sind im Stand der Technik seit Mitte der sechziger Jahre bekannt (E. Tenor und C.-F. Kröger, *Chem. Ber.* Vol. 97 (1964) S. 1373-1382). Sie weisen an Position 7 der allgemeinen Formel (I) einen Methylsubstituenten und an Position 2 einen Ethyl- oder n-Propyl-Rest auf.

Hintergrund der Erfindung

Überraschenderweise wurde gefunden, daß Imidazotriazolopyrimidine der allgemeinen Formel (I) eine Affinität zu Adenosin-Rezeptoren aufweisen und somit eine neue Klasse von Adenosin-Antagonisten darstellen.

Adenosin-Antagonisten können in den Fällen eine therapeutisch nutzbare Wirkung entfalten, in denen Krankheiten oder pathologische Situationen mit einer Aktivierung von Adenosin-Rezeptoren verbunden sind.

Adenosin ist ein enaogener Modulator mit überwiegend hemmenden (inhibitorischen) Wirkungen im ZNS, im Herzen, in den Nieren und anderen Organen. Die Effekte von Adenosin werden über mindestens drei Rezeptor-Subtypen vermittelt: Adenosin $A_1$-, $A_2$- und $A_3$- Rezeptoren. Adenosin $A_2$ Rezeptoren sind weiter in zwei Subtypen geteilt, $A_{2a}$ und $A_{2b}$. Die zwei $A_2$ Rezeptor-Subtypen können differenziert werden, z.B. weil bestimmte Adenosin Agonisten wie CGS 21680 überwiegend nur den $A_{2a}$ Subtyp stimulieren. Es wird vermutet, daß der $A_{2b}$ Subtyp eine relativ niedrige Affinität für Adenosin hat. Darum sind relativ hohe Konzentrationen Adenosin notwendig um diesen Subtyp zu stimulieren. Solche hohe Konzentrationen vermute man z.B. in der Epitheloberflächenflüssigkeit der Lunge von Asthmatikern oder bei ischaemischen Gewebeschäden.

Im ZNS entfaltet Adenosin inhibitorische Wirkungen vorwiegend über die Aktivierung von $A_1$-Rezeptoren: praesynaptisch durch Hemmung der synaptischen Übertragung (Hemmung der Freisetzung von Neurotransmittern wie Acetylcholin, Dopamin, Noradrenalin, Serotonin, Glutamat u.a.), postsynaptisch durch Hemmung der neuronalen Aktivität.

$A_1$-Antagonisten heben die inhibitorischen Wirkungen von Adenosin auf und fördern die neuronale Transmission und die neuronale Aktivität.

$A_1$ Antagonisten sind deshalb von großem Interesse für die Therapie zentralnervöser degenerativer Erkrankungen wie senile Demenz vom Morbus Alzheimer Typ und altersassoziierte Störungen der Gedächtnis- und Lernleistungen.

Die Krankheit umfaßt neben der Vergeßlichkeit in der milden Form und der völligen Hilflosigkeit und absoluten Pflegebedürftigkeit bei der schwersten Form eine Reihe anderer Begleitsymptome wie Schlafstörungen, Moto-Koordinationsstörungen bis zum Bild eines Morbus Parkinson, ferner eine erhöhte Affektlabilität sowie auch depressive Symptome. Die Krankheit ist progredient und kann zum Tode führen. Die bisherige Therapie ist unbefriedigend. Spezifische Therapeutika fehlen bis jetzt vollständig. Therapieversuche mit Acetylchoünesterase-Inhibitoren zeigen nur

bei einem geringen Teil der Patienten eine Wirkung, sind jedoch mit einer hohen Nebenwirkungsrate verbunden.

Die Pathophysiologie des M. Alzheimer und SDAT ist charakterisiert durch eine schwere Beeinträchtigung des cholinergen Systems, jedoch sind auch andere Transmittersysteme betroffen. Durch den Verlust praesynaptischer cholinerger und anderer Neurone und der daraus resultierenden mangelnden Bereitstellung von Neurotransmittern ist die neuronale Übertragung und die neuronale Aktivität in den für Lernen und Gedächtnis essentiellen Hirnarealen empfindlich vermindert.

Selektive Adenosin $A_1$-Rezeptor Antagonisten fördern die neuronale Transmission durch vermehrte Bereitstellung von Neurotransmittern, erhöhen die Erregbarkeit postsynaptischer Neurone und können damit der Krankheit symptomatisch entgegenwirken.

Die hohe Rezeptoraffinität und -Selektivität einiger der beanspruchten Verbindungen sollte es erlauben, M. Alzheimer und SDAT mit niedrigen Dosen zu therapieren, so daß kaum mit Nebenwirkungen zu rechnen ist, die nicht auf die Blockade von $A_1$-Rezeptoren zurückzuführen sind.

Eine weitere Indikation für zentralwirksame Adenosin-$A_1$-Antagonisten ist die Depression. Der Therapieerfolg antidepressiver Substanzen scheint mit einer Aufregulation von $A_1$-Rezeptoren verbunden zu sein. $A_1$-Antagonisten können zur Aufregulierung von Adenosin-$A_1$-Rezeptoren führen und somit einen neuen Therapieansatz zur Behandlung von depressiven Patienten bieten.

Weitere Einsatzgebiete insbesondere für $A_2$-selektive Adenosinantagonisten sind neurodegenerative Erkrankungen wie Morbus Parkinson und darüber hinaus die Migräne. Adenosin hemmt die Freisetzung von Dopamin aus zentralen synaptischen Endigungen durch Interaktionen mit Dopamin-$D_2$-Rezeptoren. $A_2$ Antagonisten steigern die Freisetzung und die Verfügbarkeit von Dopamin und bieten damit ein neues therapeutisches Prinzip zur Behandlung des M. Parkinson.

Bei der Migräne scheint eine über $A_2$-Rezeptoren mediierte Vasodilatation cerebraler Gefäße mitbeteiligt zu sein. Selektive $A_2$-Antagonisten hemmen die Vasodilatation und können somit nützlich zur Behandlung der Migräne sein.

Auch zur Therapie peripherer Indikationen können Adenosinantagonisten Verwendung finden.

Beispielsweise kann die Aktivierung von $A_1$-, $A_{2b}$ oder $A_3$ Rezeptoren in der Lunge zu einer Bronchokonstriktion führen. Selektive Adenosin $A_1$- Antagonisten relaxieren die tracheale glatte Muskulatur, bewirken eine Bronchodilatation und können dadurch als Antiasthmamittel nützlich sein.

Adenosin $A_{2b}$ oder $A_3$ Rezeptoren finden sich auf Mastzellen. Deren Aktivierung verursacht die Freisetzung von Mastzellprodukten wie Histamin, Tryptase oder Interleukin 8. Adenosin $A_3$ Rezeptoren finden sich auf Eosinophilen und die Stimulierung dieser Rezeptoren kann die Aktivierung, Chemotaxis und Apoptose von Eosinophilen beeinflussen. Deshalb sind Antagonisten der $A_{2b}$ oder $A_3$ Rezeptoren vielversprechend für die Behandlung von allergischen Krankheiten wie z.B. Rhinitis, Urticaria, Pruritis, allergische Dermatitis, allergische Augenkrankheiten und nasale Polypen. Zusätzlich kann die Wirkung von Adenosin $A_{2b}$ und $A_3$ Antagonisten auf Mastzellen und Eosinophilen auch bei der Asthmatherapie hilfreich sein. Weiter kann die anti-Mastzell Wirkung nützlich sein für die Verminderung von Reperfusionsschäden nach Herzischaemie.

Über die Aktivierung von $A_2$-Rezeptoren kann Adenosin unter anderem eine respiratorische Depression und Atemstillstand hervorrufen. $A_2$-Antagonisten bewirken eine respiratorische Stimulation. Beispielsweise werden Adenosin-Antagonisten (Theophyllin) zur Behandlung der Atemnot und zur Vorbeugung des "plötzlichen Kindstodes" bei Frühgeburten eingesetzt.

Adenosin stimuliert die Produktion von Mucus durch Epitheizellen. Die Aktivierung von Adenosin $A_{2b}$ Rezeptoren auf bronchiale Epithelzellen stimuliert den Chlorid-Transport, der die Konsistenz von Mucus beeinflußt. Deshalb bieten Adenosin Antagonisten neue Therapieansätze für die Behandlung von Krankheiten bei denen die Menge oder Konsistenz des Mucus pathologisch ist wie z.B. bei Bronchitis und chronisch obstruktiv pulmonaren Krankheiten.

Adenosin $A_{2b}$ Rezeptoren finden sich auch auf den Epithelzellen des Darms. Auch bei den Darmzellen kann die Aktivierung dieser Rezeptoren zu erhöhtem Chlorid-Transport führen. Es wird vermutet, daß während Darmentzündungen Adenosin z.B. von Neutrophilen freigesetzt wird. Der Effekt des freigesetzten Adenosins auf den Chlorid-Transport beeinflußt die Motilität und Absorptionsfähigkeit des Darmepithels. Deshalb sind Adenosin Antagonisten mögliche Therapiemittel für entzündliche Darmkrankheiten und Diaorrhea.

Wichtige Therapiefelder für Adenosin-Antagonisten sind ferner kardiovaskuläre Erkrankungen und Nierenerkrankungen.

Am Herzen entfaltet Adenosin über die Aktivierung von $A_1$-Rezeptoren eine Hemmung der elektrischen und kontraktilen Aktivität. Verbunden mit einer über $A_2$-Rezeptoren mediierten koronaren Vasodilatation wirkt Adenosin negativ chronotrop,-inotrop,-dromotrop, -bathmotrop, bradykard und erniedrigt das Herzminutenvolumen.

Adenosin $A_1$-Rezeptor-Antagonisten oder Adenosin $A_3$ Rezeptor-Antagonisten vermögen durch Ischämie und nachfolgende Reperfusion bedingte Schädigungen am Herzen und am Gehirn oder der Lunge zu verhindern. Deshalb könnten Adenosinantagonisten zur Prävention oder frühen Behandlung von Ischämie-Reperfusions bedingten Schädigungen des Herzens z.B. nach coronar Bypass-Chirurgie, Herztransplantation, Angioplastie oder thrombolytischer Therapie des Herzens und ähnlicher Eingriffe eingesetzt werden. Ferner können Adenosinantagonisten zur frühen Behandlung von zerebraler Ischaemie eingesetzt werden. Entsprechendes gilt für die Lunge.

An den Nieren bewirkt die Aktivierung von $A_1$-Rezeptoren eine Vasokonstriktion afferenter Arteriolen und dadurch bedingt einen Abfall des renalen Blutflusses und der glomerulären Filtration.

$A_1$ Antagonisten wirken an der Niere wie starke kaliumsparende Diuretika und können somit zur Nierenprotektion sowie zur Behandlung von Oedemen, Niereninsuffizienz und akutem Nierenversagen eingesetzt werden.

Aufgrund des Adenosin-Antagonismus am Herzen und der diuretischen Wirkung können $A_1$-Antagonisten bei verschiedenen kardiovaskulären Erkrankungen therapeutisch wirksam eingesetzt werden wie z.B. bei Herzinsuffizienz, Arrhytmien (Bradyarrhytmien) assoziiert mit Hypoxie oder Ischämie, Überleitungsstörungen, Hypertonie, Ascites bei Leberversagen (hepato-renales Syndrom) und als Analgetikum bei Durchblutungsstörungen.

Adenosin kann, über die Adenosin A3-Rezeptoren und abhängig vom Zelltyp und der Konzentration des Rezeptorligands, die Zellapoptose stimulieren oder hemmen. Dieser Effekt kann von Adenosin $A_3$-Rezeptor Antagonisten aufgehoben werden. Deshalb sind Adenosin $A_3$-Rezeptor Antagonisten mögliche Therapiemittel für Krankheiten bei denen eine Störung der Apoptose eine Rolle spielt, wie z.B. entzündliche Krankheiten wie Arthritis, Autoimmunkrankheiten, wie Lupus erythematodes disseminatus oder Scleroderma und Krebs. Besonders geeignet für eine Therapie mit Adenosin $A_3$-Rezeptor Antagonisten wären Krebsarten die den Adenosin Rezeptor überexprimieren. Ferner könnten Adenosin $A_3$-Rezeptor Antagonisten eingesetzt werden, um die immunologischen Schutzmechanismen gegen verbleibenge bösartige Krebszellen zu fördern (z.B. nach chirurgischer Entfernung eines Kolonkarzinom oder anderer Tumorarten).

Die zystische Fibrose - auch als Mukoviszidose bekannt - ist eine erbliche Stoffwechselstörung, hervorgerufen durch einen genetischen Defekt eines bestimmten Chromosoms. Durch eine vermehrte Produktion und erhöhte Viskosität des Sekrets der mukösen Drüsen in den Bronchien kann es zu schweren Komplikationen im Bereich der Atemwege kommen. Erste Untersuchungen haben gezeigt, daß $A_1$-Antagonisten den Efflux von Chloridionen z.B. bei CF PAC Zellen erhöhen. Ausgehend von diesen Befunden kann erwartet werden, daß bei Patienten, die an zystischer Fibrose (Mukovizidose) erkrankt sind, die erfindungsgemäßen Verbindungen den gestörten Elektrolythaushalt der Zellen regulieren und die Symptome der Erkrankung gemildert werden.

Detaillierte Beschreibung der Erfindung

Die Erfindung betrifft neue Imidazotriazolopyrimidin-Derivate, Verfahren zu ihrer Herstellung und ihre Verwendung zur Herstellung eines Arzneimittels mit adenosinantagonistischer Wirkung.

Die neuen Imidazotriazolopyrimidinderivate besitzen die Struktur der allgemeinen Formel (I):

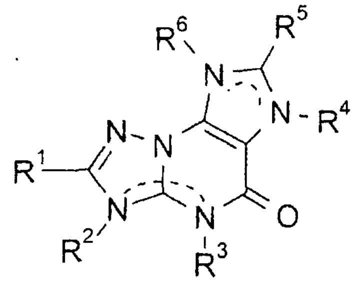

(I)

worin die gestrichelten Linien zwischen den Stickstoffatomen der zuvor genannten allgemeinen Formel I die Existenz einer Doppelbindung in einer von zwei möglichen Positionen beschreiben, sodaß die Reste $R^4$ und $R^6$ beziehungsweise $R^3$ und $R^2$ nicht gleichzeitig vorhanden sein können und

worin

$R^1$ Wasserstoff, ein $C_1$-$C_8$-Alkyl-, $C_2$-$C_8$-Alkenyl- oder $C_2$-$C_8$-Alkinyl-Rest, der gegebenenfalls ein oder mehrfach durch -CN, $-CH_2NR^7R^8$, $-COOR^9$, $-CONR^7R^8$, -CHO, $-COR^{10}$, $-CH(OH)R^{10}$, $-CH(OR^9)_2$, $-CH=CH-R^{11}$, -CH=NOH, $-CH=NOR^9$, $-NR^7R^8$, $-NHCOR^9$, $-NHCONR^7R^8$, $-NHCOOR^9$, -NHCONHPhenyl, $-OR^9$, $-OCOR^9$, -OCOPyridyl, $-OCH_2COOR^9$, $-OCH_2-CONR^7R^8$, $-OCH_2-CH_2-NR^7R^8$, $-OCH_2CH_2OR^9$, $-OCH_2-CH_2OCOR^9$, $OCONR^7R^8$, Halogen, gegebenenfalls durch Methyl-substituiertes 1,3-Dioxolan oder 1,3-Dioxan substituiert sein kann, oder

| | |
|---|---|
| $R^1$ | -CHO, $-COOR^9$, $-CONR^7R^8$ oder $NR^7R^8$, oder |
| $R^1$ | $C_3$-$C_7$-Cycloalkyl, bevorzugt Cyclopentyl oder Cyclohexyl, das gegebenenfalls durch = O, $-OR^9$, $OCOR^9$ oder -OCOPyridyl substituiert sein kann, oder |
| R' | Phenyl, das gegebenenfalls durch $C_1$-$C_4$-Alkyl, bevorzugt Methyl, -CN, $-COOR^9$, $NR^7R^8$, $-OR^9$, $-OCH_2COOR^9$, $-OCH_2CONR^7R^8$,$-SO_3H$, oder Halogen substituiert sein kann, oder |
| $R^1$ | Phenyl-$C_1$-$C_6$-alkyl-, bevorzugt Phenyl-$C_1$-$C_4$-alkyl-, Phenyl-$C_2$-$C_6$-alkenyl- oder Phenyl-$C_2$-$C_6$-alkinyl-, wobei der Phenylring gegebenenfalls entweder direkt oder über eine Alkylenbrücke mit 1 bis 4 C-Atomen substituiert sein kann durch einen oder mehrere der Reste $C_1$-$C_3$-Alkyl, -CN, $-CH_2OCOR^9$, $-COOR^9$, $-CF_3$, $-CONR^7R^8$, $-CH_2OR^9$, -CHO, $-CH=NOR^9$, $-COR^{10}$, $-CH(OH)R^{10}$, $-CH(OR^9)_2$, $-CH=CH-R^{11}$, $-CH_2$-$O-CONR^7R^8$, $-CH_2-CH_2-O-CONR^7R^8$, $-CO-R^9$, -CO-$C_1$-$C_4$-Alkyl-$NR^7R^8$, $NR^7R^8$, $-NO_2$, $-NHCOR^9$, $-NHCONR^7R^8$, $-NHCOOR^9$, $-OR^9$, $-OCOR^9$, -OCOPyridyl, $-OCH_2COOR^9$, $-OCH_2-CONR^7R^8$, $-OCH_2-CH_2-NR^7R^8$, $-OCH_2CH_2OR^9$, $-OCH_2-CH_2OCOR^9$, $-OCONR^7R^8$, Halogen, gegebenenfalls durch Methyl substituiertes 1,3-Dioxolan oder 1,3-Dioxan, oder |
| $R^1$ | $C_3$-$C_7$-Cycloalkyl-$C_1$-$C_6$-alkyl-, $C_3$-$C_7$-Cycloalkyl-$C_2$-$C_6$-alkenyl-, $C_3$-$C_7$-Cycloalkyl-$C_2$-$C_6$-alkinyl-, wobei der Cycloalkylrest gegebenenfalls entweder direkt oder über eine Alkylenbrücke mit 1 bis 4 C-Atomen substituiert sein kann durch einen oder mehrere der Reste $C_1$-$C_3$-Alkyl, -CN, $-CH_2OCOR^9$, $-COOR^9$, $-CF_3$, $-CONR^7R^8$, $-CH_2OR^9$, -CHO, $-CH=NOR^9$, $-COR^{10}$, $-CH(OH)R^{10}$, $-CH(OR^9)_2$, $-CH=CH-R^{11}$, $-CH_2$-$O-CONR^7R^8$, $-CH_2-CH_2-O-CONR^7R^8$, $-CO-R^9$, -CO-$C_1$-$C_4$-Alkyl-$NR^7R^8$, $NR^7R^8$, $-NO_2$, $-NHCOR^9$, $-NHCONR^7R^8$, $-NHCOOR^9$, $-OR^9$, $-OCOR^9$, -OCOPyridyl, $-OCH_2COOR^9$, $-OCH_2-CONR^7R^8$, $-OCH_2-CH_2-NR^7R^8$, $-OCH_2CH_2OR^9$, $-OCH_2-CH_2OCOR^9$, $-OCONR^7R^8$, Halogen, gegebenenfalls durch Methyl substituiertes 1,3-Dioxolan oder 1,3-Dioxan, oder |
| $R^1$ | ein Norbornan-, Norbornen-, $C_3$-$C_6$-Dicycloalkyl-methyl-, bevorzugt Dicyclopropylmethyl-, Adamantan- oder Noradamantan-Rest, der gegebenenfalls durch $C_1$-$C_4$-Alkyl, bevorzugt Methyl substituiert sein kann, oder |
| $R^1$ | einen Rest der Formel A-$C_1$-$C_6$-Alkyl-, A-CONH-$C_1$-$C_6$-Alkyl-, A-CONH-$C_2$-$C_6$-Alkenyl-, A-CONH-$C_2$-$C_6$-Alkinyl-, A-NH-CO-$C_1$-$C_6$-Alkyl-, A-NH-CO-$C_2$-$C_6$-Alkenyl-, A-NH-CO-$C_2$-$C_6$-Alkinyl-, A-$C_2$-$C_6$-Alkenyl-, A-$C_2$-$C_6$-Alkinyl oder A-, wobei A ein C- oder N-verknüpfter 5, 6 oder 7-gliedriger Heterocyclus ist, der ein oder mehrere Heteroatome aus der Gruppe Stickstoff, Sauerstoff oder Schwefel enthält und gegebenenfalls ein oder mehrfach, bevorzugt einfach, durch Benzyl, gegebenenfalls Methoxy-substituiertes Benzyl, $C_1$-$C_4$-Alkyl, -CN, $-CH_2NR^7R^8$, $-COOR^9$, $-CONR^7R^8$, $-COR^{10}$, $-NO_2$, $-NH_2$, $-OR^9$, =O, ein Ketal, Ethylenketal, $-SO_3H$, $-SO_2-R^9$ oder Halogen, substituiert sein kann; |
| $R^2$ oder $R^3$ | ein $C_1$-$C_8$-Alkyl-, $C_2$-$C_8$-Alkenyl- oder $C_2$-$C_8$-Alkinyl-Rest, der gegebenenfalls ein oder mehrfach durch -CN, $-CH_2NR^7R^8$, $-COOR^9$, $-CONR^7R^8$, -CHO, $-COR^{10}$, $-CH(OH)R^{10}$, $-CH(OR^9)_2$, $-CH=CH-R^{11}$, -CH=NOH, $-CH=NOR^9$, $-NR^7R^8$, $-NHCOR^9$, $-NHCONR^7R^8$, $-NHCOOR^9$, -NHCONHPhenyl, $-OR^9$, $-OCOR^9$, -OCOPyridyl, $-OCH_2COOR^9$, $-OCH_2-CONR^7R^8$, $-OCH_2-CH_2-NR^7R^8$, $-OCH_2CH_2OR^9$, $-OCH_2-CH_2OCOR^9$, $OCONR^7R^8$, Halogen, gegebenenfalls durch Methyl-substituiertes 1,3-Dioxolan oder 1,3-Dioxan substituiert sein kann, oder |
| $R^2$ oder $R^3$ | Phenyl-$C_1$-$C_6$-alkyl-, bevorzugt Phenyl-$C_1$-$C_4$-alkyl-, Phenyl-$C_2$-$C_6$-alkenyl- oder Phenyl-$C_2$-$C_6$-alkinyl-, wobei der Phenylring gegebenenfalls entweder direkt oder über eine Alkylenbrücke mit 1 bis 4 C-Atomen substituiert sein kann durch einen oder mehrere der Reste $C_1$-$C_3$-Alkyl, -CN, $-CH_2OCOR^9$, $-COOR^9$, $-CF_3$, $-CONR^7R^8$, $-CH_2OR^9$, -CHO, $-CH=NOR^9$, $-COR^{10}$, $-CH(OH)R^{10}$, $-CH(OR^9)_2$, $-CH=CH-R^{11}$, $-CH_2$-$O-CONR^7R^8$, $-CH_2-CH_2-O-CONR^7R^8$, $-CO-R^9$, -CO-$C_1$-$C_4$-Alkyl-$NR^7R^8$, $NR^7R^8$, $-NO_2$, $-NHCOR^9$, $-NHCONR^7R^8$, $-NHCOOR^9$, $-OR^9$, $-OCOR^9$, -OCOPyridyl, $-OCH_2COOR^9$, $-OCH_2-CONR^7R^8$, $-OCH_2-CH_2-NR^7R^8$, $-OCH_2CH_2OR^9$, $-OCH_2-CH_2OCOR^9$, $-OCONR^7R^8$, Halogen, gegebenenfalls durch Methyl substituiertes 1,3-Dioxolan oder 1,3-Dioxan, oder |
| $R^2$ oder $R^3$ | einen Rest der Formel A-$C_1$-$C_6$-Alkyl-, A-CONH-$C_1$-$C_6$-Alkyl-, A-CONH-$C_2$-$C_6$-Alkenyl-, A-CONH-$C_2$-$C_6$-Alkinyl-, A-NH-CO-$C_1$-$C_6$-Alkyl-, A-NH-CO-$C_2$-$C_6$-Alkenyl-, A-NH-CO-$C_2$-$C_6$-Alkinyl-, A-$C_2$-$C_6$-Alkenyl-, A-$C_2$-$C_6$-Alkinyl oder A-, wobei A ein C- oder N-verknüpfter 5, 6 oder 7-gliedriger Heterocyclus ist, der ein oder mehrere Heteroatome aus der Gruppe Stickstoff, Sauerstoff oder Schwefel |

enthält und gegebenenfalls ein oder mehrfach, bevorzugt einfach, durch Benzyl, gegebenenfalls Methoxy-substituiertes Benzyl, $C_1$-$C_4$-Alkyl, -CN, -$CH_2NR^7R^8$, -$COOR^9$, -$CONR^7R^8$, -$COR^{10}$, -$NO_2$. -$NH_2$, -$OR^9$, =O, ein Ketal, Ethylenketal, -$SO_3H$, -$SO_2$-$R^9$ oder Halogen, substituiert sein kann;

$R^4$ oder $R^6$ Wasserstoff, $C_1$-$C_4$-Alkyl, das gegebenenfalls durch -$NR^7R^8$ substituiert sein kann, Benzyl, bevorzugt Wasserstoff;

$R^5$ Wasserstoff, ein $C_1$-$C_8$-Alkyl-, $C_2$-$C_8$-Alkenyl- oder $C_2$-$C_8$-Alkinyl-Rest, der gegebenenfalls ein oder mehrfach durch -CN, -$CH_2NR^7R^8$, -$COOR^9$, -$CONR^7R^8$, -CHO, -$COR^{10}$, -CH(OH)$R^{10}$, -CH$(OR^9)_2$, -CH=CH-$R^{11}$, -CH=NOH, -CH=$NOR^9$, -$NR^7R^8$, -$NHCOR^9$, -$NHCONR^7R^8$, -$NHCOOR^9$, -NHCONHPhenyl, -$OR^9$, -$OCOR^9$, -OCOPyridyl, -$OCH_2COOR^9$, -$OCH_2$-$CONR^7R^8$, -$OCH_2$-$CH_2$-$NR^7R^8$, -$OCH_2CH_2OR^9$, -$OCH_2$-$CH_2OCOR^9$, $OCONR^7R^8$, Halogen, gegebenenfalls durch Methyl-substituiertes 1,3-Dioxolan oder 1,3-Dioxan substituiert sein kann, oder

$R^5$ $C_3$ - $C_7$-Cycloalkyl, bevorzugt Cyclopentyl oder Cyclohexyl, das gegebenenfalls durch = O, -OH, -$OR^9$, $OCOR^9$ oder -OCOPyridyl substituiert sein kann, oder

$R^5$ Phenyl. das gegebenenfalls durch -OH, Halogen, -$OR^9$, $C_1$-$C_4$-Alkyl, - bevorzugt -$CH_3$-, -$NH_2$, -COOH, -$SO_3H$, -$COOR^9$, -$OCH_2COOR^9$, -CN oder -$OCH_2CONR^7R^8$ substituiert sein kann, oder

$R^5$ Phenyl-$C_1$-$C_6$-alkyl-, bevorzugt Phenyl-$C_1$-$C_4$-alkyl-, Phenyl-$C_2$-$C_6$-alkenyl- oder Phenyl-$C_2$-$C_6$-alkinyl-, wobei der Phenylring gegebenenfalls entweder direkt oder über eine Alkylenbrücke mit 1 bis 4 C-Atomen substituiert sein kann durch einen oder mehrere der Reste $C_1$-$C_3$-Alkyl, -CN, -$CH_2OCOR^9$, -$COOR^9$, -$CF_3$, -$CONR^7R^8$, -$CH_2OR^9$, -CHO, -CH=$NOR^9$, -$COR^{10}$, -CH(OH)$R^{10}$, -CH$(OR^9)_2$, -CH=CH-$R^{11}$, -$CH_2$-O-$CONR^7R^8$, -$CH_2$-$CH_2$-O-$CONR^7R^8$, -CO-$R^9$, -CO-$C_1$-$C_4$-Alkyl-$NR^7R^8$, $NR^7R^8$, -$NO_2$, -$NHCOR^9$, -$NHCONR^7R^8$, -$NHCOOR^9$, -$OR^9$, -$OCOR^9$, -OCOPyridyl, -$OCH_2COOR^9$, -$OCH_2$-$CONR^7R^8$, -$OCH_2$-$CH_2$-$NR^7R^8$, -$OCH_2CH_2OR^9$, -$OCH_2$-$CH_2OCOR^9$, -$OCONR^7R^8$, Halogen, gegebenenfalls durch Methyl substituiertes 1,3-Dioxolan oder 1,3-Dioxan, oder

$R^5$ ein Norbornan-, Norbornen-, $C_3$-$C_6$-Dicycloalkyl-methyl-, bevorzugt Dicyclopropylmethyl-, Adamantan- oder Noradamantan-Rest, der gegebenenfalls durch $C_1$-$C_4$-Alkyl, bevorzugt Methyl substituiert sein kann, oder

$R^5$ -CHO, -$COOR^9$, -$CONR^7R^8$ oder $NR^7R^8$, oder

$R^5$ $C_3$-$C_7$-Cycloalkyl-$C_1$-$C_6$-alkyl-, $C_3$-$C_7$-Cycloalkyl-$C_2$-$C_6$-alkenyl-, $C_3$-$C_7$-Cycloalkyl-$C_2$-$C_6$-alkinyl-, wobei der Cycloalkylrest gegebenenfalls entweder direkt oder über eine Alkylenbrücke mit 1 bis 4 C-Atomen substituiert sein kann durch einen oder mehrere der Reste $C_1$-$C_3$-Alkyl, -CN, -$CH_2OCOR^9$, -$COOR^9$, -$CF_3$, -$CONR^7R^8$, -$CH_2OR^9$, -CHO, -CH=$NOR^9$, -$COR^{10}$, -CH(OH)$R^{10}$, -CH$(OR^9)_2$, -CH=CH-$R^{11}$, -$CH_2$-O-$CONR^7R^8$, -$CH_2$-$CH_2$-O-$CONR^7R^8$, -CO-$R^9$, -CO-$C_1$-$C_4$-Alkyl-$NR^7R^8$, $NR^7R^8$, -$NO_2$, -$NHCOR^9$, -$NHCONR^7R^8$, -$NHCOOR^9$, -$OR^9$, -$OCOR^9$, -OCOPyridyl, -$OCH_2COOR^9$, -$OCH_2$-$CONR^7R^8$, -$OCH_2$-$CH_2$-$NR^7R^8$, -$OCH_2CH_2OR^9$, -$OCH_2$-$CH_2OCOR^9$, -$OCONR^7R^8$, Halogen, gegebenenfalls durch Methyl substituiertes 1,3-Dioxolan oder 1,3-Dioxan, oder

$R^5$ einen Rest der Formel A-$C_1$-$C_6$-Alkyl-, A-CONH-$C_1$-$C_6$-Alkyl-, A-CONH-$C_2$-$C_6$-Alkenyl-, A-CONH-$C_2$-$C_6$-Alkinyl-, A-NH-CO-$C_1$-$C_6$-Alkyl, A-NH-CO-$C_2$-$C_6$-Alkenyl, A-NH-CO-$C_2$-$C_6$-Alkinyl, A-$C_2$-$C_6$-Alkenylen, A-$C_2$-$C_6$-Alkinylen oder A-, wobei A ein C- oder N-verknüpfter 5, 6 oder 7-gliedriger Heterocyclus ist, der ein oder mehrere Heteroatome aus der Gruppe Stickstoff, Sauerstoff oder Schwefel enthält und gegebenenfalls ein oder mehrfach, bevorzugt einfach, durch Benzyl, gegebenenfalls Methoxy-substituiertes Benzyl. $C_1$-$C_4$-Alkyl, -CN, -$CH_2NR^7R^8$, -$COOR^9$, -$CONR^7R^8$, -$COR^{10}$, -$NO_2$, -$NH_2$, -$OR^9$, =O, ein Ketal, Ethylenketal, -$SO_3H$, -$SO_2$-$R^9$ oder Halogen, substituiert sein kann;

R Wasserstoff, $C_1$-$C_8$-Alkyl, bevorzugt $C_1$-$C_4$-Alkyl, das gegebenenfalls durch -$COOR^9$, -$COR^{10}$, -$OR^9$, -$OCOR^9$, Amino, Phenyl, Methoxy-substituiertes Phenyl oder Amino substituiert sein kann, oder ein $C_3$-$C_6$-Cycloalkyl, oder

$R^7$ ein direkt oder über eine Alkylkette mit 1 bis 4 C-Atomen C-verknüpfter 5, 6 oder 7-gliedriger Heterocyclus, der ein, zwei oder 3 Heteroatome aus der Gruppe Stickstoff, Sauerstoff oder Schwefel enthält und

gegebenenfalls ein oder mehrfach, bevorzugt einfach, durch Benzyl, Methoxy-substituiertes Benzyl, $C_1$-$C_4$-Alkyl, Halogen, -$OR^9$, -CN, -$NO_2$, -$NH_2$, =O, -$SO_3H$ oder -$COOR^9$ substituiert sein kann;

$R^8$ Wasserstoff, $C_1$-$C_8$-Alkyl, bevorzugt $C_1$-$C_4$-Alkyl, das gegebenenfalls durch -$COOR^9$, -$COR^{10}$, -$OR^9$, -$OCOR^9$, Amino, Phenyl, Methoxy-substituiertes Phenyl oder Amino substituiert sein kann, oder ein $C_3$-$C_6$-Cycloalkyl, oder

$R^8$ ein direkt oder über eine Alkylkette mit 1 bis 4 C-Atomen C-verknüpfter 5, 6 oder 7-gliedriger Heterocyclus, der ein, zwei oder 3 Heteroatome aus der Gruppe Stickstoff, Sauerstoff oder Schwefel enthält und gegebenenfalls ein oder mehrfach, bevorzugt einfach, durch Benzyl, Methoxy-substituiertes Benzyl, $C_1$-$C_4$-Alkyl, Halogen, -$OR^9$, -CN, -$NO_2$, -$NH_2$, =O, -$SO_3H$ oder -$COOR^9$ substituiert sein kann; oder $R^7$ und $R^8$ bilden zusammen mit dem Stickstoffatom einen gesättigten oder ungesättigten 5- oder 6-Ring, der als weitere Heteroatome Stickstoff, Sauerstoff oder Schwefel enthalten kann, wobei der Heterocyclus durch eine verzweigte oder unverzweigte Alkylgruppe mit 1 bis 4 Kohlenstoffatomen, bevorzugt Methyl substituiert sein kann oder einen der Reste -CN, -$COOR^9$, -$CONH_2$, -$NO_2$, -$NH_2$, -$OR^9$, -$SO_3H$, -$SO_2$-$R^9$, Halogen, oder -$(CH_2)_n$-Phenyl, -$(CH_2)$n-$NH_2$, = O, -O-$CH_2$-$CH_2$-O-, -O-$CH_2$-$CH_2$-$CH_2$-O-, $(CH_2)_n$NH-$C_1$-$C_4$-Alkyl, -$(CH_2)_n$-N($C_1$-$C_4$-Alkyl$)_2$, -$(CH_2)_n$-$NHCOOR^9$, wobei n = 1, 2, 3 oder 4 bedeutet, tragen kann;

$R^9$ Wasserstoff, $C_1$-$C_4$-Alkyl, $C_2$-$C_4$-Alkenyl, $C_2$-$C_4$-Alkinyl, einen Benzyl- oder Phenyl-Rest, der gegebenenfalls ein- oder mehrfach durch $OCH_3$ substituiert sein kann;

$R^{10}$ $C_1$-$C_4$-Alkyl, $C_2$-$C_4$-Alkenyl, $C_2$-$C_4$-Alkinyl, gegebenenfalls Methoxy-substituiertes Phenyl, gegebenenfalls Methoxy-substituiertes Benzyl, $C_3$-$C_6$-Cycloalkyl;

$R^{11}$ Wasserstoff, $C_1$-$C_3$-Alkyl, -$COOR^9$, -$CH_2OR^9$, -$CH_2NR^7R^8$, -$CONR^7R^8$ oder gegebenenfalls Methoxy-substituiertes Phenyl, bedeuten können, gegebenenfalls in Form ihrer Racemate, ihrer Enantiomere, ihrer Diastereomere und ihrer Gemische, sowie gegebenenfalls ihrer pharmakologisch unbedenklichen Säureadditionssalze.

Bevorzugt sind Verbindungen der allgemeinen Formel (I), worin

$R^1$ Wasserstoff, $C_1$-$C_8$-Alkyl, das gegebenenfalls durch -COOH, -COO-$C_1$-$C_4$-Alkyl, -COO-Phenyl, -COO-Benzyl, -$CONR^7R^8$, -CO-$C_1$-$C_4$-Alkyl, -CHO, $NR^7R^8$, -NHCO-$C_1$-$C_4$-Alkyl, -NHCO-Phenyl, Hydroxy, $C_1$-$C_4$-Alkyloxy, Phenyloxy, -O-Phenyl-Methoxy, Benzyloxy, -O-Benzyl-O-Methoxy, -OCO-$C_1$-$C_4$-Alkyl, -OCO-Phenyl, -OCO-Benzyl, -OCO-Pyridyl, -O-$C_2$-$C_4$-Alkenyl, oder Halogen, substituiert sein kann, oder

$R^1$ -CHO, -COOH, -COO-$C_1$-$C_4$-Alkyl, -COO-Phenyl, -COO-Benzyl, -$CONR^7R^8$; oder ein Amin der allgemeinen Formel $NR^7R^8$, oder

$R^1$ Phenyl, welches gegebenenfalls durch $C_1$-$C_4$-Alkyl, -CH=NOH, -COOH, -COO-$C_1$-$C_4$-Alkyl, -COO-Phenyl, -COO-Benzyl, $NR^7R^8$, Hydroxy, $C_1$-$C_4$-Alkyloxy, Benzyloxy, Phenyloxy, -OCO-$C_1$-$C_4$-Alkyl, -OCO-Phenyl, -OCO-Benzyl, -OCO-Pyridyl, -O-$C_2$-$C_4$-Alkenyl oder Halogen, bevorzugt Chlor oder Fluor, substituiert sein kann, oder

$R^1$ ein $C_3$-$C_6$-Cycloalkyl- oder $C_3$-$C_6$-Cycloalkyl-$C_1$-$C_4$-Alkyl-Rest, der gegebenenfalls durch Hydroxy, $C_1$-$C_3$-Alkyl oder $C_1$-$C_3$-Alkyloxy substituiert sein kann, oder

$R^1$ ein Norbornan-, Norbornen-, Adamantan- oder Noradamantan-Rest, der gegebenenfalls durch $C_1$-$C_4$-Alkyl, bevorzugt Methyl substituiert sein kann, oder

$R^1$ Phenyl-$C_1$-$C_4$-alkyl-, bevorzugt Benzyl, Phenyl-$C_2$-$C_6$-alkenyl- oder Phenyl-$C_2$-$C_6$-alkinyl-, wobei der Phenylring gegebenenfalls durch $C_1$-$C_3$-Alkyl, -CO-$C_1$-$C_4$-Alkyl, -$C_1$-$C_4$-Alkyl-$NR^7R^8$, -COOH, -COO-$C_1$-$C_4$-Alkyl, -COO-Phenyl, -COO-Benzyl, -$CONR^7R^8$, -CO-$C_1$-$C_4$-Alkyl-$NR^7R^8$, $NR^7R^8$, Hydroxy, $C_1$-$C_4$-Alkyloxy, Benzyloxy, Phenyloxy, -OCO-$C_1$-$C_4$-Alkyl, -OCO-Phenyl, -OCO-Benzyl, -OCO-Pyridyl, -O-$C_2$-$C_4$-Alkenyl oder Halogen, bevorzugt Chlor oder Fluor, substituiert sein kann, oder

$R^1$ ein gegebenenfalls entweder direkt oder über eine Alkylenbrücke mit 1 bis 4 C-Atomen C- oder N-verknüpfter 5 oder 6-gliedriger Heterocyclus, der ein, zwei oder drei Heteroatome aus der Gruppe Stickstoff, Sauerstoff oder Schwefel enthält und gegebenenfalls ein oder mehrfach durch Benzyl oder $C_1$-$C_4$-Alkyl substituiert sein kann;

$R^2$ oder $R^3$ $C_1$-$C_8$-Alkyl, das gegebenenfalls durch $NR^7R^8$, OH, $C_1$-$C_4$-Alkyloxy, oder COOH substituiert sein kann, $C_2$-$C_8$-Alkenyl, Phenyl-$C_1$-$C_4$-alkyl, bevorzugt Benzyl, Phenyl-$C_2$-$C_6$-alkenyl oder Phenyl-$C_2$-$C_6$-alkinyl, wobei der Phenylring gegebenenfalls durch Hydroxy, $C_1$-$C_3$-Alkyl, $C_1$-$C_4$-Alkyloxy, $NR^7R^8$ oder Halogen, bevorzugt Chlor oder Fluor, substituiert sein kann, oder

$R^2$ oder $R^3$ ein gegebenenfalls entweder direkt oder über eine Alkylenbrücke mit 1 bis 4 C-Atomen C- oder N-verknüpfter 5 oder 6-gliedriger Heterocyclus, der ein, zwei oder drei Heteroatome aus der Gruppe Stickstoff, Sauerstoff oder Schwefel enthält und gegebenenfalls ein oder mehrfach durch Benzyl oder $C_1$-$C_4$-Alkyl substituiert sein kann;

$R^4$ oder $R^6$ Wasserstoff, $C_1$-$C_4$-Alkyl, das gegebenenfalls durch $NR^7R^8$ substituiert sein kann, Benzyl;

$R^5$ Wasserstoff, $C_1$-$C_8$-Alkyl, bevorzugt $C_1$-$C_6$-Alkyl, welches gegebenenfalls durch Halogen substituiert sein kann, oder

$R^5$ Phenyl, das gegebenenfalls durch $C_1$-$C_4$-Alkyl, $C_1$-$C_4$-Alkyloxy, Hydroxy oder Halogen, bevorzugt Fluor oder Chlor, substituiert sein kann, oder

$R^5$ Phenyl-$C_1$-$C_6$-alkyl-, bevorzugt Benzyl, wobei der Phenylring gegebenenfalls durch $C_1$-$C_3$-Alkyl, $C_1$-$C_4$-Alkyloxy, Hydroxy, $NR^7R^8$ oder Halogen, bevorzugt Fluor oder Chlor, substituiert sein kann, oder

$R^5$ ein Amin der Formel $NR^7R^8$, oder

$R^5$ ein gegebenenfalls entweder direkt oder über eine Alkylenbrücke mit 1 bis 4 C-Atomen C- oder N-verknüpfter 5 oder 6-gliedriger Heterocyclus, der ein oder mehrere Heteroatome aus der Gruppe Stickstoff oder Sauerstoff enthält und gegebenenfalls durch Benzyl oder $C_1$-$C_4$-Alkyl substituiert sein kann, oder

$R^5$ ein $C_3$-$C_6$-Cycloalkyl welches gegebenenfalls durch =O, Hydroxy oder $C_1$-$C_4$-Alkyloxy substituiert sein kann, oder

$R^5$ gegebenenfalls durch $C_1$-$C_4$-Alkyl, bevorzugt Methyl substituiertes Norbornan, Norbornen, Adamantan oder Noradamantan;

$R^7$ Wasserstoff, eine verzweigte oder unverzweigte Alkylgruppe mit 1 - 4 - Kohlenstoffatomen, oder

$R^7$ ein C-verknüpfter 5 oder 6-gliedriger Heterocyclus, der ein, zwei oder drei Heteroatome aus der Gruppe Stickstoff, Sauerstoff oder Schwefel enthält und gegebenenfalls durch Benzyl, $C_1$-$C_4$-Alkyl, $C_1$-$C_4$-Alkyloxy, Halogen, -CN, $-NO_2$, $-NH_2$, -OH oder =O substituiert sein kann;

$R^8$ Wasserstoff, eine verzweigte oder unverzweigte Alkylgruppe mit 1 - 4 - Kohlenstoffatomen, oder

$R^8$ ein C-verknüpfter 5 oder 6-gliedriger Heterocyclus, der ein, zwei oder drei Heteroatome aus der Gruppe Stickstoff, Sauerstoff oder Schwefel enthält und gegebenenfalls durch Benzyl, $C_1$-$C_4$-Alkyl, $C_1$-$C_4$-Alkyloxy, Halogen, -CN, $-NO_2$, $-NH_2$, -OH oder =O substituiert sein kann; oder
$R^7$ und $R^8$ bilden zusammen mit dem Stickstoffatom einen gesättigten oder ungesättigten 5- oder 6-Ring, der als weitere Heteroatome Stickstoff oder Sauerstoff enthalten kann, wobei der Heterocyclus durch eine verzweigte oder unverzweigte Alkylgruppe mit 1 bis 4 Kohlenstoffatomen, bevorzugt Methyl, oder durch einen Rest $-(CH_2)_{1-4}$-Phenyl, bevorzugt Benzyl substituiert sein kann, bedeuten können, gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

Bevorzugt sind ferner Verbindungen der allgemeinen Formeln (I), worin

$R^1$ Wasserstoff, $C_1$-$C_6$-Alkyl, bevorzugt $C_1$-$C_4$-Alkyl, das gegebenenfalls durch -CO-$C_1$-$C_4$-Alkyl, -CHO, -COOH, -COO-$C_1$-$C_4$-Alkyl, -COO-Phenyl, -COO-Benzyl, -CONR$^7$R$^8$, NR$^7$R$^8$, -NHCO-$C_1$-$C_4$-Alkyl, -NHCO-Phenyl, Hydroxy, =O, $C_1$-$C_4$-Alkyloxy, Phenyloxy, -O-Phenyl-O-Methoxy, Benzyloxy, -O-Benzyl-O-Methoxy, -OCO-$C_1$-$C_4$-Alkyl, -OCO-Phenyl, -OCO-Pyridyl, -OCO-Benzyl, -O-$C_2$-$C_4$-Alkenyl oder Halogen substituiert sein kann, oder

$R^1$ Phenyl, wobei der Phenylring durch $C_1$-$C_4$-Alkyl, Hydroxy, $C_1$-$C_4$-Alkyloxy, NR$^7$R$^8$, Halogen, bevorzugt Fluor oder Chlor, substituiert sein kann, oder

$R^1$ Phenyl-$C_1$-$C_3$-Alkyl, bevorzugt Benzyl, wobei der Phenylring durch $C_1$-$C_3$-Alkyl, -CO-$C_1$-$C_4$-Alkyl, -$C_1$-$C_4$-Alkyl-NR$^7$R$^8$, -COOH,-COO-$C_1$-$C_4$-Alkyl, -COO-Phenyl, -COO-Benzyl, -CONR$^7$R$^8$, -CO-$C_1$-$C_4$-Alkyl-NR$^7$R$^8$, NR$^7$R$^8$, Hydroxy, $C_1$-$C_4$-Alkyloxy, Benzyloxy, Phenyloxy, -OCO-$C_1$-$C_4$-Alkyl, -OCO-Phenyl, -OCO-Benzyl, -OCO-Pyridyl, -O-$C_2$-$C_4$-Alkenyl oder Halogen, bevorzugt Fluor oder Chlor, substituiert sein kann, oder

$R^1$ ein über eine Einfachbindung oder über eine Alkylenkette mit 1 bis 4 C-Atomen verknüpftes Cyclopentyl, Cyclohexyl, Hydroxycyclopentan oder Hydroxycyclohexan, oder

$R^1$ -CHO, -COOH, -COO-$C_1$-$C_4$-Alkyl, -COO-Phenyl, -COO-Benzyl, -CO-NH-$C_1$-$C_4$-Alkyl, -CO-N($C_1$-$C_4$-Alkyl)$_2$ oder -CO-NH-Phenyl, oder

$R^1$ ein Amin der allgemeinen Formel NR$^7$R$^8$, oder

$R^1$ ein über eine Einfachbindung oder über eine Alkylenkette mit 1 bis 4 C-Atomen verknüpftes Furan, Tetrahydrofuran, α-Pyran, γ-Pyran, Dioxolan, Tetrahydropyran, Dioxan, Thiophen, Thiolan, Dithiolan, Pyrrol, Pyrrolin, Pyrrolidin, Pyrazol, Pyrazolin, Imidazol, Imidazolin, Imidazolidin, Triazol, Pyridin, Piperidin, Pyridazin, Pyrimidin, Pyrazin, Piperazin, Triazin, Morpholin, Thiomorpholin, Oxazol, Isoxazol, Oxazin, Thiazol, Isothiazol, Thiadiazol, Oxadiazol oder Pyrazolidin;

$R^2$ oder $R^3$ $C_1$-$C_7$-Alkyl, bevorzugt $C_1$-$C_5$-Alkyl, das gegebenenfalls durch NR$^7$R$^8$, OH, $C_1$-$C_4$-Alkyloxy, oder COOH substituiert sein kann, $C_2$-$C_5$-Alkenyl, $C_2$-$C_5$-Alkinyl, Phenyl-$C_1$-$C_3$-alkyl, bevorzugt Benzyl, wobei der Phenylring durch $C_1$-$C_3$-Alkyl, NR$^7$R$^8$, Hydroxy, $C_1$-$C_4$-Alkyloxy oder Halogen, bevorzugt Chlor oder Fluor, substituiert sein kann, oder

$R^2$ oder $R^3$ ein über eine Einfachbindung oder über eine Alkylenkette mit 1 bis 4 C-Atomen verknüpftes Furan, Tetrahydrofuran, α-Pyran, γ-Pyran, Dioxolan, Tetrahydropyran, Dioxan, Thiophen, Thiolan, Dithiolan, Pyrrol, Pyrrolin, Pyrrolidin, Pyrazol, Pyrazolin, Imidazol, Imidazolin, Imidazolidin, Triazol, Pyridin, Piperidin, Pyridazin, Pyrimidin, Pyrazin, Piperazin, Triazin, Morpholin, Thiomorpholin, Oxazol, Isoxazol, Oxazin, Thiazol, Isothiazol, Thiadiazol, Oxadiazol oder Pyrazolidin;

$R^4$ oder $R^6$ Wasserstoff, $C_1$-$C_4$-Alkyl, bevorzugt $C_1$-$C_3$-Alkyl, $C_1$-$C_4$-Alkyl-NR$^7$R$^8$, Benzyl;

$R^5$ Wasserstoff, $C_1$-$C_6$-Alkyl, bevorzugt $C_1$-$C_4$-Alkyl, welches gegebenenfalls durch Halogen substituiert sein kann, oder

$R^5$ Cyclopentyl, Cyclohexyl, Cyclopentanon, Cyclohexanon, Hydroxycyclopentan oder Hydroxycyclohexan, oder

$R^5$ Pyridyl, Tetrahydrofuranyl, Tetrahydropyranyl, Furyl oder ein gegebenenfalls durch $C_1$-$C_4$-Alkyl oder Benzyl substituierter Morpholin-, Piperidin- oder Piperazinrest, oder

$R^5$ ein Phenylrest, der gegebenenfalls durch $C_1$-$C_4$-Alkyl, Halogen oder Hydroxy substituiert sein kann, oder

$R^5$ ein Phenyl-$C_1$-$C_3$-Alkyl, bevorzugt Benzyl, wobei der Phenylring gegebenenfalls durch $C_1$-$C_3$-Alkyl, NR$^7$R$^8$, Hydroxy, $C_1$-$C_4$-Alkyloxy oder Halogen, bevorzugt Fluor oder Chlor,substituiert sein kann, oder

$R^5$ ein Amin der allgemeinen Formel $NR^7R^8$, oder

$R^5$ ein Norbornen-, Norbornan-, Adamantan- oder Noradamantan-Rest, der gegebenenfalls durch $C_1$-$C_4$-Alkyl, bevorzugt Methyl substituiert sein kann;

$R^7$ Wasserstoff, eine verzweigte oder unverzweigte Alkylgruppe mit 1 - 4 - Kohlenstoffatomen;

$R^7$ ein gegebenenfalls durch $C_1$-$C_4$-Alkyl, $NO_2$, $NH_2$, Hydroxy, $C_1$-$C_4$-Alkyloxy, Chlor oder Brom substituiertes C-verknüpftes Pyrrol, , Pyrrolidin, Pyrazol, Imidazol, Imidazolidin, Triazol, Pyridin, Piperidin, Pyrimidin, Pyrazin, Piperazin, Morpholin, Oxazol, Isoxazol, Thiazol, Isothiazol oder Thiadiazol;

$R^8$ Wasserstoff, eine verzweigte oder unverzweigte Alkylgruppe mit 1 - 4 - Kohlenstoffatomen;

$R^8$ ein gegebenenfalls durch $C_1$-$C_4$-Alkyl, $NO_2$, $NH_2$, Hydroxy, $C_1$-$C_4$-Alkyloxy, Chlor oder Brom substituiertes C-verknüpftes Pyrrol, , Pyrrolidin, Pyrazol, Imidazol, Imidazolidin, Triazol, Pyridin, Piperidin, Pyrimidin, Pyrazin, Piperazin, Morpholin, Oxazol, Isoxazol, Thiazol, Isothiazol oder Thiadiazol;
oder
$R^7$ und $R^8$ bilden zusammen mit dem Stickstoffatom einen gesättigten oder ungesättigten 5- oder 6-Ring, der als weitere Heteroatome Stickstoff oder Sauerstoff enthalten kann, wobei der Heterocyclus durch eine verzweigte oder unverzweigte Alkylgruppe mit 1 bis 4 Kohlenstoffatomen, bevorzugt Methyl, oder durch einen Rest $-(CH_2)_{1-4}$-Phenyl, bevorzugt Benzyl substituiert sein kann, bedeuten können, gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

Erfindungsgemäß bevorzugt sind Verbindungen der allgemeinen Formel (I), worin

$R^1$ Wasserstoff, $C_1$-$C_4$-Alkyl, welches gegebenenfalls durch -NHCO-$C_1$-$C_4$-Alkyl, $NR^7R^8$, Hydroxy, $C_1$-$C_4$-Alkyloxy, Fluor, Chlor oder Brom substituiert sein kann, -CHO, -COOH, -COO-$C_1$-$C_4$-Alkyl, Phenyl, Phenyl-$C_1$-$C_3$-Alkyl-, bevorzugt Benzyl, das gegebenenfalls durch $C_1$-$C_4$-Alkyl, $C_1$-$C_4$-Alkyloxy, Hydroxy, Benzyloxy oder Fluor substituiert sein kann, Phenyloxy-$C_1$-$C_3$-Alkyl-, bevorzugt Phenyloxymethyl, das gegebenenfalls durch Methoxy substituiert sein kann, Benzyloxy-$C_1$-$C_3$-Alkyl-, bevorzugt Benzyloxymethyl, das gegebenenfalls durch Methoxy substituiert sein kann, Benzoyloxymethyl, Pyridylcarbonyloxymethyl, Cyclohexylmethyl, Pyridylmethyl, Pyrrolylmethyl, Morpholinomethyl, Cyclopentyl oder Furyl;

$R^2$ oder $R^3$ $C_1$-$C_5$-Alkyl, das gegebenenfalls durch Hydroxy, Methoxy oder Cyclopropyl substituiert sein kann, $C_2$-$C_4$-Alkenyl, $C_2$-$C_4$-Alkinyl oder Benzyl;

$R^4$ oder $R^6$ Wasserstoff, $C_1$-$C_3$-Alkyl, $C_1$-$C_3$-Alkyl-$NR^7R^8$, N-Morpholinoethyl oder Benzyl;

$R^5$ Wasserstoff, $C_1$-$C_4$-Alkyl, welches gegebenenfalls durch Fluor substituiert sein kann, Phenyl, Benzyl, wobei der Phenylring gegebenenfalls durch Fluor substituiert sein kann,. Pyridyl, Piperidinyl, Morpholinyl, Piperazinyl, 4-Benzylpiperazinyl, Furyl, Tetrahydrofuranyl, Tetrahydropyranyl, $NR^7R^8$, Cyclopentyl, Cyclohexyl, Adamantyl, Noradamantyl, Norbomyl oder Norbomenyl;

$R^7$ Wasserstoff, $C_1$-$C_4$-Alkyl oder Pyridyl;

$R^8$ Wasserstoff, $C_1$-$C_4$-Alkyl oder Pyridyl bedeuten können, gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

Bevorzugt sind ferner Verbindungen der allgemeinen Formel (I), worin

$R^1$ Wasserstoff, Methyl, das gegebenenfalls durch $-NH_2$, -NHMe, $-NMe_2$, -N(i-Propyl)$_2$, -NHAcetyl, Hydroxy, Methoxy, Ethoxy, Phenyloxy, Methoxyphenyloxy, Methoxybenzyloxy, Morpholin, Benzoyloxy, Pyridylcarbonyloxy, Pyridin, Pyridylamino, Methylpiperazin oder Pyrrol substituiert sein kann, Ethyl, das gegebenenfalls durch $NH_2$ oder Hydroxy substituiert sein kann, n-Propyl, i-Propyl, n-Butyl, tert.-Butyl, Cyclopentyl, Cyclohexylmethyl, Benzyl, das gegebenenfalls durch Hydroxy, Methoxy, Benzyloxy, Dimethylaminoethoxy, N-Morpholinoethoxy oder Fluor substituiert sein kann, Phenyl, Phenylethyl, -COOH, -COO-

Methyl, -COOPropyl oder -COOButyl;

$R^2$oder $R^3$ Methyl, das gegebenenfalls durch Cyclopropyl substituiert sein kann, Ethyl, das gegebenenfalls durch Hydroxy oder Methoxy substituiert sein kann, n-Propyl, i-Propyl, n-Butyl, i-Butyl, tert.-Butyl, Butenyl, n-Pentyl, Allyl oder Propargyl;

$R^4$oder $R^6$ Wasserstoff, Methyl oder Ethyl, das gegebenenfalls durch Morpholin substituiert sein kann;

$R^5$ Wasserstoff, Methyl, Ethyl, n-Propyl, i-Propyl, n-Butyl, tert-Butyl, Phenyl, Benzyl, Pyridin, Piperidin, Morpholin, Piperazin, 4-Benzylpiperazinyl, Tetrahydrofuranyl, Tetrahydropyranyl, -$NMe_2$, Cyclopentyl, Cyclohexyl, Adamantyl, Noradamantyl, Nombornanyl oder Norbomenyl bedeuten können, gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

Besonders bevorzugt sind femer Verbindungen der allgemeinen Formel (I), worin

$R^1$ Methyl, das gegebenenfalls durch -$NH_2$, -NHMe, -N(i-Propyl)$_2$, -NHAcetyl, Hydroxy, Phenyloxy, Methylpiperazin oder Pyrrol substituiert sein kann, Ethyl, das gegebenenfalls durch $NH_2$ oder Hydroxy substituiert sein kann, Cyclopentyl, Benzyl, das gegebenenfalls durch Hydroxy, Methoxy, Benzyloxy, Dimethylaminoethoxy, N-Morpholinoethoxy oder Fluor substituiert sein kann, Phenylethyl, -COOH, -COOPropyl oder -COOButyl;

$R^2$oder $R^3$ Methyl, das gegebenenfalls durch Cyclopropyl substituiert sein kann, Ethyl, das gegebenenfalls durch Hydroxy oder Methoxy substituiert sein kann, n-Propyl, i-Propyl, n-Butyl, i-Butyl, Butenyl, n-Pentyl, Allyl oder Propargyl;

$R^4$oder $R^6$ Wasserstoff, Methyl oder Ethyl, das gegebenenfalls durch Morpholin substituiert sein kann;

$R^5$ Methyl, Ethyl, n-Propyl, tert-Butyl, Cyclopentyl oder Norbornenyl bedeuten können gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

Besonders bevorzugt sind erfindungsgemäß Verbindungen der allgemeinen Formel (I), worin

$R^1$ Methyl, das gegebenenfalls durch Phenyloxy oder Pyrrol substituiert sein kann, Benzyl, das gegebenenfalls durch Hydroxy, Methoxy, Dimethylaminoethoxy oder Fluor substituiert sein kann, Cyclopentyl oder Phenylethyl;

$R^2$oder $R^3$ Ethyl, n-Propyl, Allyl oder Propargyl;

$R^4$oder $R^6$ Wasserstoff;

$R^5$ Methyl, Ethyl, n-Propyl, tert-Butyl, Cyclopentyl oder Norbornenyl bedeuten können gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

Die Verbindungen der allgemeinen Formel (I) bilden die folgenden Isomere:

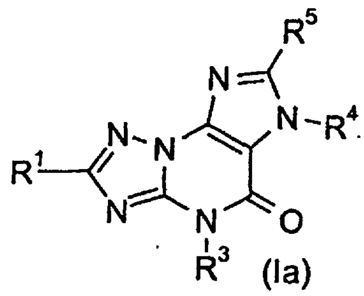
(Ia)

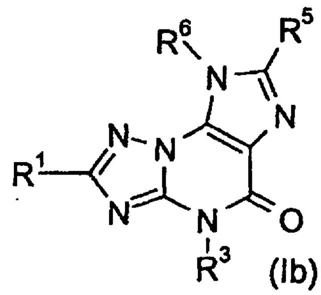
(Ib)

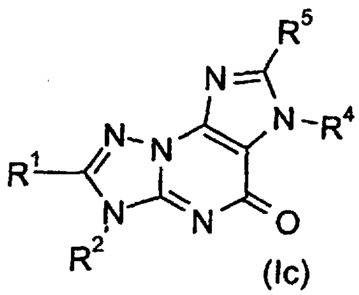
(Ic)

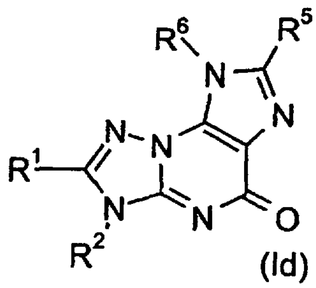
(Id)

wobei die Isomere der allgemeinen Formeln (Ia) und (Ib) bevorzugt sind, insbesondere solche worin $R^4$ oder $R^6$ Wasserstoff oder $C_1$-$C_4$-Alkyl sind. Besonders bevorzugt sind Verbindungen der allgemeinen Formel (Ia) und (Ib), worin $R^4$ und $R^6$ Wasserstoff bedeuten - in diesem Fall sind die Isomere (Ia) und (Ib) Tautomere. Erfindungsgemäß besonders bevorzugt sind die Isomeren (Ia), besonders jene, die in den Positionen 2, 4 und 7 einen Substituenten tragen.

Von besonderem Interesse sind erfindungsgemäß die folgenden Verbindungen:

2-Cyclopentyl-4,7-di-n-propyl-imidazo[4.5-e]-s-triazolo[1 .5-a]pyrimidin-5-on
2-Benzyl-7-methyl-4-n-propyl-imidazo[4.5-e]-s-triazolo[15-a]pyrimidin-5-on
4-Ethyl-7-methyl-2-(2-phenylethyl)-imidazo[4.5-e]-s-triazolo[1.5-a]pyrimidin-5-on
4-Ethyl-2-(2-phenylethyl)-7-n-propyl-imidazo[4.5-e]-s-triazolo[1.5-a]pyrimidin-5-on
7-t-Butyl-4-ethyl-2-(4-hydroxybenzyl)-imidazo[4. 5-e]-s-triazolo[1.5-a]pyrimidin-5-on
7-t-Butyl-4-ethyl-2-(4-methoxybenzyl)-imidazo[4.5-e]-s-triazolo[1.5-a]pyrimidin-5-on
7-Cyclopentyl-4-ethyl-2-phenyloxymethyl-imidazo[4.5-e]-s-triazolo[1.5-a]pyrimidin-5-on
7-Cyclopentyl-4-ethyl-2-(N-pyrrolylmethyl)-imidazo[4.5-e]-s-triazolo[1.5-a]pyrimidin-5-on
2-(2-Furanyl)-7-methyl-4-n-propyl-imidazo[4.5-e]-s-triazolo[1.5-a]pyrimidin-5-on
2-(2-Furanyl)-4,7-di-n-propyl-imidazo[4.5-e]-s-triazolo[1.5-a]pyrimidin-5-on
2-Cyclopentyl-7-ethyl-4-(1-propen-3-yl)-imidazo [4.5-e]-s-triazolo[1.5-a]pyrimidin-5-on
2-Cyclopentyl-7-ethyl-4-(1-propin-3-yl)-imidazo [4.5-e]-s-triazolo[1.5-a]pyrimidin-5-on

Gegebenenfalls können die Verbindungen der allgemeinen Formel (I) in ihre Salze, insbesondere für die pharmazeutische Anwendung, in ihre physiologisch verträglichen Salze mit einer anorganischen oder organischen Säure, überführt werden. Als Säuren kommen hierfür beispielsweise Bernsteinsäure, Bromwasserstoffsäure, Essigsäure, Fumarsäure, Maleinsäure, Methansulfonsäure, Milchsäure, Phosphorsäure, Salzsäure, Schwefelsäure, Weinsäure oder Zitronensäure in Betracht. Ferner können Mischungen der vorgenannten Säuren eingesetzt werden.

Als Alkylgruppen (auch soweit sie Bestandteil anderer Reste sind) werden verzweigte und unverzweigte Alkylgruppen mit 1bis 8 Kohlenstoffatomen bevorzugt 1 - 4 Kohlenstoffatomen betrachtet, beispielsweise werden genannt: Methyl, Ethyl, n-Propyl, iso-Propyl. n-Butyl, iso-Butyl, sec. Butyl, tert.-Butyl, Pentyl, iso-Pentyl, Hexyl, Heptyl und Octyl. Gegebenenfalls werden für vorstehend genannten Gruppen auch die Abkürzungen Me, Et, n-Pr, i-Pr, n-Bu, i-Bu, t-Bu, etc. verwendet.

Als Alkenylgruppen (auch soweit sie Bestandteil anderer Reste sind) werden verzweigte und unverzweigte Alkenylgruppen mit 2 bis 8 Kohlenstoffatomen, bevorzugt 2 bis 4 Kohlenstoffatomen genannt, soweit sie mindestens eine Doppelbindung aufweisen, beispielsweise auch oben genannte Alkylgruppen bezeichnet, soweit sie mindestens eine Doppelbindung aufweisen, wie zum Beispiel Vinyl (soweit keine unbeständigen Enamine oder Enolether gebildet

werden), Propenyl, iso-Propenyl, Butenyl, Pentenyl, Hexenyl.

Als Alkinylgruppen (auch soweit sie Bestandteil anderer Reste sind) werden Alkinylgrupppen mit 2 bis 8 Kohlenstoffatomen bezeichnet, soweit sie mindestens eine Dreifachbindung aufweisen, beispielsweise Ethinyl, Propargyl, Butinyl, Pentinyl, Hexinyl.

Als Cycloalkylreste mit 3 - 6 Kohlenstoffatomen werden beispielsweise Cyclopropyl, Cyclobutyl, Cyclopentyl oder Cyclohexyl bezeichnet, die auch durch verzweigtes oder unverzweigtes Alkyl mit 1 bis 4 Kohlenstoffatomen, Hydroxy, und/oder Halogen oder wie zuvor definiert substituiert sein können. Als Halogen wird im allgemeinen Fluor, Chlor, Brom oder Jod bezeichnet.

Als Beispiele für N-verknüpfte cyclische Reste der allgemeinen Formel $NR^7R^8$ werden genannt: Pyrrol, Pyrrolin, Pyrrolidin, Piperidin, Piperazin, Morpholin, Thiomorpholin, Imidazol, Imidazolin, Imidazolidin, Pyrazol, Pyrazolin, Pyrazolidin, bevorzugt Morpholin, Piperazin, und Piperidin, wobei die genannten Heterocyclen auch durch Benzyl, Alkyl mit 1 bis 4 Kohlenstoffatomen, bevorzugt Methyl, oder wie in den Definitionen angegeben substituiert sein können.

Als C-verknüpfte 5- oder 6-gliedrige heterocyclische Ringe, die als Heteroatome Stickstoff, Sauerstoff oder Schwefel enthalten können, werden beispielsweise Furan, Tetrahydrofuran, Tetrahydrofuranon, γ-Butylrolacton, α-Pyran, γ-Pyran, Dioxolan, Tetrahydropyran, Dioxan, Thiophen, Dihydrothiophen, Thiolan, Dithiolan, Pyrrol, Pyrrolin, Pyrrolidin, Pyrazol, Pyrazolin, Imidazol, Imidazolin, Imidazolidin, Triazol,Tetrazol, Pyridin, Piperidin, Pyridazin, Pyrimidin, Pyrazin, Piperazin, Triazin, Tetrazin, Morpholin, Thiomorpholin, Oxazol, Isoxazol, Oxazin, Thiazol, Isothiazol, Thiadiazol, Oxadiazol, Pyrazolidin genannt, wobei der Heterocyclus wie in den Definitionen angegeben substituiert sein kann. "=O" bedeutet ein über eine Doppelbindung verknüpftes Sauerstoffatom.

Die Herstellung der erfindungsgemäßen Verbindungen kann in der durch den Stand der Technik (Tenor et al., *Chem. Ber.* Vol. 97 (1964) S. 1373-1382) beschriebenen Vorgehensweise erfolgen, auf die an dieser Stelle inhaltlich Bezug genommen wird (Schema 1).

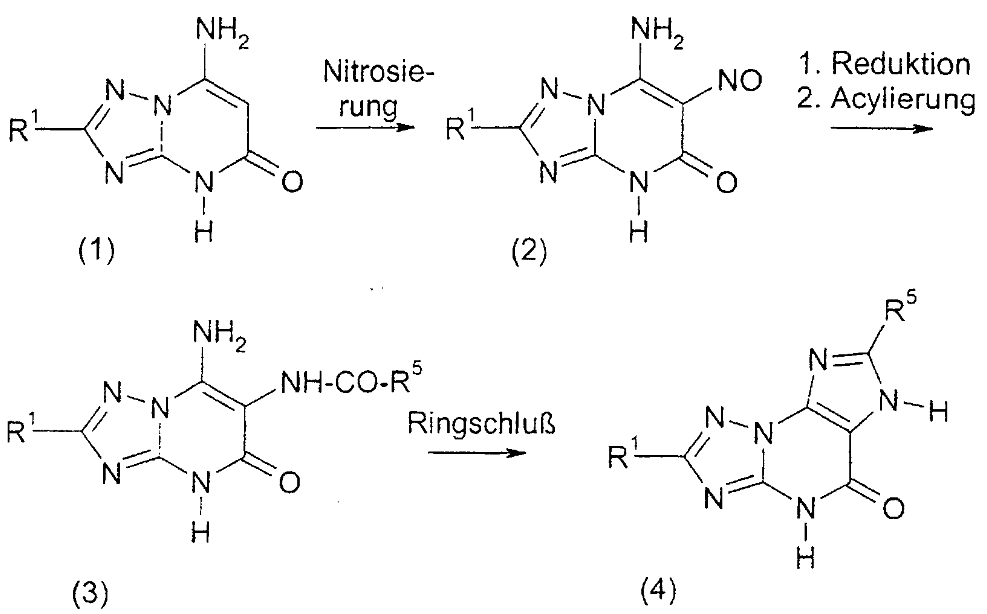

(1)
(2)
(3)
(4)

Schema 1:

Obige, bereits durch den Stand der Technik offenbarte Syntheseroute ist zur Herstellung der erfindungsgemäßen Verbindungen der allgemeinen Formel (I) anwendbar, bei denen die Reste $R^2$ bzw. $R^3$ und $R^4$ bzw. $R^6$ Wasserstoff bedeuten. In diesem Fall sind die Verbindungen der allgemeinen Formel (I) Tautomere, deren eine tautomere Form durch die in Schema 1 genannte Formel (4) verkörpert wird. Die Herstellung der in Schema 1 als Verbindungen (1) bezeichneten Triazolopyrimidone kann in der durch Tenor et al. einleitend beschriebenen Umsetzung von Cyanessigesterderivaten mit geeignet substituierten 3-Amino-1,2,4-triazolen erfolgen. Letztere sind gemäß literaturbekannter Verfahren zugänglich (*J. Org, Chem.* 1926, S. 1729; *Org. Synthesis* 26, S. 11; *J. Chem. Soc.* 1929, S. 816).

Zum Erhalt der Verbindungen der allgemeinen Formel (I), in denen die Reste $R^4$ bzw. $R^6$ und die Reste $R^3$ bzw. $R^2$ eine andere Bedeutung als Wasserstoff aufweisen, ist die folgende Vorgehensweise denkbar.
Vor der durch den Stand der Technik beschriebenen Reaktionsfolge nach Schema 1 kann eine Derivatisierung der Verbindungen (1) gemäß Schema 2 erfolgen. Erfindungesgemäß ist als Derivatisierung eine Alkylierung oder Benzylierung bevorzugt.

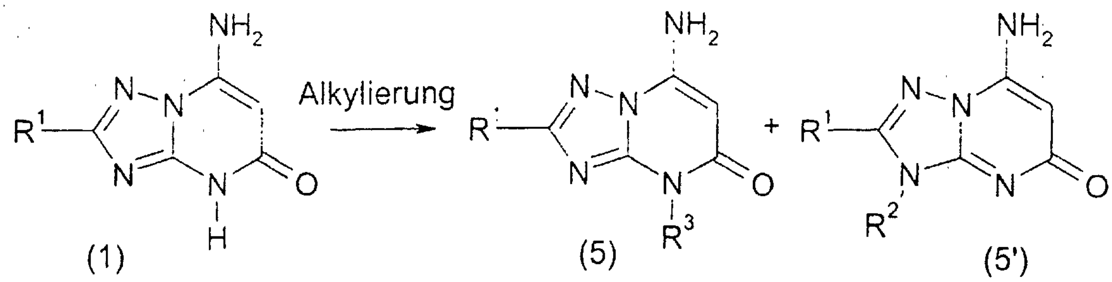


(1) (5) (5')

Schema 2:

Die durchzuführende Alkylierung oder Benzylierung führt zu den Verbindungen (5). Gegebenenfalls können auch die isomeren Alkylierungsprodukte (5') erhalten werden. Gegebenenfalls auftretende Produktgemische von (5) und (5') lassen sich durch dem Fachmann vertraute Reinigungsverfahren wie fraktionierende Kristallisation oder Chromatographie in die reinen Alkylierungsprodukte (5) und (5') auftrennen.

Die Verbindungen (5) bzw. (5') werden anschließend der von Tenor et al. beschriebenen Reaktionsfolge unterworfen, was exemplarisch für die Umsetzung der Verbindungen (5) in Schema 3 skizziert ist.

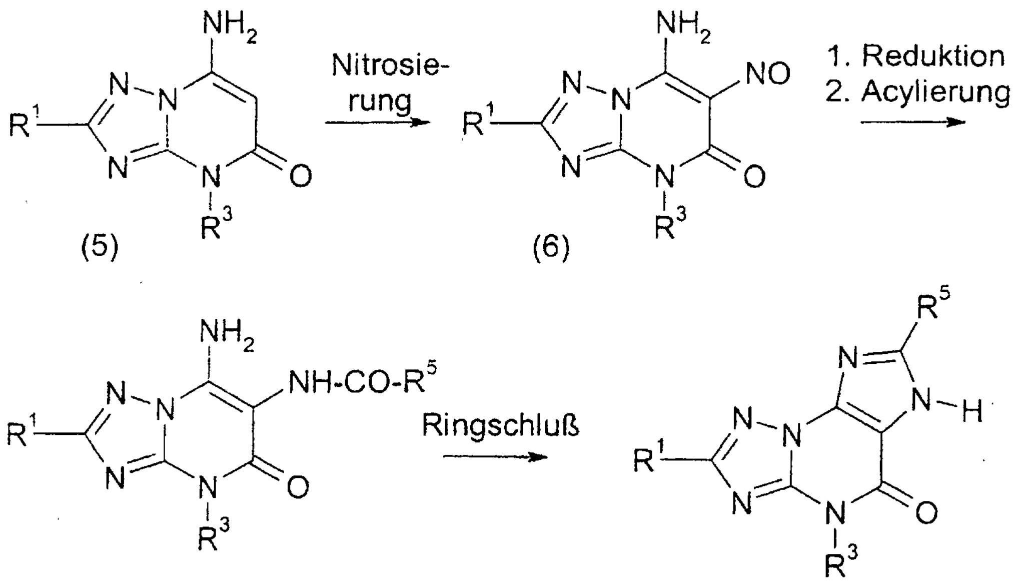


(5) (6) (7) (8)

Schema 3:

Vorstehend beschriebene und in Analogie zum Stand der Technik durchführbare Vorgehensweise ist zur Herstellung der erfindungsgemäßen Verbindungen der allgemeinen Formel (I) anwendbar, bei denen die Reste $R^4$ bzw. $R^6$ Wasserstoff bedeuten. In diesem Fall sind die Verbindungen der allgemeinen Formel (I) Tautomere, deren eine tautomere Form durch die in Schema 3 genannte Formel (8) verkörpert wird.

Ausgehend von den Alyklierungsprodukten (5') lassen sich in entsprechender Art und Weise die Verbindungen (8') erhalten (Schema 4).

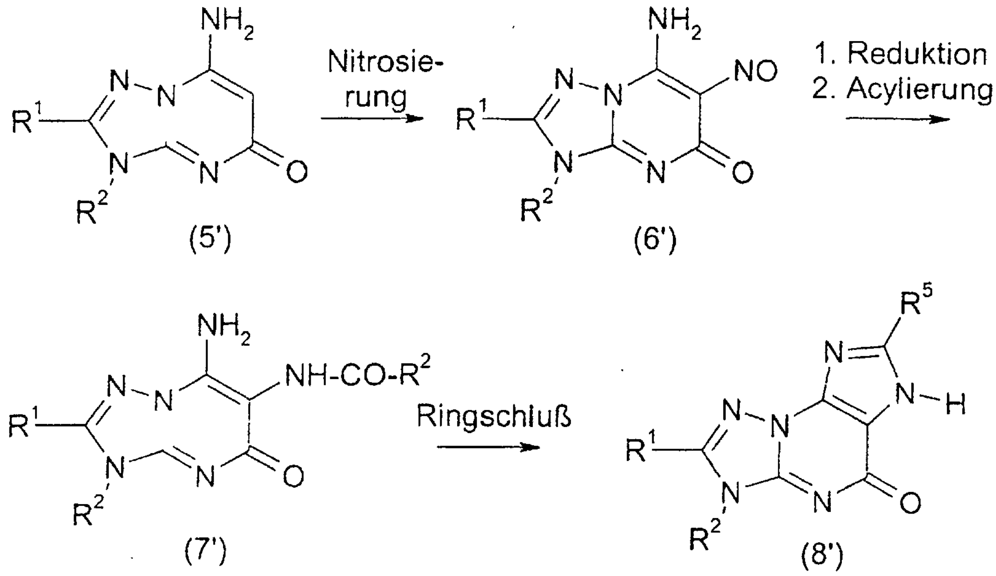


(5') (6') (7') (8')

Schema 4:

Vorstehend beschriebene und in Analogie zum Stand der Technik durchführbare Synthesestrategie ist zur Herstellung der erfindungsgemäßen Verbindungen der allgemeinen Formel (I) anwendbar, bei denen die Reste $R^4$ bzw. $R^6$ Wasserstoff bedeuten. In diesem Fall sind die Verbindungen der allgemeinen Formel (I) Tautomere, deren eine tautomere Form durch die in Schema 4 genannte Formel (8') verkörpert wird.

Durch erneute Alkylierung der Verbindungen (8) sind die Verbindungen (Ia) bzw. (Ib) zugänglich (Schema 5).

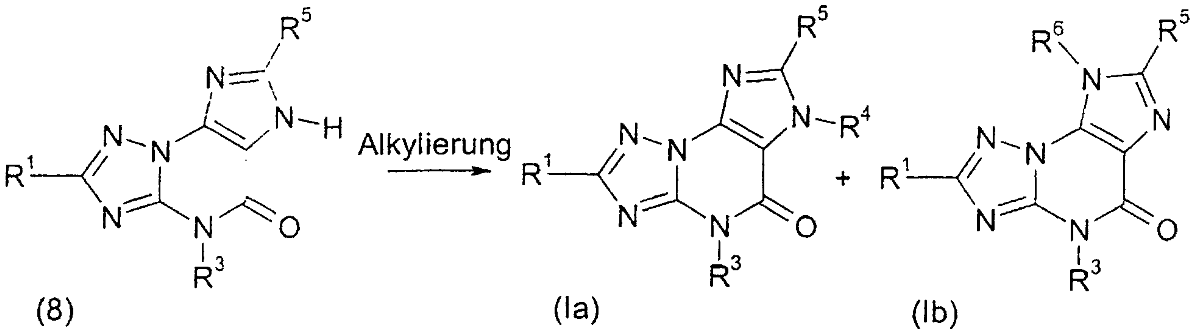


(8) (Ia) (Ib)

Schema 5:

Hierbei werden zumeist die Verbindungen (Ia) als Hauptprodukte isoliert.

In analoger Weise führt die Umsetzung der Derivate (8') zu den Verbindungen (Ic) und (Id, Schema 6).

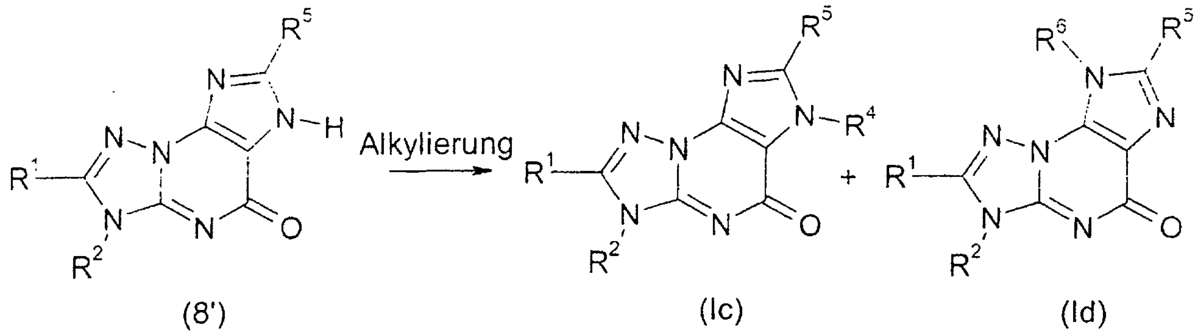


(8') (Ic) (Id)

Schema 6:

Hierbei werden zumeist die Verbindungen (Ic) als Hauptprodukte isoliert.

Je nach Substitutionsmuster lassen sich die Verbindungen (Ia), (Ib), (Ic) oder (Id), in denen die Reste $R^1$ bis $R^6$ die zuvor genannten Bedeutungen haben können, nach literaturbekannten Verfahren weitergehend funktionalisieren. Diese Funktionalisierungen umfassen die dem Fachmann vertrauten Prozesse der Oxidation, Reduktion, Etherspaltung, Acylierungen, Alkylierungen, etc. Ferner kann der Einsatz gängiger Schutzgruppen, insbesondere gängiger Hydroxyl- und Aminoschutzgruppen erforderlich sein. Besonders bevorzugt ist der Einsatz der p-Methoxybenzylgruppe als Schutzgruppe beispielsweise einer Hydroxylfunktion in Kombination mit der Benzylgruppe als Aminoschutzgruppe.

Wesentliche Unterschiede der erfindungsgemäßen Vorgehensweise gegenüber den aus dem Stand der Technik bereits bekannten Verfahren werden im folgenden experimentellen Teil anhand wichtiger Schlüsselschritte detaillierter erläutert. Die folgenden Beispiele dienen der Illustration, ohne die Erfindung auf deren Umfang zu beschränken.

**Synthesebeispiel I:** 7-Cyclopentyl-4-ethyl-2-phenyloxymethyl-imidazo[4.5-e]-s-triazolo[1.5-a]pyrimidin-5-on

Vorstufen:

Ausgehend von Anisalkohol und Chloressigsäure wird unter alkalischen Reaktionsbedingungen 4-Methoxybenzyloxy-essigsäure zugänglich. Die experimentelle Durchführung solcher Ethersynthesen ist dem Fachmann bekannt und bedarf keiner detaillierteren experimentellen Betrachtungsweise. In Anlehnung an literaturbekannte Verfahren (*J. Org, Chem.* 1926, S. 1729; *Org. Synthesis* 26, S. 11; *J. Chem. Soc.* 1929, S. 816) ist daraus durch Umstzung mit Aminoguanidin das entsprechend substituierte 3-Aminotriazol-derivat zugänglich, welches in der durch Tenor et al. (siehe oben) offenbarten Vorgehensweise in das 7-Amino-2-[(4-methoxybenzyloxy)methyl]-s-triazolo[1.5-a]pyrimidin-5-on überführt wird.

7-Amino-4-ethyl-2-[(4-methoxybenzyloxylmethyl]-s-triazolo[1.5-a]pyrimidin-5-on:

In 1500 ml Dimethylformamid weren 145.6 g 7-Amino-2-[(4-methoxybenzyloxy) methyl]-s-triazolo[1.5-a]pyrimidin-5-on gelöst und mit 76,8 g Kaliumcarbonat versetzt. Nach Zugabe von 45,2 ml Ethyliodid wird ca. 1,5 Tage bei Raumtemperatur gerührt. Zur Aufarbeitung wird das Dimethylformamid im Vakuum abdestilliert und der verbleibende Rückstand in 500 ml Wasser (destilliert), 500 ml gesättigte NaCl-Lösung und 1000 ml Dichlormethan aufgenommen. Nach Abtrennen der organischen Phase wird die verbleibende wässrige Phase nochmals dreimal mit je 200 ml Dichlormethan extrahiert. Die vereinigten organischen Phasen werden über Magnesiumsulfat getrocknet und anschließend vom Lösemittel befreit. Es verbleiben ca. 180 g eines Feststoffs, der ohne weitere Reinigung in die nächste Stufe eingesetzt wird.

7-Amino-4-ethyl-2-[(4-methoxybenzyloxy)methyl]-6-nitroso-s-triazolo[1.5-a]pyrimidin-5-on:

In 17 ml Dimethylformamid weren 3,7 g der Vorstufe (7-Amino-4-ethyl-2-[(4-methoxybenzyloxy)methyl]-s-triazolo[1.5-a]pyrimidin-5-on) gelöst und unter Eiskühlung mit 2,69 ml Isoamylnitrit versetzt. Anschließend wird bei Raumtemperatur gerührt. Nach vollständigem Umsatz (ca. 3 Stunden) werden bei 5-10°C 34 ml Wasser zugegeben. Der erhaltene Feststoff wird abgetrennt, mit Wasser und Diethylether gewaschen und aus 80 ml Ethanol umkristallisiert. Es verbleiben 2,0 g 7-Amino-4-ethyl-2-[(4-methoxybenzyloxy)methyl]-6-nitroso-s-triazolo[1.5-a]pyrimidin-5-on.

6.7-Diamino-4-ethyl-2-[(4-methoxybenzyloxy)methyl]-s-triazolo[1.5-a]pyrimidin-5-on:

In 64 ml wässriger Ammoniaklösung (25%-ig) und 13 ml Ethanol werden bei Raumtemperatur 1,5 g 7-Amino-4-ethyl-2-[(4-methoxybenzyloxy)methyl]-6-nitroso-striazolo[1.5-a]pyrimidin-5-on eingetragen und gelöst. Bei 30-35°C wird eine Lösung aus 3,3 g $Na_2S_2O_4$in 30 ml Wasser zugetropft. Die so erhaltene Mischung wird bei Raumtemperatur bis zum vollständigen Umsatz gerührt (ca. 3-4 Stunden). Nach Abkühlen im Eisbad werden die erhaltenen Kristalle abgesaugt und getrocknet. Es werden 1,2 g 6,7-Diamino-4-ethyl-2-[(4-methoxybenzyloxy)methyl]-s-trlazolo[1.5-a]pyrimidin-5-on erhalten, die ohne weitere Reinigung in die nächste Stufe eingesetzt werden.

7-Amino-6-cyclopentylcarbonylamino-4-ethyl-2-[(4-methoxybenzyloxy)methyl]-striazolo[1.5-a]pyrimidin-5-on:

In 19 ml Dimethylformamid werden 1,1 g 6,7-Diamino-4-ethyl-2-[(4-methoxybenzyloxy)methyl]-s-triazolo [1.5-a]pyrimidin-5-on aufgenommen und mit 0,7 g Dimethylaminopyridin (DMAP) versetzt. Bei 3-5 °C erfolgt unter Rühren die Zugabe von 0,5 ml Cyclopentancarbonsäurechlorid (gelöst in 2 ml Dimethylformamid). Die erhaltene Mischung wird bei konstanter Temperatur 2 Stunden gerührt. Unter Rühren wird der Ansatz bis zum vollständigen Umsatz (ca. 12-16 Stunden) bei Raumtemperatur gehalten. Zur Aufarbeitung wird das Dimethylformamid im Vakuum abdestilliert und der verbleibende Rückstand in 20 ml Wasser und 20 ml Diethylether aufgenommen. Der ausgefallene Feststoff wird abgetrennt und mit Wasser und Diethylether gewaschen. Nach Trocknen der erhaltenen Kristalle (1,27 g) wird das so erhaltene Acylierungsprodukt ohne weitere Reinigung in die nächste Stufe eingesetzt.

7-Cyclopentyl-4-ethyl-2-[(4-methoxybenzyloxy)methyl-imidazo[4,5-e]-s-triazolo[1,5-a]pyrimidin-5-on:

In 15 ml Wasser und 7 ml Ethanol werden 1,25 g des zuvor synthetisierten Acylierungsproduktes aufgenommen und nacheinander mit 3,5 ml einer wässrigen Natriumhydroxidlösung (50%-ig) sowie 0,95 g Calziumhydroxid versetzt. Die so erhaltene Mischung wird bei erhöhter Temperatur (ca. 95°C) bis zum vollständigen Umsatz gerührt (ca. 6 Stunden). Zur Aufarbeitung wird abgekühlt (Eiskühlung) und mit ca. 20 ml wässriger, 4 n HCl-Lösung versetzt. Der ausgefallene Feststoff wird abgetrennt und getrocknet. Es verbleiben 1,1 g für die Folgeumsetzungen ausreichend reines 7-Cyclopentyl-4-ethyl-2-[(4-methoxybenzyloxy)methyl-imidazo[45-e]-s-triazolo[1.5-a]pyrimidin-5-on.

6-Benzyl-7-cyclopentyl-4-ethyl-2-[(4-methoxybenzyloxy)methyl-imidazo[4,5-e]-s-triazolo[1.5-a]pyrimidin-5-on:

In 50 ml Dimethylformamid werden 2,11 g 7-Cyclopentyl-4-ethyl-2-[(4-methoxybenzyloxy)methyl-imidazo [4.5-e]-s-triazolo[1.5-a]pyrimidin-5-on und 0,76 g Kaliumcarbonat aufgenommen. Unter Kühlung und Rühren werden 0,65 ml Benzylbromid tropfenweise zugegeben. Die erhaltene Lösung wird anschließend 2 bis 3 Tage bei Raumtemperatur gerührt. Zur Aufarbeitung wird das Dimethylformamid im Vakuum abdestilliert und der verbleibende Rückstand in 20 ml Dichlormethan aufgenommen und mit 20 ml Wasser gewaschen. Die organische Phase wird abgetrennt und über Magnesiumsulfat getrocknet. Nach Abdestillieren des Lösemittels wird der verbleibende Rückstand in 20 ml Diethylether verrührt. Der dabei ausfallende Feststoff wird abgetrennt und getrocknet. Es werden 2,6 g 6-Benzyl-7-cyclopentyl-4-ethyl-2-[(4-methoxybenzyloxy)methyl-imidazo[4.5-e]-s-triazolo[1.5-a]pyrimidin-5-on erhalten. Eine weitergehende Reinigung erfolgt nicht.

6-Benzyl-7-cyclopentyl-4-ethyl-2-hydroxymethyl-imidazo[4.5-e]-s-triazolo[1.5-a]pyrimidin-5-on:

6-Benzyl-7-cyclopentyl-4-ethyl-2-[(4-methoxybenzyloxy)methyl-imidazo[4.5-e]-s-triazolo[1.5-a]pyrimidin-5-on (1,03 g) wird in 20 ml Dichlormethan gelöst und mit 1,2 ml Wasser versetzt. Bei Raumtemperatur wird unter Rühren 2,3-Dichlor-5,6-dicyanobenzochinon (DDQ, 0,68 g) zugegeben und weitere 4 Stunden gerührt. Nach Abtrennen des ausgefallenen 2,3-Dichlor-5,6-dicyanohydrochinons wird die verbleibende Lösung mit gesättigter, wässriger Natriumhydrogencarbonatlösung und gesättigter, wässriger Natriumchloridlösung gewaschen und über Natriumsulfat getrocknet. Das Lösemittel wird abdestilliert und der verbleibende Rückstand aus Diethylether kristallisiert. Es werden 0,74 g 6-Benzyl-7-cyclopentyl-4-ethyl-2-hydroxymethyl-imidazo[4.5-e]-s-triazolo[1.5-a]pyrimidin-5-on in Form leicht rötlicher Kristalle erhalten, die in die nächste Stufe ohne weitergehende Reinigung eingesetzt werden können.

6-Benzyl-2-brommethyl-7-cyclopentyl-4-ethyl-imidazo[4.5-e]-s-triazolo[1,5-a]pyrimidin-5-on:

In 7 ml Dichlormethan werden 0,6 g 6-Benzyl-7-cyclopentyl-4-ethyl-2-hydroxymethylimidazo[4.5-e]-s-triazolo [1.5-a]pyrimidin-5-on aufgenommen und unter Rühren bei Raumtemperatur mit 0,25 ml Triethylamin versetzt. Anschließend erfolgt die Zugabe von 0,15 ml Thionylbromid. Nach 2 Stunden Rühren bei konstanter Temperatur ist die Reaktion vollständig. Zur Aufarbeitung wird mit 20 ml Dichlormethan verdünnt und nacheinander mit 15 ml Wasser und 15 ml wässriger Natriumchloridlösung gewaschen. Die organische Phase wird über Magnesiumsulfat getrocknet und das Lösemittel im Vakuum abdestilliert. Der verbleibende Rückstand wird aus Diethylether umkristallisiert. Es verbleiben 0,45 g der für die Folgeumsetzungen ausreichend reinen Titelverbindung.

6-Benzyl-7-cyclopentyl-4-ethyl-2-phenyloxymethyl-imidazo[4.5-e]-s-triazolo[1.5-a]pyrimidin-5-on:

Zu einer Lösung von 0,16 g Phenol in 15 ml Dimethylformamid werden 66 mg Natriumhydrid (60%-ig in Mineralöl) gegeben. Nach 30 Minuten Rühren bei Raumtemperatur erfolgt die Zugabe von 0,68 g 6-Benzyl-2-brommethyl-7-cyclopentyl-4-ethyl-imidazo[4.5-e]-s-triazolo[1.5-a]pyrimidin-5-on. Es wird bei erhöhter Temperatur (ca. 100°C) bis zum vollständigen Umsatz gerührt (ca. 8 Stunden). Zur Aufarbeitung wird etwas Wasser zugesetzt und anschließend das Lösemittel im Vakuum abdestilliert. Der verbleibende Rückstand wird in 20 ml Dichlormethan und 20 ml Wasser aufgenommen. Nach Ansäuern mit wässriger, 1n HCl-Lösung wird die organische Phase abgetrennt, mit Wasser gewaschen und über Magnesiumsulfat getrocknet. Das Lösemittel wird abdestilliert und der verbleibende Rückstand aus Diethylether umkristallisiert. Es werden 0,6 g der für die Folgeumsetzungen ausreichend reinen Titelverbindung erhalten.

7-Cyclopentyl-4-ethyl-2-phenyloxymethyl-imidazo[4.5-e]-s-triazolo[1.5-a]pyrimidin-5-on:

0,6 g 6-Benzyl-7-cyclopentyl-4-ethyl-2-phenyloxymethyl-imidazo[4.5-e]-s-triazolo[1.5-a]pyrimidin-5-on werden in 30 ml Methanol an 0,1 g Pearlman-Katalysator hydriert (60°C, 5 bar). Nach Abtrennen des Katalysators wird der Rückstand zur Trockne eingeengt. Die Reinigung der Titelverbindung erfolgt durch Chromatographie an Kieselgel (Dichlormethan:Methanol 99:1). Gemäß vorstehender Vorgehensweise wurden 0,2 g der Titelverbindung (Schmp.: 229°C) erhalten.

**Synthesebeispiel II:** 7-Cyclopentyl-4-ethyl-2-(methylaminomethyl)-imidazo[4.5-e]-striazolo[1.5-a] pyrimidin-5-on

6-Benzyl-7-cyclopentyl-4-ethyl-2-formyl-imidazo[4.5-e]-s-triazolo[1.5-a]pyrimidin-5-on:

In 1,1 ml Dichlormethan werden 0,3 g 6-Benzyl-7-cyclopentyl-4-ethyl-2-hydroxymethyl-imidazo[4.5-e]-s-triazola[1.5-a]pyrimidin-5-on (Herstellung siehe Synthesebeispiel 1) gelöst und mit 0,45 g Pyridiniumdichromat versetzt. Die erhaltene braune Suspension wird ca. 24 Stunden bei Raumtemperatur gerührt. Das Lösemittel wird abdestilliert und der verbleibende Rückstand mit einem Ethylacetat-Cyclohexan Gemisch (2:1, 5 ml) ausgerührt. Die klare, überstehende leicht gelbe Lösung wird über Kieselgel filtriert. Nach Abdestillieren des Lösemittels verbleiben 0,15 g der Titelverbindung in Form eines farblosen Harzes.

6-Benzyl-7-cyclopentyl-4-ethyl-2-(methylaminomethyl)-imidazo[4,5-e]-s-triazolo[1,5-a]pyrimidin-5-on:

In 30 ml Methanol werden 0,5 g 6-Benzyl-7-cyclopentyl-4-ethyl-2-formyl-imidazo[4.5-e]-s-triazolo[1.5-a]pyrimidin-5-on gemeinsam mit 0,1 g Methylamin an 50 mg Palladium auf Kohle (10%-ig) hydriert (5 bar, 20°C, 3 Stunden). Zur Aufarbeitung wird der Katalysator abfiltriert und das Lösemittel im Vakuum abdestilliert. Die Reinigung des Rohprodukts erfolgt durch Chromatographie an Kieselgel (Dichlormethan:Methanol 95:5) und liefert 0,42 g der Titelverbindung in Form eines farblosen Öls.

7-Cyclopentyl-4-ethyl-2-(methylaminomethyl)-imidazo[4,5-e]-s-triazolo[1.5-a]pyrimidin-5-on:

In 30 ml Eisessig werden 0,37 g 2-Aminomethyl-6-benzyl-7-cyclopentyl-4-ethyl-imidazo[4-5-e]-s-triazolo [1.5-a]pyrimidin-5-on an 50 mg Palladium auf Kohle (10%-ig) hydriert (5 bar, 20°C, 19 Stunden). Zur Aufarbeitung wird der Katalysator abfiltriert und das Lösemittel im Vakuum abdestilliert. Die Reinigung des Rohprodukts erfolgt durch Chromatographie an Kieselgel (Dichlormethan:Methanol 90:10) und liefert 0,18 g der Titelverbindung in Form farbloser Kristalle (Schmp.: 172-175 °C).

**Synthesebeispiel** III: 2-(N-Acetylaminomethyl)-7-cyclopentyl-4-ethyl-imidazo[4.5-e]-s-triazolo[1.5-a]pyrimidin-5-on

6-Benzyl-2-(N-acetylaminomethyl)-7-cyclopentyl-4-ethyl-imidazo[4.5-e]-s-triazolo[1.5-a]pyrimidin-5-on:

In 10 ml Dichlormethan und 0,2 ml Pyridin werden 0,5 g 2-Aminomethyl-6-benzyl-7-cyclopentyl-4-ethyl-imidazo[4.5-e]-s-triazola[1.5-a]pyrimidin-5-on (Herstellung siehe Synthesebeispiel II) gelöst und unter Kühlung mit 0,1 ml Acetylchlorid tropfenweise versetzt. Nach 2 Stunden Rühren bei konstanter Temperatur wird der Ansatz zur Aufarbeitung mit 10 ml Dichlormethan verdünnt und zweimal mit verdünnter, wässriger Salzsäure gewaschen. Die organische Phase wird abgetrennt, über Magnesiumsulfat getrocknet und das Lösemittel im Vakuum abdestilliert. Es verbleiben 0,7 g der Titelverbindung in Form eines amorphen, farblosen Feststoffs.

2-(N-Acetylaminomethyl)-7-cyclopentyl-4-ethyl-imidazo[4.5-e]-s-triazolo[1.5-a]pyrimidin-5-on:

In 30 ml Eisessig werden 0,7 g 6-Benzyl-2-(N-acetylaminomethyl)-7-cyclopentyl-4-ethyl-imidaza[4.5-e]-s-triazolo[1.5-a]pyrimidin-5-on an 50 mg Pearlman-Katalysator hydriert (5 bar, 60°C. 5,3 Stunden). Zur Aufarbeitung wird der Katalysator abfiltriert und das Lösemittel im Vakuum abdestilliert. Die Reinigung des Rohprodukts erfolgt durch Chromatographie an Kieselgel (Dichlormethan:Methanol 95:5) und liefert 0,3 g der Titelverbindung in Form farbloser Kristalle (Schmp.: 269-271°C).

**Synthesebeispiel IV**: 2-Aminomethyl-7-cyclopentyl-4-ethyl-imidazo[4.5-e]-s-triazolo[1.5-a] pyrimidin-5-on

2-Aminomethyl-6-benzyl-7-cyclopentyl-4-ethyl-imidazo[4.5-e]-s-triazolo[1.5-a]pyrimidin-5-on:

In 30 ml Methanol werden 2 g 6-Benzyl-7-cyclopentyl-4-ethyl-2-formyl-imidazo[4.5-e]-s-triazolo[1.5-a]pyrimidin-5-on (Herstellung siehe Synthesebeispiel II) gemeinsam mit 1 g Ammoniak an 0,2 g Palladium auf Kohle (10%-ig) hydriert (5 bar, 20°C, 2,3 Stunden). Zur Aufarbeitung wird der Katalysator abfiltriert und das Lösemittel im Vakuum abdestilliert. Die Reinigung des Rohprodukts erfolgt durch Chromatographie an Kieselgel (Dichlormethan:Methanol 95:5) und liefert 0,65 g der Titelverbindung in Form eines farblosen, amorphen Feststoffs.

2-Aminomethyl-7-cyclopentyl-4-ethyl-imidazo[4.5-e]-s-triazolo[1.5-a]pyrimidin-5-on:

In 30 ml Eisessig werden 0,56 g 2-Aminomethyl-6-benzyl-7-cyclopentyl-4-ethyl-imidazo[4.5-e]-s-triazolo [1.5-a]pyrimidin-5-on an 1 g Palladium auf Kohle (10%-ig) hydriert (5 bar, 60°C, 6,4 Stunden). Zur Aufarbeitung wird der Katalysator abfiltriert und das Lösemittel im Vakuum abdestilliert. Die Reinigung des Rohprodukts erfolgt durch Chromatographie an Kieselgel (Dichlormethan:Methanol 90:10) und liefert 0,18 g der Titelverbindung in Form farbloser Kristalle (Schmp.: 240-245°C).

**Synthesebeispiel V:** 7-Cyclopentyl-4-ethyl-2-(N-pyrrolylmethyl)-imidazo[4.5-e]-s-triazolo[1.5-a]pyrimidin-5-on

6-Benzyl-7-cyclopentyl-4-ethyl-2-(N-pyrrolylmethyl)-imidazo[4.5-e]-s-triazolo[1.5-a]pyrimidin-5-on:

Zu einer Lösung von 0,8 g Pyrrol in 10 ml Dimethylformamid werden 48 mg Natriumhydrid (60%-ig in Mineralöl) gegeben. Nach 30 Minuten Rühren bei Raumtemperatur erfolgt die Zugabe von 0,46 g 6-Benzyl-2-brommethyl-7-cyclopentyl-4-ethyl-imidazo[4.5-e]-s-triazolo[1.5-a]pyrimidin-5-on (Herstellung siehe Synthesebeispiel 1). Es wird bei erhöhter Temperatur (ca. 60°C) bis zum vollständigen Umsatz gerührt (ca. 6 Stunden). Zur Aufarbeitung wird etwas Wasser zugesetzt und anschließend das Lösemittel im Vakuum abdestilliert. Der verbleibende Rückstand wird in 15 ml Dichlormethan und 10 ml Wasser aufgenommen. Nach Ansäuern mit wässriger, 1n HCl-Lösung wird die organische Phase abgetrennt. mit Wasser gewaschen und über Magnesiumsulfat getrocknet. Das Lösemittel wird abdestilliert und der verbleibende Rückstand durch Chromatographie an Kieselgel gereinigt (Ethylacetat:Cyclohexan 1:1). Es werden 0,2 g der Titelverbindung in Form eines farblosen Feststoffs erhalten.

7-Cyclopentyl-4-ethyl-2-(N-pyrrolylmethyl)-imidazo[4.5-e]-s-triazolo[1.5-a]pyrimidin-5-on:

1 g 6-Benzyl-7-cyclopentyl-4-ethyl-2-(N-pyrrolylmethyl)-imidazo[4.5-e]-s-triazolo[1.5-a]pyrimidin-5-on werden in 100 ml Methanol an 0,1 g Pearlman-Katalysator hydriert (60°C, 5 bar, 4,5 Stunden). Nach Abtrennen des Katalysators wird der Rückstand zur Trockne eingeengt. Die Reinigung der Titelverbindung erfolgt durch Chromatographie an Kieselgel (Dichlormethan:Methanol 97:3).
Gemäß vorstehender Vorgehensweise wurden 0,67 g der Titelverbindung (Schmp.:282-283°C) erhalten.

**Synthesebeispiel VI:** 2-(2-Aminoethyl)-7-cyclopentyl-4-ethyl-imidazo[4.5-e]-s-triazolo[1.5-a] pyrimidin-5-on

6-Benzyl-2-cyanomethyl-7-cyclopentyl-4-ethyl-imidazo[4.5-e]-s-triazolo[1.5-a]pyrimidin-5-on:

Zu einer zum Sieden erhitzten Lösung von 0,72 g KCN in 0,7 ml Wasser und 2,4 ml Ethanol werden 0,46 g 6-Benzyl-2-brommethyl-7-cyclopentyl-4-ethyl-imidazo[4.5-e]-s-triazolo[1.5-a]pyrimidin-5-on (Herstellung siehe Synthesebeispiel 1) gegeben. Es wird weitere 30 Minuten bis zum vollständigen Umsatz gekocht. Zur Aufarbeitung wird mit 10 ml Wasser verdünnt und mit 15 ml Dichlormethan extrahiert. Die organische Phase wird abgetrennt, mit gesättigter Natriumchloridlösung gewaschen und über Magnesiumsulfat getrocknet. Das Lösemittel wird abdestilliert und der verbleibende Rückstand durch Chromatographie an Kieselgel gereinigt (Dichlormethan:Methanol 99:1). Es werden 0,3 g der Titelverbindung in Form eines farblosen Feststoffs erhalten.

2-(2-Aminoethyl)-7-cyclopentyl-4-ethyl-imidazo[4.5-e]-s-triazolo[1.5-a] pyrimidin-5-on:

0,26 g 6-Benzyl-2-cyanomethyl-7-cyclopentyl-4-ethyl-imidazo[4.5-e]-s-triazolo[1.5-a]pyrimidin-5-on werden in 30 ml Methanol und 1 ml konzentrierter Salzsäure (32%-ig) an 50 mg Palladium-Katalysator (10%-ig) hydriert (60°C, 5 bar, 3,3 Stunden) hydriert. Nach Abtrennen des Katalysators wird der Rückstand zur Trockne eingeengt. Die Reinigung der Titelverbindung erfolgt durch Kristallisation aus Methanol. Gemäß vorstehender Vorgehensweise wurden

0,23 g der Titelverbindung (Schmp.:272-275°C) erhalten.

Gemäß den zuvor beschriebenen Verfahren oder in Anlehnung an Analogieverfahren konnten die in Tabelle 1 aufgeführten Verbindungen der allgemeinen Formel (I) hergestellt werden.

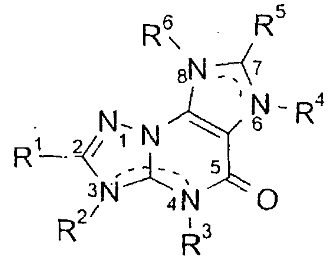

(I)

Tabelle 1:

| Nr. | $R^1$ | $R^2$ | $R^3$ | $R^4$ (oder $R^6$) | $R^5$ | Schmp. [°C] | Bezeichnung |
|---|---|---|---|---|---|---|---|
| 1 | Cyclopentyl | - | n-Propyl | Methyl | Ethyl | 155-157 | 2-Cyclopentyl-7-ethyl-6-methyl-4-n-propyl-imidazo[4.5-e]-s-triazolo[1.5-a]pyrimidin-5-on |
| 2 | Cyclopentyl | - | n-Propyl | H | n-Propyl | 244-246 | 2-Cyclopentyl-4,7-di-n-propyl-imidazo[4.5-e]-s-triazolo[1.5-a]pyrimidin-5-on |
| 3 | Cyclopentyl | - | i-Propyl | H | Ethyl | 260-262 | 2-Cyclopentyl-7-ethyl-4-i-propyl-imidazo [4.5-e]-s-triazolo[1.5-a]pyrimidin-5-on |
| 4 | Cyclopentyl | - | n-Butyl | H | Ethyl | 265 | 4-n-Butyl-2-cyclopentyl-7-ethyl-imidazo [4.5-e]-s-triazolo[1.5-a]pyrimidin-5-on |
| 5 | Cyclopentyl | - | i-Butyl | H | Ethyl | 297 | 4-i-Butyl-2-cyclopentyl-7-ethyl-imidazo [4.5-e]-s-triazolo[1.5-a]pyrimidin-5-on |
| 6 | Cyclopentyl | - | n-Pentyl | H | Ethyl | 255 | 2-Cyclopentyl-7-ethyl-4-n-pentyl-imidazo [4.5-e]-s-triazolo[1.5-a]pyrimidin-5-on |

| Nr. | $R^1$ | $R^2$ | $R^3$ | $R^4$ (oder $R^6$) | $R^5$ | Schmp. [°C] | Bezeichnung |
|---|---|---|---|---|---|---|---|
| 7 | Cyclopentyl | - | 1-Buten-4-yl | H | Ethyl | 243 | 4-(1-Buten-4-yl)-2-cyclopentyl-7-ethyl-imidazo [4.5-e]-s-triazolo[1.5-a]pyrimidin-5-on |
| 8 | Benzyl | - | n-Propyl | H | Methyl | 231-233 | 2-Benzyl-7-methyl-4-n-propyl-imidazo[4.5-e] s-triazolo[1.5-a]pyrimidin 5 on |
| 9 | Benzyl | - | n-Propyl | Methyl | Ethyl | 167-169 | 2-Benzyl-7-ethyl-6-methyl-4-n-propyl-imidazo[4.5-e]-s-triazolo[1.5-a]pyrimidin-5-on |
| 10 | 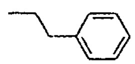 | - | Ethyl | H | Methyl | 283-284 | 4-Ethyl-7-methyl-2-(2-phenylethyl)-imidazo[4.5-e]-s-triazolo[1.5-a]pyrimidin-5-on |
| 11 | 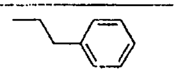 | - | Ethyl | H | n-Propyl | 250-253 | 4-Ethyl-2-(2-phenylethyl)-7-n-propyl-imidazo[4.5-e]-s-triazolo[1.5-a]pyrimidin-5-on |
| 12 | 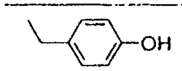 | - | Ethyl | H | t-Butyl | 297-298 | 7-t-Butyl-4-ethyl-2-(4-hydroxybenzyl)-imidazo[4.5-e]-s-triazolo[1.5-a]pyrimidin-5-on |
| 13 | 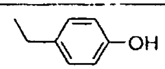 | - | Ethyl | Methyl | t-Butyl | 202-204 | 7-t-Butyl-4-ethyl-2-(4-hydroxybenzyl)-6-methyl-imidazo[4.5-e]-s-triazolo[1.5-a]pyrimidin-5-on |
| 14 | 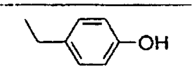 | - | Ethyl | 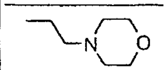 | t-Butyl | - | 7-t-Butyl-4-ethyl-2-(4-hydroxybenzyl)-6-[2-(N-morpholino)-ethyl]-imidazo[4.5-e]-s-triazolo[1.5-a]pyrimidin-5-on |
| 15 | 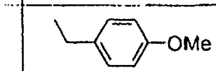 | - | Ethyl | H | t-Butyl | 204 Zers. | 7-t-Butyl-4-ethyl-2-(4-methoxybenzyl)-imidazo[4.5-e]-s-triazolo[1.5-a]pyrimidin-5-on |

| Nr. | $R^1$ | $R^2$ | $R^3$ | $R^4$ (oder $R^6$) | $R^5$ | Schmp. [°C] | Bezeichnung |
|---|---|---|---|---|---|---|---|
| 16 | 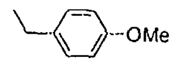 | - | Ethyl | Methyl | t-Butyl | 127-128 | 7-t-Butyl-4-ethyl-2-(4-methoxybenzyl)-6-methyl-imidazo[4.5-e]-s-triazolo[1.5-a]pyrimidin-5-on |
| 17 | 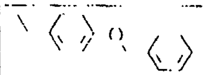 | - | Ethyl | H | t-Butyl | 245-246 | 2-(4-Benzyloxy-benzyl)-7-t-butyl-4-ethyl-imidazo[4.5-e]-s-triazolo[1.5-a]pyrimidin-5-on |
| 18 | 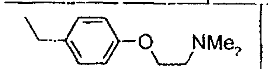 | - | Ethyl | H | t.-Butyl | 204 Zers. | 7-t-Butyl-2-{4-[2-(dimethylamino)-ethyloxy]-benzyl}-4-ethyl-imidazo[4.5-e]-s-triazolo[1.5-a]pyrimidin-5-on |
| 19 | 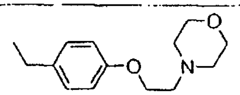 | - | Ethyl | H | t.-Butyl | 228-230 | 7-t-Butyl-4-ethyl-2-{4-[2-(N-morpholino)-ethyloxy]-benzyl}-imidazo[4.5-e]-s-triazolo[1.5-a]pyrimidin-5-on |
| 20 | 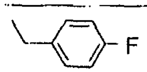 | - | Ethyl | H | 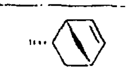 | 255-257 | 4-Ethyl-2-(4-fluorbenzyl)-7-(5-norbornen-2(*S*)-yl)-imidazo[4.5-e]-s-triazolo[1.5-a]pyrimidin-5-on |
| 21 | $-CH_2-NH_2$ | - | Ethyl | H | Cyclopentyl | 240-245 | 2-Aminomethyl-7-cyclopentyl-4-ethyl-imidazo[4.5-e]-s-triazolo[1.5-a] pyrimidin-5-on |
| 22 | $-CH_2-NHMe$ | - | Ethyl | H | Cyclopentyl | 172-175 | 7-Cyclopentyl-4-ethyl-2-(methylaminomethyl)-imidazo[4.5-e]-s-triazolo[1.5-a] pyrimidin-5-on |
| 23 | $-CH_2-N(i-Pr)_2$ | - | Ethyl | H | t-Butyl | 262-264 | 7-t-Butyl-4-ethyl-2-(diisopropylamino-methyl)-imidazo[4.5-e]-s-triazolo[1.5-a]pyrimidin-5-on |
| 24 | 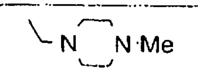 | - | Ethyl | H | t-Butyl | 283 Zers. | 7-t-Butyl-4-ethyl-2-[(4-methylpiperazin-1-yl)methyl]-imidazo[4.5-e]-s-triazolo[1.5-a]pyrimidin-5-on |

| Nr. | $R^1$ | $R^2$ | $R^3$ | $R^4$ (oder $R^6$) | $R^5$ | Schmp. [°C] | Bezeichnung |
|---|---|---|---|---|---|---|---|
| 25 | $-CH_2-NHAc$ | - | Ethyl | H | Cyclopentyl | 269-271 | 2-(N-Acetylaminomethyl)-7-cyclopentyl-4-ethyl-imidazo[4.5-e]-s-triazolo[1.5-a]pyrimidin-5-on |
| 26 | $-CH_2CH_2-NH_2$ | - | Ethyl | H | Cyclopentyl | 272-275 | 2-(2-Aminoethyl)-7-cyclopentyl-4-ethyl-imidazo[4.5-e]-s-triazolo[1.5-a]pyrimidin-5-on |
| 27 | $-CH_2-OH$ | - | Ethyl | H | t-Butyl | 236 | 7-t-Butyl-4-ethyl-2-hydroxymethyl-imidazo[4.5-e]-s-triazolo[1.5-a]pyrimidin-5-on |
| 28 | -CH(OH)Me | - | Ethyl | H | Cyclopentyl | 264-266 | 7-Cyclopentyl-4-ethyl-2-(1-hydroxyethyl)-imidazo[4.5-e]-s-triazolo[1.5-a]pyrimidin-5-on |
| 29 | $-CH_2-OPhenyl$ | - | Ethyl | H | Cyclopentyl | 229 | 7-Cyclopentyl-4-ethyl-2-phenyloxymethyl-imidazo[4.5-e]-s-triazolo[1.5-a]pyrimidin-5-on |
| 30 | -COOH | - | Ethyl | H | Cyclopentyl | >300 | 2-Carboxy-7-cyclopentyl-4-ethyl-imidazo[4.5-e]-s-triazolo[1.5-a]pyrimidin-5-on |
| 31 | COO-iPropyl | - | Ethyl | H | t-Butyl | 298-302 | 7-t.-Butyl-2-carboxyisopropyl-4-ethyl-imidazo[4.5-e]-s-triazolo[1.5-a]pyrimidin-5-on |
| 32 | COO-iPropyl | - | Ethyl | H | Cyclopentyl | 304-305 | 2-Carboxyisopropyl-7-cyclopentyl-4-ethyl-imidazo[4.5-e]-s-triazolo[1.5-a]pyrimidin-5-on |
| 33 | COO-t-Butyl | - | Ethyl | H | t-Butyl | >300 | 7-t.-Butyl-2-(carboxy-tert.butyl)-4-ethyl-imidazo[4.5-e]-s-triazolo[1.5-a]pyrimidin-5-on |

| Nr. | $R^1$ | $R^2$ | $R^3$ | $R^4$ (oder $R^6$) | $R^5$ | Schmp. [°C] | Bezeichnung |
|---|---|---|---|---|---|---|---|
| 34 | COO-t-Butyl | - | Ethyl | H | Cyclopentyl | >300 | 2-(Carboxy-tert.butyl)-7-cyclopentyl-4-ethyl-imidazo[4.5-e]-s-triazolo[1.5-a]pyrimidin-5-on |
| 35 | 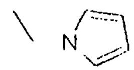 | - | Ethyl | H | Cyclopentyl | 282-283 | 7-Cyclopentyl-4-ethyl-2-(N-pyrrolylmethyl)-imidazo[4.5-e]-s-triazolo[1.5-a]pyrimidin-5-on |
| 36 | 2-Furanyl | - | n-Propyl | H | Methyl | >300 | 2-(2-Furanyl)-7-methyl-4-n-propyl-imidazo[4.5-e]-s-triazolo[1.5-a]pyrimidin-5-on |
| 37 | 2-Furanyl | - | n-Propyl | H | n-Propyl | >300 | 2-(2-Furanyl)-4,7-di-n-propyl-imidazo[4.5-e]-s-triazolo[1.5-a]pyrimidin-5-on |
| 38 | Cyclopentyl | - | Allyl | H | Ethyl | 231-232 | 2-Cyclopentyl-7-ethyl-4-(1-propen-3-yl)-imidazo [4.5-e]-s-triazolo[1.5-a]pyrimidin-5-on |
| 39 | Cyclopentyl | - | Propargyl | H | Ethyl | 274 | 2-Cyclopentyl-7-ethyl-4-(1-propin-3-yl)-imidazo [4.5-e]-s-triazolo[1.5-a]pyrimidin-5-on |
| 40 | Cyclopentyl | - | 2-Methoxy-ethyl | H | Ethyl | 220 | 2-Cyclopentyl-7-ethyl-4-(2-methoxyethyl)-imidazo [4.5-e]-s-triazolo[1.5-a]pyrimidin-5-on |
| 41 | Cyclopentyl | - | Cyclopropyl-methyl | H | Ethyl | - | 2-Cyclopentyl-4-cyclopropylmethyl-7-ethyl-imidazo [4.5-e]-s-triazolo[1.5-a]pyrimidin-5-on |
| 42 | Cyclopentyl | - | n-Butyl | H | Methyl | 260 | 4-n-Butyl-2-cyclopentyl-7-methyl-imidazo [4.5-e]-s-triazolo[1.5-a]pyrimidin-5-on |

| Nr. | $R^1$ | $R^2$ | $R^3$ | $R^4$ (oder $R^6$) | $R^5$ | Schmp. [°C] | Bezeichnung |
|---|---|---|---|---|---|---|---|
| 43 | Cyclopentyl | - | 2-Hydroxy-ethyl | H | Ethyl | 285 | 2-Cyclopentyl-7-ethyl-4-(2-hydroxyethyl)-imidazo [4.5-e]-s-triazolo[1.5-a]pyrimidin-5-on |
| 44 | Cyclopentyl | - | 2-Methoxy-ethyl | H | Methyl | 212-214 | 2-Cyclopentyl-4-(2-methoxyethyl)-7-methyl-imidazo [4.5-e]-s-triazolo[1.5-a]pyrimidin-5-on |

Die nachfolgend beschriebenen Rezeptorbindungswerte wurden in Analogie zu Ensinger et al. in "Cloning and functional characterisation of human $A_1$ adenosine Receptor - Biochemical and Biophysical Communications, Vol 187, No. 2, 919-926, 1992" bestimmt.

Die nachfolgende Tabelle enthält $KiA_1$ (human) Rezeptorbindungswerte.

Tabelle 2:

| Beispiel- Nr.: | $K_iA_1$ [nM] |
|---|---|
| 11 | 8,1 |
| 12 | 6,0 |
| 15 | 2,1 |
| 29 | 3,6 |
| 35 | 2,6 |

Die nachstehend genannten $A_3$-Rezeptorbindungswerte wurden in Analogie zu Salvatore et al. "Molecular cloning and characterization of the human $A_3$-adenosine receptor" (Proc. Natl. Acad. Sci. USA 90, 10365-10369, 1993) ermittelt.
Tabelle 3 enthält $KiA_3$ (human) Rezeptorbindungswerte.

Tabelle 3:

| Beispiel Nr. | $K_iA_3$ [nM] |
|---|---|
| 2 | 5,6 |
| 8 | 0,9 |
| 10 | 7,2 |
| 36 | 2,2 |
| 37 | 8,3 |

Die neuen Verbindungen der allgemeinen Formel (Ia) bis (Id) können oral, transdermal, inhalativ oder parenteral verabreicht werden. Die erfindungsgemäßen Verbindungen liegen hierbei als aktive Bestandteile in üblichen Darreichungsformen vor. beispielsweise in Zusammensetzungen, die im wesentlichen aus einem inerten pharmazeutischen Träger und einer effektiven Dosis des Wirkstoffs bestehen, wie beispielsweise Tabletten, Dragees, Kapseln, Oblaten, Pulver, Lösungen, Suspensionen, Emulsionen, Sirupe. Suppositorien, transdermale Systeme etc.. Eine wirksame Dosis der erfindungsgemäßen Verbindungen liegt bei einer oralen Anwendung zwischen 1 und 100, vorzugsweise zwischen 1 und 50, besonders bevorzugt zwischen 5-30 mg/Dosis, bei intravenöser oder intramuskulärer Anwendung zwischen 0,001 und 50, vorzugsweise zwischen 0,1 und 10 mg/Dosis. Für die Inhalation sind erfindungsgemäß Lösungen geeignet, die 0,01 bis 1,0, vorzugsweise 0,1 bis 0,5 % Wirkstoff enthalten. Für die inhalative Applikation ist die Verwendung von Pulvern bevorzugt. Gleichfalls ist es möglich, die erfindungsgemäßen Verbindungen als Infusionslösung, vorzugsweise in einer physiologischen Kochsafzlösung oder Nährsalzlösung einzusetzen.

Die Verbindungen der allgemeinen Formel (I) können allein oder in Kombination mit anderen erfindungsgemäßen Wirkstoffen, gegebenenfalls auch in Kombination mit weiteren pharmakologisch aktiven Wirkstoffen, zur Anwendung gelangen. Geeignete Anwendungsformen sind beispielsweise Tabletten, Kapseln, Zäpfchen, Lösungen, Säfte, Emulsionen oder dispersible Pulver. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt. enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate. enthalten.

Injektionslösungen werden in üblicher Weise, z.B. unter Zusatz von Konservierungsmitteln, wie p-Hydroxybenzoaten, oder Stabilisatoren, wie Alkalisalzen der Ethylendiamintetraessigsäure hergestellt und in Injektionsflaschen. oder Ampullen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Eine therapeutisch wirksame Tagesdosis beträgt zwischen 1 und 800 mg, bevorzugt 10 - 300 mg pro Erwachsener.

Die nachfolgenden Beispiele illustrieren die vorliegende Erfindung ohne sie jedoch in ihrem Umfang zu beschränken:

Pharmazeutische Formulierungsbeispiele

A)

| Tabletten | pro Tablette |
|---|---|
| Wirkstoff | 100 mg |
| Milchzucker | 140 mg |
| Maisstärke | 240 mg |
| Polyvinylpyrrolidon | 15 mg |
| Magnesiumstearat | 5 mg |
| | 500 mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpreßt.

B)

| Tabletten | pro Tablette |
|---|---|
| Wirkstoff | 80 mg |
| Maisstärke | 190 mg |
| Milchzucker | 55 mg |
| Mikrokristalline Cellulose | 35 mg |
| Polyvinylpyrrolidon | 15 mg |
| Natrium-carboxymethylstärke | 23 mg |
| Magnesiumstearat | 2 mg |
| | 400 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natrium-carboxymethylstärke und das Magnesiumstearat, vermischt und verpreßt das Gemisch zu Tabletten geeigneter Größe.

C)

| Dragées | pro Dragée |
|---|---|
| Wirkstoff | 5 mg |
| Maisstärke | 41,5 mg |
| Milchzucker | 30 mg |
| Polyvinylpyrrolidon | 3 mg |
| Magnesiumstearat | 0.5 mg |
| | 80 mg |

Der Wirkstoff, Maisstärke, Milchzucker und Polyvinylpyrrolidon werden gut gemischt und mit Wasser befeuchtet. Die feuchte Masse drückt man durch ein Sieb mit 1 mm-Maschenweite, trocknet bei ca. 45°C und schlägt das Granulat anschließend durch dasselbe Sieb. Nach dem Zumischen von Magnesiumstearat werden auf einer Tablettiermaschine gewölbte Dragéekerne mit einem Durchmesser von 6 mm gepreßt. Die so hergestellten Dragéekerne werden auf bekannte Weise mit einer Schicht überzogen, die im wesentlichten aus Zucker und Talkum besteht. Die fertigen Dragées werden mit Wachs poliert.

D)

| Kapseln | pro Kapsel |
|---|---|
| Wirkstoff | 50 mg |
| Maisstärke | 268,5 mg |
| Magnesiumstearat | 1,5 mg |
| | 320 mg |

Substanz und Maisstärke werden gemischt und mit Wasser befeuchtet. Die feuchte Masse wird gesiebt und getrocknet. Das trockene Granulat wird gesiebt und mit Magensiumstearat gemischt. Die Endmischung wird in Hartgelatinekapseln Größe 1 abgefüllt.

E)

| Ampullenlösung | |
|---|---|
| Wirkstoff | 50 mg |
| Natriumchlorid | 50 mg |
| Aqua pro inj. | 5 ml |

Der Wirkstoff wird bei Eigen-pH oder gegebenenfalls bei pH 5,5 bis 6,5 in Wasser gelöst und mit Natriumchlorid als Isotonans versetzt. die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

F)

| Suppositorien | |
|---|---|
| Wirkstoff | 50 mg |
| Adeps solidus | 1650 mg |
| | 1700 mg |

Das Hartfett wird geschmolzen. Bei 40°C wird die gemahlene Wirksubstanz homogen dispergiert. Es wird auf 38°C abgekühlt und in schwach vorgekühlte Suppositorienformen ausgegossen.

G)

| orale Suspension | |
|---|---|
| Wirkstoff | 50 mg |
| Hydroxyethylcellulose | 50 mg |
| Sorbinsäure | 5 mg |
| Sorbit (70%ig) | 600 mg |
| Glycerin | 200 mg |
| Aroma | 15 mg |
| Wasser ad | 5 ml |

Destilliertes Wasser wird auf 70°C erhitzt. Hierin wird unter Rühren Hydroxyethylcellulose gelöst. Nach Zugabe von Sorbitlösung und Glycerin wird auf Raumtemperatur abgekühlt. Bei Raumtemperatur werden Sorbinsäure, Aroma und Substanz zugegeben. Zur Entlüftung der Suspension wird unter Rühren evakuiert.

## Patentansprüche

1. Verbindungen der allgemeinen Formel (I)

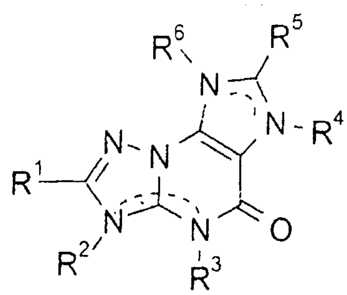

(I)

worin die gestrichelten Linien zwischen den Stickstoffatomen der zuvor genannten allgemeinen Formel I die Existenz einer Doppelbindung in einer von zwei möglichen Positionen beschreiben, sodaß die Reste $R^4$ und $R^6$ beziehungsweise $R^3$ und $R^2$ nicht gleichzeitig vorhanden sein können und
worin

$R^1$ Wasserstoff, ein $C_1$-$C_8$-Alkyl-, $C_2$-$C_8$-Alkenyl- oder $C_2$-$C_8$-Alkinyl-Rest, der gegebenenfalls ein oder mehrfach durch -CN, $-CH_2NR^7R^8$, $-COOR^9$, $-OONR^7R^8$, -CHO, $-COR^{10}$, $-CH(OH)R^{10}$, $-CH(OR^9)_2$, $-CH=CH-R^{11}$, -CH=NOH, $-CH=NOR^9$, $-NR^7R^8$, $-NHCOR^9$, $-NHCONR^7R^8$, $-NHCOOR^9$, -NHCONHPhenyl, $-OR^9$, $-OCOR^9$, -OCOPyridyl, $-OCH_2COOR^9$, $-OCH_2-CONR^7R^8$, $-OCH_2-CH_2-NR^7R^8$, $-OCH_2CH_2OR^9$, $-OCH_2-CH_2OCOR^9$, $OCONR^7R^8$, Halogen, gegebenenfalls durch Methyl-substituiertes 1,3-Dioxolan oder 1,3-Dioxan substituiert sein kann, oder

$R^1$ -CHO, $-COOR^9$, $-CONR^7R^8$ oder $NR^7R^8$, oder

$R^1$ $C_3$-$C_7$-Cycloalkyl, bevorzugt Cyclopentyl oder Cyclohexyl, das gegebenenfalls durch = O, $-OR^9$, $OCOR^9$ oder -OCOPyridyl substituiert sein kann, oder

$R^1$ Phenyl, das gegebenenfalls durch $C_1$-$C_4$-Alkyl, bevorzugt Methyl, -CN, $-COOR^9$, $NR^7R^8$, $-OR^9$, $-OCH_2COOR^9$, $-OCH_2CONR^7R^8$, $-SO_3H$, oder Halogen substituiert sein kann, oder

$R^1$ Phenyl-$C_1$-$C_6$-alkyl-, bevorzugt Phenyl-$C_1$-$C_4$-alkyl-, Phenyl-$C_2$-$C_6$-alkenyl- oder Phenyl-$C_2$-$C_6$-alkinyl-, wobei der Phenylring gegebenenfalls entweder direkt oder über eine Alkylenbrücke mit 1 bis 4 C-Atomen substituiert sein kann durch einen oder mehrere der Reste $C_1$-$C_3$-Alkyl, -CN, $-CH_2OCOR^9$, $-COOR^9$, $-CF_3$, $-CONR^7R^8$. $-CH_2OR^9$, -CHO, $-CH=NOR^9$, $-COR^{10}$, $-CH(OH)R^{10}$, $-CH(OR^9)_2$. $-CH=CH-R^{11}$, $-CH_2-O-CONR^7R^8$, $-CH_2-CH_2-O-CONR^7R^8$, $-CO-R^9$, -CO-$C_1$-$C_4$-Al-

kyl-$NR^7R^8$, $NR^7R^8$, $-NO_2$, $-NHCOR^9$, $-NHCONR^7R^8$, $-NHCOOR^9$, $-OR^9$, $-OCOR^9$, -OCOPyridyl, $-OCH_2COOR^9$, $-OCH_2-CONR^7R^8$, $-OCH_2-CH_2-NR^7R^8$, $-OCH_2CH_2OR^9$, $-OCH_2-CH_2OCOR^9$, $-OCONR^7R^8$, Halogen, gegebenenfalls durch Methyl substituiertes 1,3-Dioxolan oder 1,3-Dioxan, oder

$R^1$ $C_3$-$C_7$-Cycloalkyl-$C_1$-$C_6$-alkyl-, $C_3$-$C_7$-Cycloalkyl-$C_2$-$C_6$-alkenyl-, $C_3$-$C_7$-Cycloalkyl-$C_2$-$C_6$-alkinyl-, wobei der Cycloalkylrest gegebenenfalls entweder direkt oder über eine Alkylenbrücke mit 1 bis 4 C-Atomen substituiert sein kann durch einen oder mehrere der Reste $C_1$-$C_3$-Alkyl, -CN, $-CH_2OCOR^9$, $-COOR^9$, $-CF_3$, $-CONR^7R^8$, $-CH_2OR^9$, -CHO, $-CH{=}NOR^9$, $-COR^{10}$, $-CH(OH)R^{10}$, $-CH(OR^9)_2$, $-CH{=}CH-R^{11}$, $-CH_2-O-CONR^7R^8$, $-CH_2-CH_2-O-CONR^7R^8$, $-CO-R^9$, -CO-$C_1$-$C_4$-Alkyl-$NR^7R^8$, $NR^7R^8$, $-NO_2$, $-NHCOR^9$, $-NHCONR^7R^8$, $-NHCOOR^9$, $-OR^9$, $-OCOR^9$, -OCOPyridyl, $-OCH_2COOR^9$, $-OCH_2-CONR^7R^8$, $-OCH_2-CH_2-NR^7R^8$, $-OCH_2CH_2OR^9$, $-OCH_2-CH_2OCOR^9$, $-OCONR^7R^8$, Halogen, gegebenenfalls durch Methyl substituiertes 1,3-Dioxolan oder 1,3-Dioxan, oder

$R^1$ ein Norbornan-, Norbornen-, $C_3$-$C_6$-Dicycloalkyl-methyl-, bevorzugt Dicyclopropylmethyl-, Adamantan- oder Noradamantan-Rest, der gegebenenfalls durch $C_1$-$C_4$-Alkyl, bevorzugt Methyl substituiert sein kann, oder

$R^1$ einen Rest der Formel A-$C_1$-$C_6$-Alkyl-, A-CONH-$C_1$-$C_6$-Alkyl-, A-CONH-$C_2$-$C_6$-Alkenyl-, A-CONH-$C_2$-$C_6$-Alkinyl-, A-NH-CO-$C_1$-$C_6$-Alkyl-, A-NH-CO-$C_2$-$C_6$-Alkenyl-, A-NH-CO-$C_2$-$C_6$-Alkinyl-, A-$C_2$-$C_6$-Alkenyl-, A-$C_2$-$C_6$-Alkinyl oder A-, wobei A ein C- oder N-verknüpfter 5, 6 oder 7-gliedriger Heterocyclus ist, der ein oder mehrere Heteroatome aus der Gruppe Stickstoff, Sauerstoff oder Schwefel enthält und gegebenenfalls ein oder mehrfach, bevorzugt einfach, durch Benzyl, gegebenenfalls Methoxy-substituiertes Benzyl, $C_1$-$C_4$-Alkyl, -CN, $-CH_2NR^7R^8$, $-COOR^9$, $-CONR^7R^8$, $-COR^{10}$, $-NO_2$, $-NH_2$, $-OR^9$, =O, ein Ketal, Ethylenketal, $-SO_3H$, $-SO_2-R^9$ oder Halogen, substituiert sein kann;

$R^2$ oder $R^3$ ein $C_1$-$C_8$-Alkyl-, $C_2$-$C_8$-Alkenyl- oder $C_2$-$C_8$-Alkinyl-Rest, der gegebenenfalls ein oder mehrfach durch -CN, $-CH_2NR^7R^8$, $-COOR^9$, $-CONR^7R^8$, -CHO, $-COR^{10}$, $-CH(OH)R^{10}$, $-CH(OR^9)_2$, $-CH{=}CH-R^{11}$, -CH=NOH, $-CH{=}NOR^9$, $-NR^7R^8$, $-NHCOR^9$, $-NHCONR^7R^8$, $-NHCOOR^9$, -NHCONHPhenyl, $-OR^9$, $-OCOR^9$, -OCOPyridyl, $-OCH_2COOR^9$, $-OCH_2-CONR^7R^8$, $-OCH_2-CH_2-NR^7R^8$, $-OCH_2CH_2OR^9$, $-OCH_2-CH_2OCOR^9$, $OCONR^7R^8$, Halogen, gegebenenfalls durch Methyl-substituiertes 1,3-Dioxolan oder 1,3-Dioxan substituiert sein kann, oder

$R^2$ oder $R^3$ Phenyl-$C_1$-$C_6$-alkyl-, bevorzugt Phenyl-$C_1$-$C_4$-alkyl-, Phenyl-$C_2$-$C_6$-alkenyl- oder Phenyl-$C_2$-$C_6$-alkinyl-, wobei der Phenylring gegebenenfalls entweder direkt oder über eine Alkylenbrücke mit 1 bis 4 C-Atomen substituiert sein kann durch einen oder mehrere der Reste $C_1$-$C_3$-Alkyl, -CN, $-CH_2OCOR^9$, $-COOR^9$, $-CF_3$, $-CONR^7R^8$, $-CH_2OR^9$, -CHO, $-CH{=}NOR^9$, $-COR^{10}$, $-CH(OH)R^{10}$, $-CH(OR^9)_2$, $-CH{=}CH-R^{11}$, $-CH_2-O-CONR^7R^8$, $-CH_2-CH_2-O-CONR^7R^8$, $-CO-R^9$, -CO-$C_1$-$C_4$-Alkyl-$NR^7R^8$, $NR^7R^8$, $-NO_2$, $-NHCOR^9$, $-NHCONR^7R^8$, $-NHCOOR^9$, $-OR^9$, $-OCOR^9$, -OCOPyridyl, $-OCH_2COOR^9$, $-OCH_2-CONR^7R^8$, $-OCH_2-CH_2-NR^7R^8$, $-OCH_2CH_2OR^9$, $-OCH_2-CH_2OCOR^9$, $-OCONR^7R^8$, Halogen, gegebenenfalls durch Methyl substituiertes 1,3-Dioxolan oder 1,3-Dioxan, oder

$R^2$ oder $R^3$ einen Rest der Formel A-$C_1$-$C_6$-Alkyl-, A-CONH-$C_1$-$C_6$-Alkyl-, A-CONH-$C_2$-$C_6$-Alkenyl-, A-CONH-$C_2$-$C_6$-Alkinyl-, A-NH-CO-$C_1$-$C_6$-Alkyl-, A-NH-CO-$C_2$-$C_6$-Alkenyl- , A-NH-CO-$C_2$-$C_6$-Alkinyl-, A-$C_2$-$C_6$-Alkenyl-, A-$C_2$-$C_6$-Alkinyl oder A-, wobei A ein C- oder N-verknüpfter 5, 6 oder 7-gliedriger Heterocyclus ist, der ein oder mehrere Heteroatome aus der Gruppe Stickstoff, Sauerstoff oder Schwefel enthält und gegebenenfalls ein oder mehrfach, bevorzugt einfach durch Benzyl, gegebenenfalls Methoxy-substituiertes Benzyl, $C_1$-$C_4$-Alkyl, -CN, $-CH_2NR^7R^8$, $-COOR^9$, $-CONR^7R^8$, $-COR^{10}$, $-NO_2$, $-NH_2$, $-OR^9$, =O, ein Ketal, Ethylenketal, $-SO_3H$, $-SO_2-R^9$ oder Halogen, substituiert sein kann;

$R^4$ oder $R^6$ Wasserstoff, $C_1$-$C_4$-Alkyl, das gegebenenfalls durch $-NR^7R^8$ substituiert sein kann, Benzyl, bevorzugt Wasserstoff;

$R^5$ Wasserstoff, ein $C_1$-$C_8$-Alkyl-, $C_2$-$C_8$-Alkenyl- oder $C_2$-$C_8$-Alkinyl-Rest, der gegebenenfalls ein oder

mehrfach durch -CN, $-CH_2NR^7R^8$, $-COOR^9$, $-CONR^7R^8$, -CHO, $-COR^{10}$, $-CH(OH)R^{10}$, $-CH(OR^9)_2$, $-CH=CH-R^{11}$, -CH=NOH, $-CH=NOR^9$, $-NR^7R^8$, $-NHCOR^9$, $-NHCONR^7R^8$, $-NHCOOR^9$, -NHCON-HPhenyl, $-OR^9$, $-OCOR^9$, -OCOPyridyl, $-OCH_2COOR^9$, $-OCH_2-CONR^7R^8$, $-OCH_2-CH_2-NR^7R^8$, $-OCH_2CH_2OR^9$, $-OCH_2-CH_2OCOR^9$, $OCONR^7R^8$, Halogen, gegebenenfalls durch Methyl-substituiertes 1,3-Dioxolan oder 1,3-Dioxan substituiert sein kann, oder

$R^5$ $C_3$ - $C_7$-Cycloalkyl, bevorzugt Cyclopentyl oder Cyclohexyl, das gegebenenfalls durch = O, -OH, $-OR^9$, $OCOR^9$ oder -OCOPyridyl substituiert sein kann, oder

$R^5$ Phenyl, das gegebenenfalls durch -OH, Halogen, $-OR^9$, $C_1$-$C_4$-Alkyl, - bevorzugt $-CH_3$-, $-NH_2$, -COOH, $-SO_3H$, $-COOR^9$, $-OCH_2COOR^9$, -CN oder $-OCH_2CONR^7R^8$ substituiert sein kann, oder

$R^5$ Phenyl-$C_1$-$C_6$-alkyl-, bevorzugt Phenyl-$C_1$-$C_4$-alkyl-, Phenyl-$C_2$-$C_6$-alkenyl- oder Phenyl-$C_2$-$C_6$-alkinyl-, wobei der Phenylring gegebenenfalls entweder direkt oder über eine Alkylenbrücke mit 1 bis 4 C-Atomen substituiert sein kann durch einen oder mehrere der Reste $C_1$-$C_3$-Alkyl, -CN, $-CH_2OCOR^9$, $-COOR^9$, $-CF_3$, $-CONR^7R^8$, $-CH_2OR^9$, -CHO, $-CH=NOR^9$, $-COR^{10}$, $-CH(OH)R^{10}$, $-CH(OR^9)_2$, $-CH=CH-R^{11}$, $-CH_2-O-CONR^7R^8$, $-CH_2-CH_2-O-CONR^7R^8$, $-CO-R^9$, -CO-$C_1$-$C_4$-Alkyl-$NR^7R^8$, $NR^7R^8$, $-NO_2$, $-NHCOR^9$, $-NHCONR^7R^8$, $-NHCOOR^9$, $-OR^9$, $-OCOR^9$, -OCOPyridyl, $-OCH_2COOR^9$, $-OCH_2-CONR^7R^8$, $-OCH_2-CH_2-NR^7R^8$, $-OCH_2CH_2OR^9$, $-OCH_2-CH_2OCOR^9$, $-OCONR^7R^8$, Halogen, gegebenenfalls durch Methyl substituiertes 1,3-Dioxolan oder 1,3-Dioxan, oder

$R^5$ ein Norbornan-, Norbornen-, $C_3$-$C_6$-Dicycloalkyl-methyl-, bevorzugt Dicyclopropylmethyl-, Adamantan- oder Noradamantan-Rest, der gegebenenfalls durch $C_1$-$C_4$-Alkyl, bevorzugt Methyl substituiert sein kann, oder

$R^5$ -CHO, $-COOR^9$, $-CONR^7R^8$ oder $NR^7R^8$, oder

$R^5$ $C_3$-$C_7$-Cycloalkyl-$C_1$-$C_6$-alkyl-, $C_3$-$C_7$-Cycloalkyl-$C_2$-$C_6$-alkenyl-, $C_3$-$C_7$-Cycloalkyl-$C_2$-$C_6$-alkinyl-, wobei der Cycloalkylrest gegebenenfalls entweder direkt oder über eine Alkylenbrücke mit 1 bis 4 C-Atomen substituiert sein kann durch einen oder mehrere der Reste $C_1$-$C_3$-Alkyl, -CN, $-CH_2OCOR^9$, $-COOR^9$, $-CF_3$, $-CONR^7R^8$, $-CH_2OR^9$, -CHO, $-CH=NOR^9$, $-COR^{10}$, $-CH(OH)R^{10}$, $-CH(OR^9)_2$, $-CH=CH-R^{11}$, $-CH_2-O-CONR^7R^8$, $-CH_2-CH_2-O-CONR^7R^8$, $-CO-R^9$, -CO-$C_1$-$C_4$-Alkyl-$NR^7R^8$, $NR^7R^8$, $-NO_2$, $-NHCOR^9$, $-NHCONR^7R^8$, $-NHCOOR^9$, $-OR^9$, $-OCOR^9$, -OCOPyridyl, $-OCH_2COOR^9$, $-OCH_2-CONR^7R^8$, $-OCH_2-CH_2-NR^7R^8$, $-OCH_2CH_2OR^9$, $-OCH_2-CH_2OOOR^9$, $-OCONR^7R^8$, Halogen, gegebenenfalls durch Methyl substituiertes 1,3-Dioxolan oder 1,3-Dioxan, oder

$R^5$ einen Rest der Formel A-$C_1$-$C_6$-Alkyl-, A-CONH-$C_1$-$C_6$-Alkyl-, A-CONH-$C_2$-$C_6$-Alkenyl-, A-CONH-$C_2$-$C_6$-Alkinyl-, A-NH-CO-$C_1$-$C_6$-Alkyl, A-NH-CO-$C_2$-$C_6$-Alkenyl, A-NH-CO-$C_2$-$C_6$-Alkinyl, A-$C_2$-$C_6$-Alkenylen, A-$C_2$-$C_6$-Alkinylen oder A-, wobei A ein C- oder N-verknüpfter 5, 6 oder 7-gliedriger Heterocyclus ist, der ein oder mehrere Heteroatome aus der Gruppe Stickstoff, Sauerstoff oder Schwefel enthält und gegebenenfalls ein oder mehrfach, bevorzugt einfach, durch Benzyl, gegebenenfalls Methoxy-substituiertes Benzyl, $C_1$-$C_4$-Alkyl, -CN, $-CH_2NR^7R^8$, $-COOR^9$, $-CONR^7R^8$, $-COR^{10}$, $-NO_2$, $-NH_2$, $-OR^9$, =O, ein Ketal, Ethylenketal, $-SO_3H$, $-SO_2-R^9$ oder Halogen, substituiert sein kann;

$R^7$ Wasserstoff, $C_1$-$C_8$-Alkyl, bevorzugt $C_1$-$C_4$-Alkyl, das gegebenenfalls durch $-COOR^9$, $-COR^{10}$, $-OR^9$, $-OCOR^9$, Amino, Phenyl, Methoxy-substituiertes Phenyl oder Amino substituiert sein kann, oder ein $C_3$-$C_6$-Cycloalkyl, oder

$R^7$ ein direkt oder über eine Alkylkette mit 1 bis 4 C-Atomen C-verknüpfter 5, 6 oder 7-gliedriger Heterocyclus, der ein, zwei oder 3 Heteroatome aus der Gruppe Stickstoff, Sauerstoff oder Schwefel enthält und gegebenenfalls ein oder mehrfach, bevorzugt einfach, durch Benzyl, Methoxy-substituiertes Benzyl, $C_1$-$C_4$-Alkyl, Halogen, $-OR^9$, -CN, $-NO_2$, $-NH_2$, =O, $-SO_3H$ oder $-COOR^9$ substituiert sein kann;

$R^8$ Wasserstoff, $C_1$-$C_8$-Alkyl, bevorzugt $C_1$-$C_4$-Alkyl, das gegebenenfalls durch $-COOR^9$, $-COR^{10}$,

$-OR^9$, $-OCOR^9$, Amino, Phenyl, Methoxy-substituiertes Phenyl oder Amino substituiert sein kann, oder ein $C_3$-$C_6$-Cycloalkyl, oder

$R^8$ ein direkt oder über eine Alkylkette mit 1 bis 4 C-Atomen C-verknüpfter 5, 6 oder 7-gliedriger Heterocyclus, der ein, zwei oder 3 Heteroatome aus der Gruppe Stickstoff, Sauerstoff oder Schwefel enthält und gegebenenfalls ein oder mehrfach, bevorzugt einfach, durch Benzyl, Methoxy-substituiertes Benzyl, $C_1$-$C_4$-Alkyl, Halogen, $-OR^9$, -CN, $-NO_2$, $-NH_2$, =O, $-SO_3H$ oder $-COOR^9$ substituiert sein kann;
oder
$R^7$ und $R^8$ bilden zusammen mit dem Stickstoffatom einen gesättigten oder ungesättigten 5- oder 6-Ring, der als weitere Heteroatome Stickstoff, Sauerstoff oder Schwefel enthalten kann, wobei der Heterocyclus durch eine verzweigte oder unverzweigte Alkylgruppe mit 1 bis 4 Kohlenstoffatomen, bevorzugt Methyl substituiert sein kann oder einen der Reste -CN, $-COOR^9$, $-CONH_2$, $-NO_2$, $-NH_2$, $-OR^9$, $-SO_3H$, $-SO_2-R^9$, Halogen, oder $-(CH_2)_n$-Phenyl, $-(CH_2)_n-NH_2$, = O, $-O-CH_2-CH_2-O-$, $-O-CH_2-CH_2-CH_2-O-$, $-(CH_2)_nNH-C_1$-$C_4$-Alkyl, $-(CH_2)_n-N(C_1$-$C_4$-Alkyl$)_2$, $-(CH_2)_n-NHCOOR^9$, wobei n = 1, 2, 3 oder 4 bedeutet, tragen kann;

$R^9$ Wasserstoff, $C_1$-$C_4$-Alkyl, $C_2$-$C_4$-Alkenyl, $C_2$-$C_4$-Alkinyl, einen Benzyl- oder Phenyl-Rest, der gegebenenfalls ein- oder mehrfach durch $OCH_3$ substituiert sein kann;

$R^{10}$ $C_1$-$C_4$-Alkyl, $C_2$-$C_4$-Alkenyl, $C_2$-$C_4$-Alkinyl, gegebenenfalls Methoxy--substituiertes Phenyl, gegebenenfalls Methoxy-substituiertes Benzyl, $C_3$-$C_6$-Cycloalkyl;

$R^{11}$ Wasserstoff, $C_1$-$C_3$-Alkyl, $-COOR^9$, $-CH_2OR^9$, $-CH_2NR^7R^8$, $-CONR^7R^8$ oder gegebenenfalls Methoxy-substituiertes Phenyl, bedeuten können, gegebenenfalls in Form ihrer Racemate, ihrer Enantiomere, ihrer Diastereomere und ihrer Gemische, sowie gegebenenfalls ihrer pharmakologisch unbedenklichen Säureadditionssalze.

**2.** Verbindungen der allgemeinen Formel (I) gemäß Anspruch 1, worin

$R^1$ Wasserstoff, $C_1$-$C_8$-Alkyl, das gegebenenfalls durch -COOH, $-COO-C_1$-$C_4$-Alkyl, -COO-Phenyl, -COO-Benzyl, $-CONR^7R^8$, $-CO-C_1$-$C_4$-Alkyl, -CHO, $NR^7R^8$, $-NHCO-C_1$-$C_4$-Alkyl, -NHCO-Phenyl, Hydroxy, $C_1$-$C_4$-Alkyloxy, Phenyloxy, -O-Phenyl-Methoxy, Benzyloxy, -O-Benzyl-Methoxy, $-OCO-C_1$-$C_4$-Alkyl, -OCO-Phenyl, -OCO-Benzyl, -OCO-Pyridyl, $-O-C_2$-$C_4$-Alkenyl, oder Halogen, substituiert sein kann, oder

$R^1$ -CHO, -COOH, $-COO-C_1$-$C_4$-Alkyl, -COO-Phenyl, -COO-Benzyl, $-CONR^7R^8$; oder ein Amin der allgemeinen Formel $NR^7R^8$, oder

$R^1$ Phenyl, welches gegebenenfalls durch $C_1$-$C_4$-Alkyl, -CH=NOH, -COOH, $-COO-C_1$-$C_4$-Alkyl, -COO-Phenyl, -COO-Benzyl, $NR^7R^8$, Hydroxy, $C_1$-$C_4$-Alkyloxy, Benzyloxy, Phenyloxy, $-OCO-C_1$-$C_4$-Alkyl, -OCO-Phenyl, -OCO-Benzyl, -OCO-Pyridyl, $-O-C_2$-$C_4$-Alkenyl oder Halogen, bevorzugt Chlor oder Fluor, substituiert sein kann, oder

$R^1$ ein $C_3$-$C_6$-Cycloalkyl- oder $C_3$-$C_6$-Cycloalkyl-$C_1$-$C_4$-Alkyl-Rest, der gegebenenfalls durch Hydroxy, $C_1$-$C_3$-Alkyl oder $C_1$-$C_3$-Alkyloxy substituiert sein kann, oder

$R^1$ ein Norbornan-, Norbornen-, Adamantan- oder Noradamantan-Rest, der gegebenenfalls durch $C_1$-$C_4$-Alkyl, bevorzugt Methyl substituiert sein kann, oder

$R^1$ Phenyl-$C_1$-$C_4$-alkyl-, bevorzugt Benzyl, Phenyl-$C_2$-$C_6$-alkenyl- oder Phenyl-$C_2$-$C_6$-alkinyl-, wobei der Phenylring gegebenenfalls durch $C_1$-$C_3$-Alkyl, $-CO-C_1$-$C_4$-Alkyl, $-C_1$-$C_4$-Alkyl-$NR^7R^8$, -COOH, $-COO-C_1$-$C_4$-Alkyl, -COO-Phenyl, -COO-Benzyl, $-CONR^7R^8$, $-CO-C_1$-$C_4$-Alkyl-$NR^7R^8$, $NR^7R^8$, Hydroxy, $C_1$-$C_4$-Alkyloxy, Benzyloxy, Phenyloxy, $-OCO-C_1$-$C_4$-Alkyl, -OCO-Phenyl, -OCO-Benzyl, -OCO-Pyridyl, $-O-C_2$-$C_4$-Alkenyl oder Halogen, bevorzugt Chlor oder Fluor, substituiert sein kann, oder

$R^1$ ein gegebenenfalls entweder direkt oder über eine Alkylenbrücke mit 1 bis 4 C-Atomen C- oder N-verknüpfter 5 oder 6-gliedriger Heterocyclus, der ein, zwei oder drei Heteroatome aus der Gruppe Stickstoff, Sauerstoff oder Schwefel enthält und gegebenenfalls ein oder mehrfach durch Benzyl oder $C_1$-$C_4$-Alkyl substituiert sein kann;

$R^2$ oder $R^3$ $C_1$-$C_8$-Alkyl, das gegebenenfalls durch $NR^7R^8$, OH, $C_1$-$C_4$-Alkyloxy, oder COOH substituiert sein kann, $C_2$-$C_8$-Alkenyl, Phenyl-$C_1$-$C_4$-alkyl, bevorzugt Benzyl, Phenyl-$C_2$-$C_6$-alkenyl oder Phenyl-$C_2$-$C_6$-alkinyl, wobei der Phenylring gegebenenfalls durch Hydroxy, $C_1$-$C_3$-Alkyl, $C_1$-$C_4$-Alkyloxy, $NR^7R^8$ oder Halogen, bevorzugt Chlor oder Fluor, substituiert sein kann, oder

$R^2$ oder $R^3$ ein gegebenenfalls entweder direkt oder über eine Alkylenbrücke mit 1 bis 4 C-Atomen C- oder N-verknüpfter 5 oder 6-gliedriger Heterocyclus, der ein, zwei oder drei Heteroatome aus der Gruppe Stickstoff, Sauerstoff oder Schwefel enthält und gegebenenfalls ein oder mehrfach durch Benzyl oder $C_1$-$C_4$-Alkyl substituiert sein kann;

$R^4$ oder $R^6$ Wasserstoff, $C_1$-$C_4$-Alkyl, das gegebenenfalls durch $NR^7R^8$ substituiert sein kann, Benzyl;

$R^5$ Wasserstoff, $C_1$-$C_8$-Alkyl, bevorzugt $C_1$-$C_6$-Alkyl, welches gegebenenfalls durch Halogen substituiert sein kann, oder

$R^5$ Phenyl, das gegebenenfalls durch $C_1$-$C_4$-Alkyl, $C_1$-$C_4$-Alkyloxy, Hydroxy, oder Halogen, bevorzugt Fluor oder Chlor, substituiert sein kann, oder

$R^5$ Phenyl-$C_1$-$C_6$-alkyl-, bevorzugt Benzyl, wobei der Phenylring gegebenenfalls durch $C_1$-$C_3$-Alkyl, $C_1$-$C_4$-Alkyloxy, Hydroxy, $NR^7R^8$ oder Halogen, bevorzugt Fluor oder Chlor, substituiert sein kann, oder

$R^5$ ein Amin der Formel $NR^7R^8$, oder

$R^5$ ein gegebenenfalls entweder direkt oder über eine Alkylenbrücke mit 1 bis 4 C-Atomen C- oder N-verknüpfter 5 oder 6-gliedriger Heterocyclus, der ein oder mehrere Heteroatome aus der Gruppe Stickstoff oder Sauerstoff enthält und gegebenenfalls durch Benzyl oder $C_1$-$C_4$-Alkyl substituiert sein kann, oder

$R^5$ ein $C_3$-$C_6$-Cycloalkyl welches gegebenenfalls durch =O, Hydroxy, oder $C_1$-$C_4$-Alkyloxy substituiert sein kann, oder

$R^5$ gegebenenfalls durch $C_1$-$C_4$-Alkyl, bevorzugt Methyl substituiertes Norbornan, Norbornen, Adamantan oder Noradamantan;

$R^7$ Wasserstoff, eine verzweigte oder unverzweigte Alkylgruppe mit 1 - 4 - Kohlenstoffatomen, oder

$R^7$ ein C-verknüpfter 5 oder 6-gliedriger Heterocyclus, der ein, zwei oder drei Heteroatome aus der Gruppe Stickstoff, Sauerstoff oder Schwefel enthält und gegebenenfalls durch Benzyl, $C_1$-$C_4$-Alkyl, $C_1$-$C_4$-Alkyloxy, Halogen, -CN, $-NO_2$, $-NH_2$, -OH oder =O substituiert sein kann;

$R^8$ Wasserstoff, eine verzweigte oder unverzweigte Alkylgruppe mit 1 - 4 - Kohlenstoffatomen, oder

$R^8$ ein C-verknüpfter 5 oder 6-gliedriger Heterocyclus, der ein, zwei oder drei Heteroatome aus der Gruppe Stickstoff, Sauerstoff oder Schwefel enthält und gegebenenfalls durch Benzyl, $C_1$-$C_4$-Alkyl, $C_1$-$C_4$-Alkyloxy, Halogen, -CN, $-NO_2$, $-NH_2$, -OH oder =O substituiert sein kann;
oder
$R^7$ und $R^8$ bilden zusammen mit dem Stickstoffatom einen gesättigten oder ungesättigten 5- oder 6-Ring, der als weitere Heteroatome Stickstoff oder Sauerstoff enthalten kann, wobei der Heterocyclus durch eine verzweigte oder unverzweigte Alkylgruppe mit 1 bis 4 Kohlenstoffatomen, bevorzugt Methyl, oder durch einen Rest $-(CH_2)_{1-4}$-Phenyl, bevorzugt Benzyl substituiert sein kann, bedeuten können, gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

**3.** Verbindungen der allgemeinen Formel (I) gemäß Anspruch 1 oder 2, worin

$R^1$ Wasserstoff, $C_1$-$C_6$-Alkyl, bevorzugt $C_1$-$C_4$-Alkyl, das gegebenenfalls durch -CO-$C_1$-$C_4$-Alkyl, -CHO, -COOH, -COO-$C_1$-$C_4$-Alkyl, -COO-Phenyl, -COO-Benzyl, -CONR$^7$R$^8$, NR$^7$R$^8$, -NHCO-$C_1$-$C_4$-Alkyl, -NHCO-Phenyl, Hydroxy, =O, $C_1$-$C_4$-Alkyloxy, Phenyloxy, -O-Phenyl-O- Methoxy, Benzyloxy, -O-Benzyl-O-Methoxy, -OCO-$C_1$-$C_4$-Alkyl, -OCO-Phenyl, -OCO-Pyridyl, -OCO-Benzyl, -O-$C_2$-$C_4$-Alkenyl oder Halogen substituiert sein kann, oder

$R^1$ Phenyl, wobei der Phenylring durch $C_1$-$C_4$-Alkyl, Hydroxy, $C_1$-$C_4$-Alkyloxy, NR$^7$R$^8$, Halogen, bevorzugt Fluor oder Chlor, substituiert sein kann, oder

$R^1$ Phenyl-$C_1$-$C_3$-Alkyl, bevorzugt Benzyl, wobei der Phenylring durch $C_1$-$C_3$-Alkyl, -CO-$C_1$-$C_4$-Alkyl, -$C_1$-$C_4$-Alkyl-NR$^7$R$^8$, -COOH, -COO-$C_1$-$C_4$-Alkyl, -COO-Phenyl, -COO-Benzyl, -CONR$^7$R$^8$, -CO-$C_1$-$C_4$-Alkyl-NR$^7$R$^8$, NR$^7$R$^8$, Hydroxy, $C_1$-$C_4$-Alkyloxy, Benzyloxy, Phenyloxy, -OCO-$C_1$-$C_4$-Alkyl, -OCO-Phenyl, -OCO-Benzyl, -OCO-Pyridyl, -O-$C_2$-$C_4$-Alkenyl oder Halogen, bevorzugt Fluor oder Chlor, substituiert sein kann, oder

$R^1$ ein über eine Einfachbindung oder über eine Alkylenkette mit 1 bis 4 C-Atomen verknüpftes Cyclopentyl, Cyclohexyl, Hydroxycyclopentan oder Hydroxycyclohexan, oder

$R^1$ -CHO, -COOH, -COO-$C_1$-$C_4$-Alkyl, -COO-Phenyl, -COO-Benzyl, -CO-NH-$C_1$-$C_4$-Alkyl, -CO-N($C_1$-$C_4$-Alkyl)$_2$ oder -CO-NH-Phenyl, oder

$R^1$ ein Amin der allgemeinen Formel NR$^7$R$^8$, oder

$R^1$ ein über eine Einfachbindung oder über eine Alkylenkette mit 1 bis 4 C-Atomen verknüpftes Furan, Tetrahydrofuran, $\alpha$-Pyran, $\gamma$-Pyran, Dioxolan, Tetrahydropyran, Dioxan, Thiophen, Thiolan, Dithiolan, Pyrrol, Pyrrolin, Pyrrolidin, Pyrazol, Pyrazolin, imidazol, Imidazolin; Imidazolidin, Triazol, Pyridin, Piperidin, Pyridazin, Pyrimidin, Pyrazin, Piperazin, Triazin, Morpholin, Thiomorpholin, Oxazol, Isoxazol, Oxazin, Thiazol, Isothiazol, Thiadiazol, Oxadiazol oder Pyrazolidin;

$R^2$ oder $R^3$ $C_1$-$C_7$-Alkyl, bevorzugt $C_1$-$C_5$-Alkyl, das gegebenenfalls durch NR$^7$R$^8$, OH, $C_1$-$C_4$-Alkyloxy, oder COOH substituiert sein kann, $C_2$-$C_5$-Alkenyl, $C_2$-$C_5$-Alkinyl, Phenyl-$C_1$-$C_3$-alkyl, bevorzugt Benzyl, wobei der Phenylring durch $C_1$-$C_3$-Alkyl, NR$^7$R$^8$, Hydroxy, $C_1$-$C_4$-Alkyloxy oder Halogen, bevorzugt Chlor oder Fluor, substituiert sein kann, oder

$R^2$ oder $R^3$ ein über eine Einfachbindung oder über eine Alkylenkette mit 1 bis 4 C-Atomen verknüpftes Furan, Tetrahydrofuran, $\alpha$-Pyran, $\gamma$-Pyran, Dioxolan, Tetrahydropyran, Dioxan, Thiophen, Thiolan, Dithiolan, Pyrrol, Pyrrolin, Pyrrolidin, Pyrazol, Pyrazolin, Imidazol, Imidazolin, Imidazolidin, Triazol, Pyridin, Piperidin, Pyridazin, Pyrimidin, Pyrazin, Piperazin, Triazin, Morpholin, Thiomorpholin, Oxazol, Isoxazol, Oxazin, Thiazol, Isothiazol, Thiadiazol, Oxadiazol oder Pyrazolidin;

$R^4$ oder $R^6$ Wasserstoff, $C_1$-$C_4$-Alkyl, bevorzugt $C_1$-$C_3$-Alkyl, $C_1$-$C_4$-Alkyl-NR$^7$R$^8$, Benzyl;

$R^5$ Wasserstoff, $C_1$-$C_6$-Alkyl, bevorzugt $C_1$-$C_4$-Alkyl, welches gegebenenfalls durch Halogen substituiert sein kann, oder

$R^5$ Cyclopentyl, Cyclohexyl, Cyclopentanon, Cyclohexanon, Hydroxycyclopentan oder Hydroxycyclohexan, oder

$R^5$ Pyridyl, Tetrahydrofuranyl, Tetrahydropyranyl, Furyl oder ein gegebenenfalls durch $C_1$-$C_4$-Alkyl oder Benzyl substituierter Morpholin-, Piperidin- oder Piperazinrest, oder

$R^5$ ein Phenylrest, der gegebenenfalls durch $C_1$-$C_4$-Alkyl, Halogen oder Hydroxy substituiert sein kann,

oder

$R^5$ ein Phenyl-$C_1$-$C_3$-Alkyl, bevorzugt Benzyl, wobei der Phenylring gegebenenfalls durch $C_1$-$C_3$-Alkyl, $NR^7R^8$, Hydroxy, $C_1$-$C_4$-Alkyloxy oder Halogen, bevorzugt Fluor oder Chlor,substituiert sein kann, oder

$R^5$ ein Amin der allgemeinen Formel $NR^7R^8$, oder

$R^5$ ein Norbornen-, Norbornan-, Adamantan- oder Noradamantan-Rest, der gegebenenfalls durch $C_1$-$C_4$-Alkyl, bevorzugt Methyl substituiert sein kann;

$R^7$ Wasserstoff, eine verzweigte oder unverzweigte Alkylgruppe mit 1 - 4 - Kohlenstoffatomen;

$R^7$ ein gegebenenfalls durch $C_1$-$C_4$-Alkyl, $NO_2$, $NH_2$, Hydroxy, $C_1$-$C_4$-Alkyloxy, Chlor oder Brom substituiertes C-verknüpftes Pyrrol, Pyrrolidin, Pyrazol, Imidazol, Imidazolidin, Triazol, Pyridin, Piperidin, Pyrimidin, Pyrazin, Piperazin, Morpholin, Oxazol, Isoxazol, Thiazol, Isothiazol oder Thiadiazol;

$R^8$ Wasserstoff, eine verzweigte oder unverzweigte Alkylgruppe mit 1 - 4 - Kohlenstoffatomen;

$R^8$ ein gegebenenfalls durch $C_1$-$C_4$-Alkyl, $NO_2$, $NH_2$, Hydroxy, $C_1$-$C_4$-Alkyloxy, Chlor oder Brom substituiertes C-verknüpftes Pyrrol, Pyrrolidin, Pyrazol, Imidazol, Imidazolidin, Triazol, Pyridin, Piperidin, Pyrimidin, Pyrazin, Piperazin, Morpholin, Oxazol, Isoxazol, Thiazol, Isothiazol oder Thiadiazol; oder
$R^7$ und $R^8$ bilden zusammen mit dem Stickstoffatom einen gesättigten oder ungesättigten 5- oder 6-Ring, der als weitere Heteroatome Stickstoff oder Sauerstoff enthalten kann, wobei der Heterocyclus durch eine verzweigte oder unverzweigte Alkylgruppe mit 1 bis 4 Kohlenstoffatomen, bevorzugt Methyl, oder durch einen Rest $-(CH_2)_{1-4}$-Phenyl, bevorzugt Benzyl substituiert sein kann, bedeuten können, gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

**4.** Verbindungen der allgemeinen Formel (I) gemäß einem der Ansprüche 1 bis 3, worin

$R^1$ Wasserstoff, $C_1$-$C_4$-Alkyl, welches gegebenenfalls durch -NHCO-$C_1$-$C_4$-Alkyl, $NR^7R^8$, Hydroxy, $C_1$-$C_4$-Alkyloxy, Fluor, Chlor oder Brom substituiert sein kann, -CHO, -COOH, -COO-$C_1$-$C_4$-Alkyl, Phenyl, Phenyl-$C_1$-$C_3$-Alkyl-, bevorzugt Benzyl, das gegebenenfalls durch $C_1$-$C_4$-Alkyl, $C_1$-$C_4$-Alkyloxy, Hydroxy, Benzyloxy oder Fluor substituiert sein kann, Phenyloxy-$C_1$-$C_3$-Alkyl-, bevorzugt Phenyloxymethyl, das gegebenenfalls durch Methoxy substituiert sein kann, Benzyloxy-$C_1$-$C_3$-Alkyl-, bevorzugt Benzyloxymethyl, das gegebenenfalls durch Methoxy substituiert sein kann, Benzoyloxymethyl, Pyridylcarbonyloxymethyl, Cyclohexylmethyl, Pyridylmethyl, Pyrrolylmethyl, Morpholinomethyl, Cyclopentyl oder Furyl;

$R^2$ oder $R^3$ $C_1$-$C_5$-Alkyl, das gegebenenfalls durch Hydroxy, Methoxy oder Cyclopropyl substituiert sein kann, $C_2$-$C_4$-Alkenyl, $C_2$-$C_4$-Alkinyl oder Benzyl;

$R^4$ oder $R^6$ Wasserstoff, $C_1$-$C_3$-Alkyl, $C_1$-$C_3$-Alkyl-$NR^7R^8$, N-Morpholinoethyl oder Benzyl;

$R^5$ Wasserstoff, $C_1$-$C_4$-Alkyl, welches gegebenenfalls durch Fluor substituiert sein kann, Phenyl, Benzyl, wobei der Phenylring gegebenenfalls durch Fluor substituiert sein kann, Pyridyl, Piperidinyl, Morpholinyl, Piperazinyl, 4-Benzylpiperazinyl, Furyl, Tetrahydrofuranyl, Tetrahydropyranyl, $NR^7R^8$, Cyclopentyl, Cyclohexyl, Adamantyl, Noradamantyl, Norbomyl oder Norbornenyl;

$R^7$ Wasserstoff, $C_1$-$C_4$-Alkyl oder Pyridyl;

$R^8$ Wasserstoff, $C_1$-$C_4$-Alkyl oder Pyridyl bedeuten können, gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

**5.** Verbindungen der allgemeinen Formel (I) gemäß einem der Ansprüche 1-4, worin

$R^1$ Wasserstoff, Methyl, das gegebenenfalls durch $-NH_2$, -NHMe, $-NMe_2$, -N(i-Propyl)$_2$, -NHAcetyl, Hydroxy, Methoxy, Ethoxy, Phenyloxy, Methoxyphenyloxy, Methoxybenzyloxy, Morpholin, Benzoyloxy, Pyridylcarbonyloxy, Pyridin, Pyridylamino, Methylpiperazin oder Pyrrol substituiert sein kann, Ethyl, das gegebenenfalls durch $NH_2$ oder Hydroxy substituiert sein kann, n-Propyl, 1-Propyl, n-Butyl, tert. -Butyl, Cyclopentyl, Cyclohexylmethyl, Benzyl, das gegebenenfalls durch Hydroxy, Methoxy, Benzyloxy, Dimethylaminoethoxy, N-Morpholinoethoxy oder Fluor substituiert sein kann, Phenyl, Phenylethyl, -COOH, -COOMethyl, -COOPropyl oder -COOButyl;

$R^2$oder $R^3$ Methyl, das gegebenenfalls durch Cyclopropyl substituiert sein kann, Ethyl, das gegebenenfalls durch Hydroxy oder Methoxy substituiert sein kann, n-Propyl, i-Propyl, n-Butyl, i-Butyl, tert.-Butyl, Butenyl, n-Pentyl, Allyl oder Propargyl;

$R^4$oder $R^6$ Wasserstoff, Methyl oder Ethyl, das gegebenenfalls durch Morpholin substituiert sein kann;

$R^5$ Wasserstoff, Methyl, Ethyl, n-Propyl, i-Propyl, n-Butyl, tert-Butyl, Phenyl, Benzyl, Pyridin, Piperidin, Morpholin, Piperazin, 4-Benzylpiperazinyl, Tetrahydrofuranyl, Tetrahydropyranyl, $-NMe_2$, Cyclopentyl, Cyclohexyl, Adamantyl, Noradamantyl, Nornbornanyl oder Norbornenyl bedeuten können, gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

**6.** Verbindungen der allgemeinen Formel (I) gemäß einem der Ansprüche 1 bis 5, worin

$R^1$ Methyl, das gegebenenfalls durch $-NH_2$, -NHMe, -N(i-Propyl)$_2$, -NHAcetyl, Hydroxy, Phenyloxy, Methylpiperazin oder Pyrrol substituiert sein kann, Ethyl, das gegebenenfalls durch $NH_2$ oder Hydroxy substituiert sein kann, Cyclopentyl, Benzyl, das gegebenenfalls durch Hydroxy, Methoxy, Benzyloxy, Dimethylaminoethoxy, N-Morpholinoethoxy oder Fluor substituiert sein kann, Phenylethyl, -COOH, -COOPropyl oder -COOButyl;

$R^2$oder $R^3$ Methyl, das gegebenenfalls durch Cyclopropyl substituiert sein kann, Ethyl, das gegebenenfalls durch Hydroxy oder Methoxy substituiert sein kann, n-Propyl, i-Propyl, n-Butyl, i-Butyl, Butenyl, n-Pentyl, Allyl oder Propargyl;

$R^4$ oder $R^6$ Wasserstoff, Methyl oder Ethyl, das gegebenenfalls durch Morpholin substituiert sein kann;

$R^5$ Methyl, Ethyl, n-Propyl, tert-Butyl, Cyclopentyl oder Norbornenyl bedeuten können gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

**7.** Verbindungen der allgemeinen Formel (I) gemäß einem der Ansprüche 1 bis 6, worin

$R^1$ Methyl, das gegebenenfalls durch Phenyloxy oder Pyrrol substituiert sein kann, Benzyl, das gegebenenfalls durch Hydroxy, Methoxy, Dimethylaminoethoxy oder Fluor substituiert sein kann, Cyclopentyl oder Phenylethyl;

$R^2$oder $R^3$ Ethyl, n-Propyl, Allyl oder Propargyl;

$R^4$oder $R^6$ Wasserstoff;

$R^5$ Methyl, Ethyl, n-Propyl, tert-Butyl, Cyclopentyl oder Norbornenyl bedeuten können gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

**8.** Verbindungen der allgemeinen Formel (I) gemäß einem der Ansprüche 1 bis 7, **dadurch gekennzeichnet, daß**

sie als Isomere der Formeln (Ia) oder (Ib)

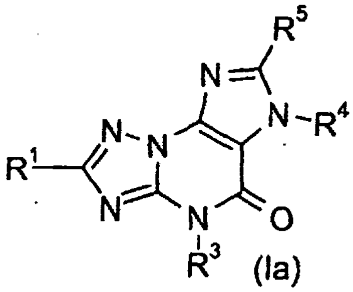

(Ia)

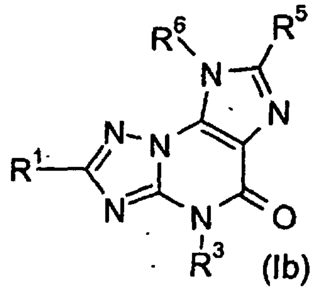

(Ib)

vorliegen.

**9.** Verbindungen der allgemeinen Formel (I) gemäß einem der Ansprüche 1 bis 8, **dadurch gekennzeichnet, daß** sie als Isomere der Formel (Ib)

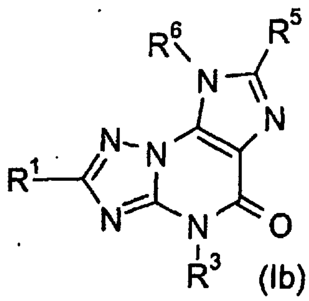

(Ib)

vorliegen.

**10.** Verbindungen der allgemeinen Formel (I) gemäß einem der Ansprüche 1 bis 8, **dadurch gekennzeichnet, daß** sie als Isomere der Formel (Ia)

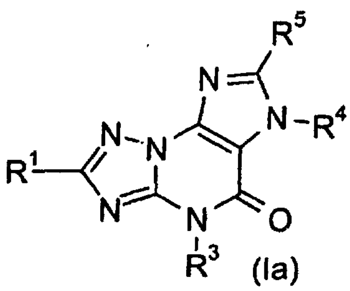

(Ia)

vorliegen.

**11.** Verbindung der allgemeinen Formel (I) gemäß einem der Anspruche 1 bis 7, ausgewählt aus der Gruppe:

2-Cyclopentyl-4,7-di-n-propyl-imidazo[4.5-e]-s-triazolo[1.5-a]pyrimidin-5-on;
2-Benzyl-7-methyl-4-n-propyl-imidazo[4.5-e]-s-triazolo[1.5-a]pyrimidin-5-on;
4-Ethyl-7-methyl-2-(2-phenylethyl)-imidazo[4.5-e]-s-triazolo[1.5-a]pyrimidin-5-on;

4-Ethyl-2-(2-phenylethyl)-7-n-propyl-imidazo[4.5-e]-s-triazolo[1.5-a]pyrimidin-5-on;
7-t-Butyl-4-ethyl-2-(4-hydroxybenzyl)-imidazo[4.5-e]-s-triazolo[1.5-a]pyrimidin-5-on;
7-t-Butyl-4-ethyl-2-(4-methoxybenzyl)-imidazo[4.5-e]-s-triazolo[1.5-a]pyrimidin-5-on;
7-Cyclopentyl-4-ethyl-2-phenyloxymethyl-imidazo[4.5-e]-s-triazolo[1.5-a]pyrimidin-5-on;
7-Cyclopentyl-4-ethyl-2-(N-pyrrolylmethyl)-imidazo[4.5-e]-s-triazolo[1,5-a]pyrimidin-5-on;
2-(2-Furanyl)-7-methyl-4-n-propyl-imidazo[4.5-e]-s-triazolo[1.5-a]pyrimidin-5-on;
2-(2-Furanyl)-4,7-di-n-propyl-imidazo[4.5-e]-s-triazolo[1.5-a]pyrimidin-5-on;
2-Cyclopentyl-7-ethyl-4-(1-propen-3-yl)-imidazo [4.5-e]-s-triazolo[1.5-a]pyrimidin-5-on oder
2-Cyclopentyl-7-ethyl-4-(1-propin-3-yl)-imidazo [4.5-e]-s-triazolo[1.5-a]pyrimidin-5-on, gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

12. Verwendung einer Verbindung gemäß einem der Ansprüche 1 bis 11 zur Herstellung eines Arzneimittels mit adenosinantagonistischer Wirkung.

13. Pharmazeutische Zubereitungen, enthaltend als Wirkstoff eine oder mehrere Verbindungen gemäß einem der Ansprüche 1 bis 11 oder deren physiologisch verträgliche Säureadditionssalze in Kombination mit üblichen Hilfs- und/oder Trägerstoffen.

## Claims

1. Compounds of general formula (I)

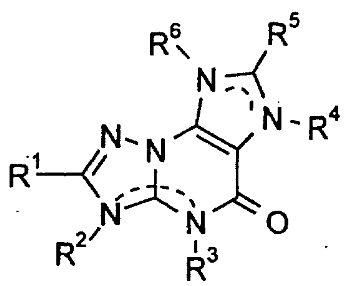

(I)

wherein the dotted lines between the nitrogen atoms of the above general formula I indicate the existence of a double bond in one of two possible positions, so that the groups $R^4$ and $R^6$ and $R^3$ and $R^2$ need not be present simultaneously and wherein

$R^1$ denotes hydrogen, a $C_{1-8}$-alkyl, $C_{2-8}$-alkenyl or $C_{2-8}$-alkynyl group which may optionally be mono- or polysubstituted by -CN, $-CH_2NR^7R^8$, $-COOR^9$, $-CONR^7R^8$, -CHO, $-COR^{10}$, $-CH(OH)R^{10}$, $-CH(OR^9)_2$, $-CH=CH-R^{11}$, -CH=NOH, $-CH=NOR^9$, $-NR^7R^8$, $-NHCOR^9$, $-NHCONR^7R^8$, $-NHCOOR^9$, -NHCONHPhenyl, $-OR^9$, $-OCOR^9$, -OCOPyridyl, $-OCH_2COOR^9$, $-OCH_2-CONR^7R^8$, $-OCH_2-CH_2-NR^7R^8$, $-OCH_2CH_2OR^9$, $-OCH_2-CH_2OCOR^9$, $OCONR^7R^8$, halogen, optionally methyl-substituted 1,3-dioxolane or 1,3-dioxane, or

$R^1$ denotes -CHO, $-COOR^9$, $-CONR^7R^8$ or $NR^7R^8$, or

$R^1$ denotes $C_{3-7}$-cycloalkyl, preferably cyclopentyl or cyclohexyl, which may optionally be substituted by =O, $-OR^9$, $OCOR^9$ or -OCOPyridyl, or

$R^1$ denotes phenyl which may optionally be substituted by $C_{1-4}$-alkyl, preferably methyl, -CN, $-COOR^9$, $NR^7R^8$, $-OR^9$, $-OCH_2COOR^9$, $-OCH_2CONR^7R^8$, $-SO_3H$ or halogen, or

$R^1$ denotes phenyl-$C_{1-6}$-alkyl, preferably phenyl-$C_{1-4}$-alkyl, phenyl-$C_{2-6}$-alkenyl or phenyl-$C_{2-6}$-alkynyl, wherein the phenyl ring may optionally be substituted, either directly or via an alkylene bridge having 1 to 4 carbon atoms, by one or more of the groups $C_{1-3}$-alkyl, -CN, $-CH_2OCOR^9$, $-COOR^9$, $-CF_3$, $-CONR^7R^8$, $-CH_2OR^9$, -CHO, $-CH=NOR^9$, $-COR^{10}$, $-CH(OH)R^{10}$, $-CH(OR^9)_2$, $-CH=CH-R^{11}$, $-CH_2-O-CONR^7R^8$, $-CH_2-CH_2-O-CONR^7R^8$, $-CO-R^9$, $-CO-C_{1-4}$-Alkyl-$NR^7R^8$, $NR^7R^8$, $-NO_2$, $-NHCOR^9$, $-NHCONR^7R^8$, $-NHCOOR^9$, $-OR^9$, $-OCOR^9$, -OCOPyridyl, $-OCH_2COOR^9$, $-OCH_2-CONR^7R^8$, $-OCH_2-CH_2-NR^7R^8$, $-OCH_2CH_2OR^9$, $-OCH_2-CH_2OCOR^9$, $-OCONR^7R^8$, halogen, optionally methyl-substituted 1,3-dioxolane or 1,3-dioxane, or

$R^1$ denotes $C_{3-7}$-cycloalkyl-$C_{1-6}$-alkyl, $C_{3-7}$-cycloalkyl-$C_{2-6}$-alkenyl, $C_{3-7}$-cycloalkyl-$C_{2-6}$-alkynyl, wherein the cycloalkyl group may optionally be substituted, either directly or via an alkylene bridge having 1 to 4 carbon atoms, by one or more of the groups $C_{1-3}$-alkyl, -CN, $-CH_2OCOR^9$, $-COOR^9$, $-CF_3$, $-CONR^7R^8$, $-CH_2OR^9$, -CHO,

-CH=NOR$^9$, -COR$^{10}$, -CH(OH)R$^{10}$, -CH(OR$^9$)$_2$, -CH=CH-R$^{11}$, -CH$_2$-O-CONR$^7$R$^8$, -CH$_2$-CH$_2$-O-CONR$^7$R$^8$, -CO-R$^9$, -CO-C$_{1-4}$-Alkyl-NR$^7$R$^8$, NR$^7$R$^8$, -NO$_2$, -NHCOR$^9$, -NHCONR$^7$R$^8$, -NHCOOR$^9$, -OR$^9$, -OCOR$^9$, -OCOPyridyl, -OCH$_2$COOR$^9$, -OCH$_2$-CONR$^7$R$^8$, -OCH$_2$-CH$_2$-NR$^7$R$^8$, -OCH$_2$CH$_2$OR$^9$, -OCH$_2$-CH$_2$OCOR$^9$, -OCONR$^7$R$^8$, halogen, optionally methyl-substituted 1,3-dioxolane or 1,3-dioxane, or

R$^1$ denotes a norbornane, norbornene, C$_{3-6}$-dicycloalkylmethyl, preferably dicyclopropylmethyl, adamantane or noradamantane group which may optionally be substituted by C$_{1-4}$-alkyl, preferably methyl, or

R$^1$ denotes a group of formula A-C$_{1-6}$-alkyl, A-CONH-C$_{1-6}$-alkyl, A-CONH-C$_{2-6}$-alkenyl, A-CONH-C$_{2-6}$-alkynyl, A-NH-CO-C$_{1-6}$-alkyl, A-NH-CO-C$_{2-6}$-alkenyl, A-NH-CO-C$_{2-6}$-alkynyl, A-C$_{2-6}$-alkenyl, A-C$_{2-6}$-alkynyl or A-, where A is a C- or N-linked 5-, 6- or 7- membered heterocycle, which contains one or more heteroatoms selected from among nitrogen, oxygen or sulphur and may optionally be mono- or polysubstituted, preferably monosubstituted, by benzyl, optionally methoxy-substituted benzyl, C$_{1-4}$-alkyl, -CN, -CH$_2$NR$^7$R$^8$, -COOR$^9$, -CONR$^7$R$^8$, -COR$^{10}$, -NO$_2$, -NH$_2$, -OR$^9$, =O, a ketal, ethyleneketal, -SO$_3$H, -SO$_2$-R$^9$ or halogen;

R$^2$ or R$^3$ denotes a C$_{1-8}$-alkyl, C$_{2-8}$-alkenyl or C$_{2-8}$-alkynyl group which may optionally be mono-or polysubstituted by -CN, -CH$_2$NR$^7$R$^8$, -COOR$^9$, -CONR$^7$R$^8$, -CHO, -COR$^{10}$, -CH(OH)R$^{10}$, -CH(OR$^9$)$_2$, -CH=CH-R$^{11}$, -CH=NOH, -CH=NOR$^9$, -NR$^7$R$^8$, -NHCOR$^9$, -NHCONR$^7$R$^8$, -NHCOOR$^9$, -NHCONHPhenyl, -OR$^9$, -OCOR$^9$, -OCOPyridyl, -OCH$_2$COOR$^9$, -OCH$_2$-CONR$^7$R$^8$, -OCH$_2$-CH$_2$-NR$^7$R$^8$, -OCH$_2$CH$_2$OR$^9$, -OCH$_2$-CH$_2$OCOR$^9$, OCONR$^7$R$^8$, halogen, optionally methyl-substituted 1,3-dioxolane or 1,3-dioxane, or

R$^2$ or R$^3$ denotes phenyl-C$_{1-6}$-alkyl, preferably phenyl-C$_{1-4}$-alkyl, phenyl-C$_{2-6}$-alkenyl or phenyl-C$_{2-6}$-alkynyl, wherein the phenyl ring may optionally be substituted, either directly or via an alkylene bridge having 1 to 4 carbon atoms, by one or more of the groups C$_{1-3}$-alkyl, -CN, -CH$_2$OCOR$^9$, -COOR$^9$, -CF$_3$, -CONR$^7$R$^8$, -CH$_2$OR$^9$, -CHO, -CH=NOR$^9$, -COR$^{10}$, -CH(OH)R$^{10}$, -CH(OR$^9$)$_2$, -CH=CH-R$^{11}$, -CH$_2$-O-CONR$^7$R$^8$, -CH$_2$-CH$_2$-O-CONR$^7$R$^8$, -CO-R$^9$, -CO-C$_{1-4}$-alkyl-NR$^7$R$^8$, NR$^7$R$^8$, -NO$_2$, -NHCOR$^9$, -NHCONR$^7$R$^8$, -NHCOOR$^9$, OR$^9$, -OCOR$^9$, -OCOPyridyl, -OCH$_2$COOR$^9$, -OCH$_2$-CONR$^7$R$^8$, -OCH$_2$-CH$_2$-NR$^7$R$^8$, -OCH$_2$CH$_2$OR$^9$, -OCH$_2$-CH$_2$OCOR$^9$, -OCONR$^7$R$^8$, halogen, optionally methyl-substituted 1,3-dioxolane or 1,3-dioxane, or

R$^2$ or R$^3$ denotes a group of formula A-C$_{1-6}$-alkyl, A-CONH-C$_{1-6}$-alkyl, A-CONH-C$_{2-6}$-alkenyl, A-CONH-C$_{2-6}$-alkynyl, A-NH-CO-C$_{1-6}$-alkyl, A-NH-CO-C$_{2-6}$-alkenyl, A-NH-CO-C$_{2-6}$-alkynyl, A-C$_{2-6}$-alkenyl, A-C$_{2-6}$-alkynyl or A-, where A is a C- or N-linked 5-, 6- or 7- membered heterocycle, which contains one or more heteroatoms selected from among nitrogen, oxygen or sulphur and may optionally be mono- or polysubstituted, preferably monosubstituted, by benzyl, optionally methoxy-substituted benzyl, C$_{1-4}$-alkyl, -CN, -CH$_2$NR$^7$R$^8$, -COOR$^9$, -CONR$^7$R$^8$, -COR$^{10}$, -NO$_2$, -NH$_2$, -OR$^9$, =O, a ketal, ethyleneketal, -SO$_3$H, -SO$_2$-R$^9$ or halogen;

R$^4$ or R$^6$ denotes hydrogen, C$_{1-4}$-alkyl which may optionally be substituted by -NR$^7$R$^8$, benzyl, preferably hydrogen;

R$^5$ denotes hydrogen, a C$_{1-8}$-alkyl, C$_{2-8}$-alkenyl or C$_{2-8}$-alkynyl group which may optionally be mono- or polysubstituted by -CN, -CH$_2$NR$^7$R$^8$, -COOR$^9$, -CONR$^7$R$^8$, -CHO, -COR$^{10}$, -CH(OH)R$^{10}$, -CH(OR$^9$)$_2$, -CH=CH-R$^{11}$, -CH=NOH, -CH=NOR$^9$, -NR$^7$R$^8$, -NHCOR$^9$, -NHCONR$^7$R$^8$, -NHCOOR$^9$, -NHCONHPhenyl, -OR$^9$, -OCOR$^9$, -OCOPyridyl, -OCH$_2$COOR$^9$, -OCH$_2$-CONR$^7$R$^8$, -OCH$_2$-CH$_2$-NR$^7$R$^8$, -OCH$_2$CH$_2$OR$^9$, -OCH$_2$-CH$_2$OCOR$^9$, OCONR$^7$R$^8$, halogen, optionally methyl-substituted 1,3-dioxolane or 1,3-dioxane, or

R$^5$ denotes C$_{3-7}$-cycloalkyl, preferably cyclopentyl or cyclohexyl, which may optionally be substituted by =O, -OH, -OR$^9$, OCOR$^9$ or -OCOPyridyl, or

R$^5$ denotes phenyl which may optionally be substituted by -OH, halogen, -OR$^9$, C$_{1-4}$-alkyl, preferably CH$_3$, NH$_2$, COOH, -SO$_3$H, -COOR$^9$, -OCH$_2$COOR$^9$, -CN or -OCH$_2$CONR$^7$R$^8$, or

R$^5$ denotes phenyl-C$_{1-6}$-alkyl, preferably phenyl-C$_{1-4}$-alkyl, phenyl-C$_{2-6}$-alkenyl or phenyl-C$_{2-6}$-alkynyl, wherein the phenyl ring may optionally be substituted, either directly or via an alkylene bridge having 1 to 4 carbon atoms, by one or more of the groups C$_{1-3}$-alkyl, -CN, -CH$_2$OCOR$^9$, -COOR$^9$, -CF$_3$, -CONR$^7$R$^8$, -CH$_2$OR$^9$, -CHO, -CH=NOR$^9$, -COR$^{10}$, -CH(OH)R$^{10}$, -CH(OR$^9$)$_2$, -CH=CH-R$^{11}$, -CH$_2$-O-CONR$^7$R$^8$, -CH$_2$-CH$_2$-O-CONR$^7$R$^8$, -CO-R$^9$, -CO-C$_{1-4}$-alkyl-NR$^7$R$^8$, NR$^7$R$^8$, -NO$_2$, -NHCOR$^9$, -NHCONR$^7$R$^8$, -NHCOOR$^9$, -OR$^9$, -OCOR$^9$, -OCOPyridyl, -OCH$_2$COOR$^9$, -OCH$_2$-CONR$^7$R$^8$, -OCH$_2$-CH$_2$-NR$^7$R$^8$, -OCH$_2$CH$_2$OR$^9$, -OCH$_2$-CH$_2$OCOR$^9$, -OCONR$^7$R$^8$, halogen, optionally methyl-substituted 1,3-dioxolane or 1,3-dioxane, or

R$^5$ denotes a norbornane, norbornene, C$_{3-6}$-dicycloalkylmethyl, preferably dicyclopropylmethyl, adamantane or noradamantane group which may optionally be substituted by C$_{1-4}$-alkyl, preferably methyl, or

R$^5$ denotes -CHO, -COOR$^9$, -CONR$^7$R$^8$ or NR$^7$R$^8$, or

R$^5$ denotes C$_{3-7}$-cycloalkyl-C$_{1-6}$-alkyl, C$_{3-7}$-cycloalkyl-C$_{2-6}$-alkenyl, C$_{3-7}$-cycloalkyl-C$_{2-6}$-alkynyl, wherein the cycloalkyl group may optionally be substituted, either directly or via an alkylene bridge having 1 to 4 carbon atoms, by one or more of the groups C$_{1-3}$-alkyl, -CN, -CH$_2$OCOR$^9$, -COOR$^9$, -CF$_3$, -CONR$^7$R$^8$, -CH$_2$OR$^9$, -CHO, -CH=NOR$^9$, -COR$^{10}$, -CH(OH)R$^{10}$, -CH(OR$^9$)$_2$, -CH=CH-R$^{11}$, -CH$_2$-O-CONR$^7$R$^8$, -CH$_2$-CH$_2$-O-CONR$^7$R$^8$, -CO-R$^9$, -CO-C$_{1-4}$-Alkyl-NR$^7$R$^8$, NR$^7$R$^8$, -NO$_2$, -NHCOR$^9$, -NHCONR$^7$R$^8$, -NHCOOR$^9$, -OCOR$^9$, -OCOPyridyl, -OCH$_2$COOR$^9$, -OCH$_2$-CONR$^7$R$^8$, -OCH$_2$-CH$_2$-NR$^7$R$^8$, -OCH$_2$CH$_2$OR$^9$, -OCH$_2$-CH$_2$OCOR$^9$, -OCONR$^7$R$^8$, halogen, optionally methyl-substituted 1,3-dioxolane or 1,3-dioxane, or

R$^5$ denotes a group of formula A-C$_{1-6}$-alkyl, A-CONH-C$_{1-6}$-alkyl, A-CONH-C$_{2-6}$-alkenyl, A-CONH-C$_{2-6}$-alkynyl,

A-NH-CO-$C_{1-6}$-alkyl, A-NH-CO-$C_{2-6}$-alkenyl, A-NH-CO-$C_{2-6}$-alkynyl, A-$C_{2-6}$-alkenylene, A-$C_{2-6}$-alkynylene or A-, where A is a Cor N-linked 5-, 6- or 7- membered heterocycle, which contains one or more heteroatoms selected from among nitrogen, oxygen or sulphur and may optionally be mono- or polysubstituted, preferably monosubstituted, by benzyl, optionally methoxy-substituted benzyl, $C_{1-4}$-alkyl, -CN, -$CH_2NR^7R^8$, -$COOR^9$, -$CONR^7R^8$, -$COR^{10}$, -$NO_2$, -$NH_2$, -$OR^9$, =O, a ketal, ethyleneketal, -$SO_3H$, -$SO_2$-$R^9$ or halogen;

$R^7$ denotes hydrogen, $C_{1-8}$-alkyl, preferably $C_{1-4}$-alkyl, which may optionally be substituted by -$COOR^9$, -$COR^{10}$, -$OR^9$, $OCOR^9$, amino, phenyl, methoxy-substituted phenyl or amino, or a $C_{3-6}$-cycloalkyl, or

$R^7$ denotes a 5-, 6- or 7- membered heterocycle C-linked directly or via a $C_{1-4}$-alkyl chain, which contains one, two or three heteroatoms selected from among nitrogen, oxygen or sulphur and which is optionally mono- or polysubstituted, preferably monosubstituted, by benzyl, methoxy-substituted benzyl, $C_{1-4}$-alkyl, halogen, -$OR^9$, -CN, -$NO_2$, -$NH_2$, =O, $SO_3H$ or -$COOR^9$;

$R^8$ denotes hydrogen, $C_{1-8}$-alkyl, preferably $C_{1-4}$-alkyl, which may optionally be substituted by -$COOR^9$, -$COR^{10}$, -$OR^9$, $OCOR^9$, amino, phenyl, methoxy-substituted phenyl or amino, or a $C_{3-6}$-cycloalkyl, or

$R^8$ denotes a 5-, 6- or 7- membered heterocycle C-linked directly or via a $C_{1-4}$-alkyl chain, which contains one, two or three heteroatoms selected from among nitrogen, oxygen or sulphur and which is optionally mono- or polysubstituted, preferably monosubstituted, by benzyl, methoxy-substituted benzyl, $C_{1-4}$-alkyl, halogen, -$OR^9$, -CN, -$NO_2$, -$NH_2$, =O, $SO_3H$ or -$COOR^9$;

or

$R^7$ and $R^8$ together with the nitrogen atom form a saturated or unsaturated 5- or 6-membered ring which may contain nitrogen, oxygen or sulphur as further heteroatoms, whilst the heterocycle may be substituted by a branched or unbranched alkyl group having 1 to 4 carbon atoms, preferably methyl, or may carry one of the groups -CN, -$COOR^9$, -$CONH_2$, -$NO_2$, -$NH_2$, -$OR^9$, -$SO_3H$, -$SO_2$-$R^9$, halogen or -$(CH_2)_n$-phenyl, -$(CH_2)_n$-$NH_2$, =O, -$OCH_2$-$CH_2$-O, -$OCH_2$-$CH_2$-$CH_2$-O, -$(CH_2)_n$-NH-$C_{1-4}$-alkyl, -$(CH_2)_n$-N($C_{1-4}$-alkyl$)_2$, -$(CH_2)_n$-$NHCOOR^9$, wherein n=1, 2, 3 or 4;

$R^9$ denotes hydrogen, $C_{1-4}$-alkyl, $C_{2-4}$-alkenyl, $C_{2-4}$-alkynyl, a benzyl or phenyl group which may optionally be mono- or polysubstituted by -$OCH_3$;

$R^{10}$ denotes $C_{1-4}$-alkyl, $C_{2-4}$-alkenyl, $C_{2-4}$-alkynyl, optionally methoxy-substituted phenyl, optionally methoxy-substituted benzyl, $C_{3-6}$-cycloalkyl;

$R^{11}$ denotes hydrogen, $C_{1-3}$-alkyl, -$COOR^9$, -$CH_2OR^9$, -$CH_2NR^7R^8$, -$CONR^7R^8$ or optionally methoxy-substituted phenyl,

optionally in the form of their racemates, their enantiomers, their diastereomers and mixtures thereof, and optionally the pharmacologically acceptable acid addition salts thereof.

**2.** Compounds of general formula (I) according to claim 1, wherein

$R^1$ denotes hydrogen, $C_{1-8}$-alkyl which may optionally be substituted by -COOH, -COO-$C_{1-4}$-alkyl, -COO-phenyl, -COO-benzyl, -$CONR^7R^8$, -CO-$C_{1-4}$-alkyl, -CHO, -$NR^7R^8$, -NHCO-$C_{1-4}$-alkyl, -NHCO-Phenyl, hydroxy, $C_{1-4}$-alkoxy, phenyloxy, -O-phenyl-methoxy, benzyloxy, O-benzyl-methoxy, -OCO-$C_{1-4}$-alkyl, -OCO-phenyl, -OCO-benzyl, -OCO-pyridyl, -O-$C_{2-4}$-alkenyl or halogen, or

$R^1$ denotes -CHO, -COOH, -COO-$C_{1-4}$-alkyl, -COO-phenyl, -COO-benzyl, -$CONR^7R^8$ or an amine of general formula $NR^7R^8$, or

$R^1$ denotes phenyl which may optionally be substituted by $C_{1-4}$-alkyl, -CH=NOH, -COOH, -COO-$C_{1-4}$-alkyl, -COO-phenyl, -COO-benzyl, $NR^7R^8$, hydroxy, $C_{1-4}$-alkyloxy, benzyloxy, phenyloxy, -OCO-$C_{1-4}$-alkyl, -OCO-phenyl, -OCO-benzyl, -OCO-pyridyl, -O-$C_{2-4}$-alkenyl or halogen, preferably chlorine or fluorine, or

$R^1$ denotes a $C_{3-6}$-cycloalkyl or $C_{3-6}$-cycloalkyl-$C_{1-4}$-alkyl group which may optionally be substituted by hydroxy, $C_{1-3}$-alkyl or $C_{1-3}$-alkyloxy, or

$R^1$ denotes a norbornane, norbornene, adamantane or noradamantane group which may optionally be substituted by $C_{1-4}$-alkyl, preferably methyl, or

$R^1$ denotes phenyl-$C_{1-4}$-alkyl, preferably benzyl, phenyl-$C_{2-6}$-alkenyl or phenyl-$C_{2-6}$-alkynyl, wherein the phenyl ring may optionally be substituted by $C_{1-3}$-alkyl, -CO-$C_{1-4}$-alkyl, $C_{1-4}$-alkyl-$NR^7R^8$, -COOH, -COO-$C_{1-4}$-alkyl, -COO-phenyl, -COO-benzyl, -$CONR^7R^8$, -CO-$C_{1-4}$-alkyl-$NR^7R^8$, $NR^7R^8$, hydroxy, $C_{1-4}$-alkyloxy, benzyloxy, phenyloxy, -OCO-$C_{1-4}$-alkyl, -OCO-phenyl, -OCO-benzyl, -OCO-pyridyl, -O-$C_{2-4}$-alkenyl or halogen, preferably chlorine or fluorine, or

$R^1$ denotes a 5- or 6-membered heterocycle optionally C- or N-linked either directly or via a $C_{1-4}$-alkylene bridge, which contains one, two or three heteroatoms selected from among nitrogen, oxygen or sulphur and which is optionally mono- or polysubstituted by benzyl or $C_{1-4}$-alkyl;

$R^2$ or $R^3$ denotes a $C_{1-8}$-alkyl which may optionally be substituted by -$NR^7R^8$, OH, $C_{1-4}$-alkyloxy or COOH, $C_{2-8}$-alkenyl, phenyl-$C_{1-4}$-alkyl, preferably benzyl, phenyl-$C_{2-6}$-alkenyl or phenyl-$C_{2-6}$-alkynyl, whilst the phenyl ring may optionally be substituted by hydroxy, $C_{1-3}$-alkyl, $C_{1-4}$-alkyloxy, -$NR^7R^8$ or halogen, preferably chlorine or fluorine, or

$R^2$ or $R^3$ denotes a 5- or 6-membered heterocycle optionally C-or N-linked either directly or via a $C_{1-4}$-alkylene bridge, which contains one, two or three heteroatoms selected from among nitrogen, oxygen or sulphur and which is optionally mono- or polysubstituted by benzyl or $C_{1-4}$-alkyl;
$R^4$ or $R^6$ denotes hydrogen, $C_{1-4}$-alkyl which may optionally be substituted by $-NR^7R^8$, or benzyl;
$R^5$ denotes hydrogen, $C_{1-8}$-alkyl, preferably $C_{1-6}$-alkyl, which may optionally be substituted by halogen, or
$R^5$ denotes phenyl which may optionally be substituted by $C_{1-4}$-alkyl, $C_{1-4}$-alkyloxy, hydroxy or halogen, preferably fluorine or chlorine, or
$R^5$ denotes phenyl-$C_{1-6}$-alkyl, preferably benzyl, whilst the phenyl ring may optionally be substituted by $C_{1-3}$-alkyl, $C_{1-4}$-alkyloxy, hydroxy, $-NR^7R^8$ or halogen, preferably fluorine or chlorine, or
$R^5$ denotes an amine of formula $-NR^7R^8$, or
$R^5$ denotes a 5- or 6-membered heterocycle optionally C- or N-linked either directly or via a $C_{1-4}$-alkylene bridge, which contains one or more heteroatoms selected from among nitrogen or oxygen and which may optionally be substituted by benzyl or $C_{1-4}$-alkyl; or
$R^5$ denotes a $C_{3-6}$-cycloalkyl which may optionally be substituted by =O, hydroxy or $C_{1-4}$-alkyloxy, or
$R^5$ denotes a norbornane, norbornene, adamantane or noradamantane group which may optionally be substituted by $C_{1-4}$-alkyl, preferably methyl;
$R^7$ denotes hydrogen, a branched or unbranched $C_{1-4}$-alkyl group, or
$R^7$ denotes a C-linked 5- or 6-membered heterocycle which contains one, two or three heteroatoms selected from among nitrogen, oxygen or sulphur and which is optionally substituted by benzyl, $C_{1-4}$-alkyl, $C_{1-4}$-alkyloxy, halogen, -CN, $-NO_2$, $-NH_2$, -OH or =O ;
$R^8$ denotes hydrogen, a branched or unbranched $C_{1-4}$-alkyl group, or
$R^8$ denotes a C-linked 5- or 6-membered heterocycle which contains one, two or three heteroatoms selected from among nitrogen, oxygen or sulphur and which is optionally substituted by benzyl, $C_{1-4}$-alkyl, $C_{1-4}$-alkyloxy, halogen, -CN, $-NO_2$, $-NH_2$, -OH or =O;
or
$R^7$ and $R^8$ together with the nitrogen atom form a saturated or unsaturated 5- or 6-membered ring which may contain nitrogen or oxygen as further heteroatoms, whilst the heterocycle may be substituted by a branched or unbranched alkyl group having 1 to 4 carbon atoms, preferably methyl, or by a $-(CH_2)_{1-4}$-phenyl group, preferably benzyl,
optionally in the form of their racemates, their enantiomers, their diastereomers and mixtures thereof, and optionally the pharmacologically acceptable acid addition salts thereof.

**3.** Compounds of general formula (I) according to claim 1 or 2, wherein
$R^1$ denotes hydrogen, $C_{1-6}$-alkyl, preferably $C_{1-4}$-alkyl, which may optionally be substituted by -CO-$C_{1-4}$-alkyl, -CHO, -COOH, COO-$C_{1-4}$-alkyl, COO-phenyl, -COO-benzyl, $-CONR^7R^8$, $-NR^7R^8$, -NHCO-$C_{1-4}$-alkyl, -NHCO-Phenyl, hydroxy, =O, $C_{1-4}$-alkoxy, phenyloxy, O-phenyl-O-methoxy, benzyloxy, O-benzyl-O-methoxy, -OCO-$C_{1-4}$-alkyl, -OCO-phenyl, -OCO-pyridyl, -OCO-benzyl, -O-$C_{2-4}$-alkenyl or halogen, or
$R^1$ denotes phenyl, whilst the phenyl ring may optionally be substituted by $C_{1-4}$-alkyl, hydroxy, $C_{1-4}$-alkyloxy, $NR^7R^8$, halogen, preferably fluorine or chlorine, or
$R^1$ denotes phenyl-$C_{1-3}$-alkyl, preferably benzyl, whilst the phenyl ring may optionally be substituted by $C_{1-3}$-alkyl, -CO-$C_{1-4}$-alkyl, -$C_{1-4}$-alkyl-$NR^7R^8$, -COOH, -COO-$C_{1-4}$-alkyl, -COO-phenyl, -COO-benzyl, $-CONR^7R^8$, -CO-$C_{1-4}$-alkyl-$NR^7R^8$, $NR^7R^8$, hydroxy, $C_{1-4}$-alkyloxy, benzyloxy, phenyloxy, -OCO-$C_{1-4}$-alkyl, -OCO-phenyl, -OCO-benzyl, -OCO-pyridyl, -O-$C_{2-4}$-alkenyl or halogen, preferably chlorine or fluorine, or
$R^1$ denotes a cyclopentyl, cyclohexyl, hydroxycyclopentane or hydroxycyclohexane linked via a single bond or via an alkylene chain having 1 to 4 carbon atoms, or
$R^1$ denotes -CHO, -COOH, -COO-$C_{1-4}$-alkyl, -COO-phenyl, -COO-benzyl, -CO-NH-$C_{1-4}$-alkyl, -CO-N($C_{1-4}$-alkyl)$_2$ or -CO-NH-phenyl, or
$R^1$ denotes an amine of general formula $NR^7R^8$, or
$R^1$ denotes a furan, tetrahydrofuran, α-pyran, γ-pyran, dioxolane, tetrahydropyran, dioxane, thiophene, thiolane, dithiolane, pyrrole, pyrroline, pyrrolidine, pyrazole, pyrazoline, imidazole, imidazoline, imidazolidine, triazole, pyridine, piperidine, pyridazine, pyrimidine, pyrazine, piperazine, triazine, morpholine, thiomorpholine, oxazole, isoxazole, oxazine, thiazole, isothiazole, thiadiazole, oxadiazole or pyrazolidine linked via a single bond or via an alkylene chain with 1 to 4 carbon atoms;
$R^2$ or $R^3$ denotes $C_{1-7}$-alkyl, preferably $C_{1-5}$-alkyl, which may optionally be substituted by $-NR^7R^8$, OH, $C_{1-4}$-alkyloxy or COOH, $C_{2-5}$-alkenyl, $C_{2-5}$-alkynyl, phenyl-$C_{1-3}$-alkyl, preferably benzyl, whilst the phenyl ring may be substituted by $C_{1-3}$-alkyl, $-NR^7R^8$, hydroxy, $C_{1-4}$-alkyloxy or halogen, preferably chlorine or fluorine, or
$R^2$ or $R^3$ denotes a furan, tetrahydrofuran, α-pyran, Y-pyran, dioxolane, tetrahydropyran, dioxane, thiophene, thiolane, dithiolane, pyrrole, pyrroline, pyrrolidine, pyrazole, pyrazoline, imidazole, imidazoline, imidazolidine, triazole, pyridine, piperidine, pyridazine, pyrimidine, pyrazine, piperazine, triazine, morpholine, thiomorpholine, oxazole, isoxazole, oxazine, thiazole, isothiazole, thiadiazole, oxadiazole or pyrazolidine linked via a single bond or via an alkylene chain with 1 to 4 carbon atoms;
$R^4$ or $R^6$ denotes hydrogen, $C_{1-4}$-alkyl, preferably $C_{1-3}$-alkyl, $C_{1-4}$-alkyl-$NR^7R^8$ or benzyl;
$R^5$ denotes hydrogen, $C_{1-6}$-alkyl, preferably $C_{1-4}$-alkyl, which may optionally be substituted by halogen, or
$R^5$ denotes cyclopentyl, cyclohexyl, cyclopentanone, cyclohexanone, hydroxycyclopentane or hydroxycyclohexane, or
$R^5$ denotes pyridyl, tetrahydrofuranyl, tetrahydropyranyl, furyl or a morpholine, piperidine or piperazine group optionally substituted by $C_{1-4}$-alkyl or benzyl, or
$R^5$ denotes a phenyl group which may optionally be substituted by $C_{1-4}$-alkyl, halogen or hydroxy, or
$R^5$ denotes phenyl-$C_{1-3}$-alkyl, preferably benzyl, whilst the phenyl ring may optionally be substituted by $C_{1-3}$-alkyl, $-NR^7R^8$, hydroxy, $C_{1-4}$-alkyloxy or halogen, preferably chlorine or fluorine, or
$R^5$ denotes an amine of general formula $-NR^7R^8$, or
$R^5$ denotes a norbornene, norbornane, adamantane or noradamantane group which may optionally be substituted by $C_{1-4}$-alkyl, preferably methyl;
$R^7$ denotes hydrogen, a branched or unbranched $C_{1-4}$-alkyl group, or
$R^7$ denotes a C-linked pyrrole, pyrrolidine, pyrazole, imidazole, imidazolidine, triazole, pyridine, piperidine, pyrimidine, pyrazine, piperazine, morpholine, oxazole, isoxazole, thiazole, isothiazole or thiadiazole optionally substituted by $C_{1-4}$-alkyl, $-NO_2$, $-NH_2$, hydroxy, $C_{1-4}$-alkyloxy, chlorine or bromine;
$R^8$ denotes hydrogen, a branched or unbranched $C_{1-4}$-alkyl group, or
$R^8$ denotes a C-linked pyrrole, pyrrolidine, pyrazole, imidazole, imidazolidine, triazole, pyridine, piperidine, pyrimidine, pyrazine, piperazine, morpholine, oxazole, isoxazole, thiazole, isothiazole or thiadiazole optionally substituted by $C_{1-4}$-alkyl, $-NO_2$, $-NH_2$, hydroxy, $C_{1-4}$-alkyloxy, chlorine or bromine;
or
$R^7$ and $R^8$ together with the nitrogen atom form a saturated or unsaturated 5- or 6-membered ring which may contain nitrogen or oxygen as further heteroatoms, whilst the heterocycle may be substituted by a branched or unbranched alkyl group having 1 to 4 carbon atoms, preferably methyl, or by a $-(CH_2)_{1-4}$-phenyl group, preferably benzyl,
optionally in the form of their racemates, their enantiomers, their diastereomers and mixtures thereof, and optionally the pharmacologically acceptable acid addition salts thereof.

**4.** Compounds of general formula (I) according to one of claims 1 to 3, wherein
$R^1$ denotes hydrogen, $C_{1-4}$-alkyl, which may optionally be substituted by -NHCO-$C_{1-4}$-alkyl, $-NR^7R^8$, hydroxy, $C_{1-4}$-alkyloxy, fluorine, chlorine or bromine, -CHO, -COOH, -COO-$C_{1-4}$-alkyl, phenyl, phenyl-$C_{1-3}$-alkyl, preferably benzyl, which may optionally be substituted by $C_{1-4}$-alkyl, $C_{1-4}$-alkyloxy, hydroxy, benzyloxy or fluorine, phenyloxy-$C_{1-3}$-alkyl, preferably phenyloxymethyl, which may optionally be substituted by methoxy, benzyloxy-$C_{1-3}$-alkyl, preferably benzyloxymethyl, which may optionally be substituted by methoxy, benzoyloxymethyl, pyridylcarbonyloxymethyl, cyclohexylmethyl, pyridylmethyl, pyrrolylmethyl, morpholinomethyl, cyclopentyl or furyl;
$R^2$ or $R^3$ denotes $C_{1-5}$-alkyl, which may optionally be substituted by hydroxy, methoxy or cyclopropyl, $C_{2-4}$-alkenyl, $C_{2-4}$-alkynyl or benzyl;
$R^4$ or $R^6$ denotes hydrogen, $C_{1-3}$-alkyl, $C_{1-3}$-alkyl-$NR^7R^8$, N-morpholinoethyl or benzyl;
$R^5$ denotes hydrogen, $C_{1-4}$-alkyl, which may optionally be fluorine-substituted, phenyl, benzyl, wherein the phenyl ring may optionally be fluorine-substituted, pyridyl, piperidinyl, morpholinyl, piperazinyl, 4-benzylpiperazinyl, furyl, tetrahydrofuranyl, tetrahydropyranyl, $NR^7R^8$, cyclopentyl, cyclohexyl, adamantyl, noradamantyl, norbornyl or norbornenyl;
$R^7$ denotes hydrogen, $C_{1-4}$-alkyl or pyridyl;
$R^8$ denotes hydrogen, $C_{1-4}$-alkyl or pyridyl,
optionally in the form of their racemates, their enantiomers, their diastereomers and mixtures thereof, and optionally the pharmacologically acceptable acid addition salts thereof.

**5.** Compounds of general formula (I) according to one of claims 1 to 4, wherein
$R^1$ denotes hydrogen, methyl, which may optionally be substituted by $-NH_2$, -NHMe, $-NMe_2$, $-N(i\text{-propyl})_2$, -NHAcetyl, hydroxy, methoxy, ethoxy, phenyloxy, methoxyphenyloxy, methoxybenzyloxy, morpholine, benzoyloxy, pyridylcarbonyloxy, pyridine, pyridylamino, methylpiperazine or pyrrole, ethyl which may optionally be substituted by $-NH_2$ or hydroxy, n-propyl, isopropyl, n-butyl, tert. butyl, cyclopentyl, cyclohexylmethyl, benzyl which may optionally be substituted by hydroxy, methoxy, benzyloxy, dimethylaminoethoxy, N-morpholinoethoxy or fluorine, phenyl, phenylethyl, -COOH, -COOMethyl, -CooPropyl or -COOButyl;
$R^2$ or $R^3$ denotes methyl, which may optionally be substituted by cyclopropyl, ethyl which may optionally be substituted by hydroxy or methoxy, n-propyl, isopropyl, n-butyl, isobutyl, tert. butyl, butenyl, n-pentyl, allyl or propargyl; $R^4$ or $R^6$ denotes hydrogen, methyl or ethyl, which may optionally be substituted by morpholine; $R^5$ denotes hydrogen, methyl, ethyl, n-propyl, isopropyl, n-butyl, tert. butyl, phenyl, benzyl, pyridine, piperidine, morpholine, piperazine, 4-benzylpiperazinyl, tetrahydrofuranyl, tetrahydropyranyl, $-NMe_2$, cyclopentyl, cyclohexyl, adamantyl, noradamantyl, norbornanyl or norbornenyl, optionally in the form of their racemates, their enantiomers, their diastereomers and mixtures thereof, and optionally the pharmacologically acceptable acid addition salts thereof.

**6.** Compounds of general formula (I) according to one of claims 1 to 5, wherein $R^1$ denotes methyl which may optionally be substituted by $-NH_2$, -NHMe, -N(i-propyl)$_2$, -NHAcetyl, hydroxy, phenyloxy, methylpiperazine or pyrrole, ethyl which may optionally be substituted by $-NH_2$ or hydroxy, cyclopentyl, benzyl which may optionally be substituted by hydroxy, methoxy, benzyloxy, dimethylaminoethoxy, N-morpholinoethoxy or fluorine, phenylethyl, -COOH, -COOPropyl or -COOButyl; $R^2$ or $R^3$ denotes methyl which may optionally be substituted by cyclopropyl, ethyl which may optionally be substituted by hydroxy or methoxy, n-propyl, isopropyl, n-butyl, isobutyl, butenyl, n-pentyl, allyl or propargyl; $R^4$ or $R^6$ denotes hydrogen, methyl or ethyl, which may optionally be substituted by morpholine; $R^5$ denotes methyl, ethyl, n-propyl, tert. butyl, cyclopentyl or norbornenyl, optionally in the form of their racemates, their enantiomers, their diastereomers and mixtures thereof, and optionally the pharmacologically acceptable acid addition salts thereof.

**7.** Compounds of general formula (I) according to one of claims 1 to 6, wherein $R^1$ denotes methyl, which may optionally be substituted by phenyloxy or pyrrole; benzyl which may optionally be substituted by hydroxy, methoxy, dimethylaminoethoxy or fluorine, or cyclopentyl or phenylethyl; $R^2$ or $R^3$ denotes ethyl, n-propyl, allyl or propargyl; $R^4$ or $R^6$ denotes hydrogen; $R^5$ denotes methyl, ethyl, n-propyl, tert. butyl, cyclopentyl or norbornenyl, optionally in the form of their racemates, their enantiomers, their diastereomers and mixtures thereof, and optionally the pharmacologically acceptable acid addition salts thereof.

**8.** Compounds of general formula (I) according to one of claims 1 to 7, **characterised in that** they occur as isomers of formulae (Ia) or (Ib)

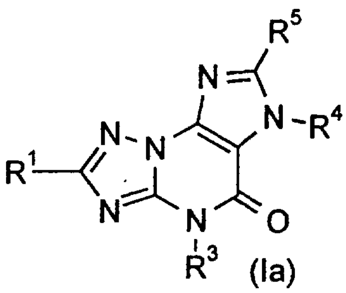
(Ia)

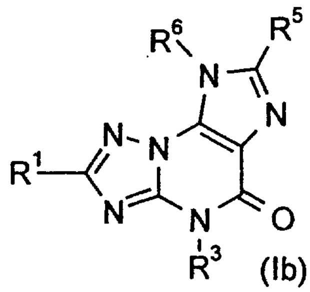
(Ib)

**9.** Compounds of general formula (I) according to one of claims 1 to 8, **characterised in that** they occur as isomers of formula (Ib)

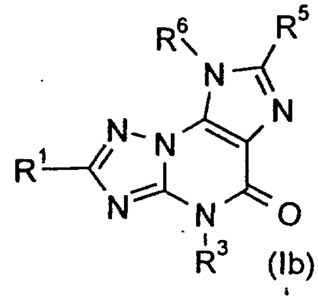

(Ib)

**10.** Compounds of general formula (I) according to one of claims 1 to 8, **characterised in that** they occur as isomers of formula (Ia)

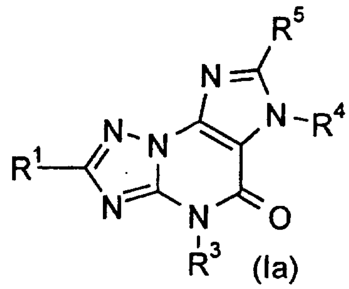

(Ia)

**11.** Compound of general formula (I) according to one of claims 1 to 7, selected from among:

2-Cyclopentyl-4,7-di-n-propyl-imidazo[4.5-e]-s-triazolo[1.5-a]pyrimidin-5-one;
2-Benzyl-7-methyl-4-n-propyl-imidazo[4.5-e]-s-triazolo[1.5-a]pyrimidin-5-one;
4-Ethyl-7-methyl-2-(2-phenylethyl)-imidazo[4.5-e]-5-triazolo[1.5-a]pyrimidin-5-one;
4-Ethyl-2-(2-phenylethyl)-7-n-propyl-imidazo[4.5-e]-s-triazolo[1.5-a]pyrimidin-5-one;
7-t-Butyl-4-ethyl-2-(4-hydroxybenzyl)-imidazo[4.5-e]-s-triazolo[1.5-a]pyrimidin-5-one;
7-t-Butyl-4-ethyl-2-(4-methoxybenzyl)-imidazo[4.5-e]-s-triazolo[1.5-a]pyrimidin-5-one;
7-Cyclopentyl-4-ethyl-2-phenyloxymethyl-imidazo[4.5-e]-s-triazolo[1.5-a]pyrimidin-5-one;
7-Cyclopentyl-4-ethyl-2-(N-pyrrolylmethyl)-imidazo[4.5-e]-s-triazolo[1.5-a]pyrimidin-5-one;
2-(2-Furanyl)-7-methyl-4-n-propyl-imidazo[4.5-e]-s-triazolo[1.5-a]pyrimidin-5-one;
2-(2-Furanyl)-4,7-di-n-propyl-imidazo[4.5-e]-s-triazolo[1.5-a]pyrimidin-5-one;
2-Cyclopentyl-7-ethyl-4-(1-propen-3-yl)-imidazo [4.5-e]-s-triazolo[1.5-a]pyrimidin-5-one or
2-Cyclopentyl-7-ethyl-4-(1-propyn-3-yl)-imidazo [4.5-e]-s-triazolo[1.5-a]pyrimidin-5-one,

optionally in the form of their racemates, their enantiomers, their diastereomers and mixtures thereof, and optionally the pharmacologically acceptable acid addition salts thereof.

**12.** Use of a compound according to one of claims 1 to 11 for preparing a pharmaceutical composition with an adenosine-antagonistic activity.

**13.** Pharmaceutical preparations containing as active substance one or more compounds according to one of claims 1 to 11 or the physiologically acceptable acid addition salts thereof in conjunction with conventional excipients and/or carriers.

## Revendications

**1.** Composés de formule générale (I) :

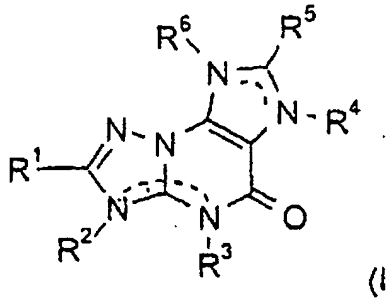

(I)

dans laquelle les lignes pointillées entre les atomes d'azote de la formule générale I indiquée ci-dessus, décrivent l'existence d'une double liaison en l'une des deux positions possibles, de sorte que les restes $R^4$ et $R^6$ et respectivement, $R^3$ et $R^2$ ne peuvent pas être présents simultanément, et

dans laquelle

$R^1$ peut représenter hydrogène, alkyle en $C_1$-$C_8$, alcényle en $C_2$-$C_8$ ou alcynyle en $C_2$-$C_8$, qui peuvent être le cas échéant, substitués une ou plusieurs fois par -CN, $-CH_2NR^7R^8$, $-COOR^9$, $-CONR^7R^8$, -CHO, $-COR^{10}$, -CH(OH) $R^{10}$, $-CH(OR^9)_2$, $-CH=CHR^{11}$, -CH=NOH, $-CH=NOR^9$, $-NR^7R^8$, $-NHCOR^9$, $-NHCONR^7R^8$, $-NHCOOR^9$, -NHCON-Hphényle, $-OR^9$, $-OCOR^9$, -OCOpyridyle, $-OCH_2COOR^9$, $-OCH_2-CONR^7R^8$, $-OCH_2-CH_2-NR^7R^8$, $-OCH_2CH_2OR^9$, $-OCH_2CH_2OCOR^9$, $OCONR^7R^8$, halogène, 1,3-dioxolanne ou 1,3-dioxanne le cas échéant substitués par méthyle, ou

$R^1$ peut représenter -CHO, $-COOR^9$, $-CONR^7R^8$, ou $NR^7R^8$, ou

$R^1$ peut représenter cycloalkyle en $C_3$-$C_7$, de préférence cyclopentyle ou cyclohexyle, qui peut être le cas échéant, substitué par =O, $-OR^9$, $-OCOR^9$ ou -OCOpyridyle,

ou

$R^1$ peut représenter phényle, qui peut être substitué le cas échéant, par alkyle en $C_1$-$C_4$, de préférence méthyle, -CN, $-COOR^9$, $-NR^7R^8$, $-OR^9$, $-OCH_2COOR^9$, $-OCH_2-CONR^7R^8$, $-SO_3H$, ou halogène, ou

$R^1$ peut représenter phényl(alkyle en $C_1$-$C_6$), de préférence phényl(alkyle en $C_1$-$C_4$), phényl(alcényle en $C_2$-$C_6$) ou phényl(alcynle en $C_2$-$C_6$), le cycle phényle pouvant être substitué le cas échéant, soit directement, soit par un pont alkylène ayant de 1 à 4 atomes C, par un ou plusieurs des radicaux alkyle en $C_1$-$C_3$, -CN, $-CH_2OCOR^9$, $-COOR^9$, $-CF_3$, $-CONR^7R^8$, $-CH_2OR^9$, -CHO, $-CH=NOR^9$, $-COR^{10}$, $-CH(OH)R^{10}$, $-CH(OR^9)_2$, $-CH=CH-R^{11}$, $-CH_2-OCONR^7R^8$, $-CH_2-CH_2-OCONR^7R^8$, $-COR^9$, -CO-(alkyl en $C_1$-$C_4$)$NR^7R^8$, $NR^7R^8$, $-NO_2$, $-NHCOR^9$, $-NHCONR^7R^8$, $-NHCOOR^9$, $-OR^9$, $-OCOR^9$, -OCOpyridyle, $-OCH_2COOR^9$, $-OCH_2-CONR^7R^8$, $-OCH_2-CH_2-NR^7R^8$, $-OCH_2-CH_2-OR^9$, $-OCH_2-CH_2-OCOR^9$, $-OCONR^7R^8$, halogène, 1,3-dioxolanne ou 1,3-dioxanne le cas échéant substitués par méthyle, ou

$R^1$ peut représenter (cycloalkyle en $C_3$-$C_7$)alkyle en $C_1$-$C_6$, (cycloalkyle en $C_3$-$C_7$)alcényle en $C_2$-$C_8$, (cycloalkyle en $C_3$-$C_7$)alcynyle en $C_2$-$C_8$, où le reste cycloalkyle peut être le cas échéant, substitué de manière directe ou par un pont alkylène ayant 1 à 4 atomes C, par un ou plusieurs des restes alkyle en $C_1$-$C_3$, -CN, $-OCH_2OCOR^9$, $-COOR^9$, $-CF_3$, $-CONR^7R^8$, $-CH_2OR^9$, -CHO, $-CH=NOR^9$, $-COR^{10}$, $-CH(OH)R^{10}$, $-CH(OR^9)_2$, $-CH=CHR^{11}$, $-CH_2-OCONR^7R^8$, $-CH_2-CH_2OCONR^7R^8$, $-COR^9$, -CO-(alkyl en $C_1$-$C_4$)$NR^7R^8$, $-NR^7R^8$, $-NO_2$, $-NHCOR^9$, $-NHCONR^7R^8$, $-NHCOOR^9$, $-OR^9$, $-OCOR^9$, -OCOpyridyle, $-OCH_2COOR^9$, $-OCH_2-CONR^7R^8$, $-OCH_2-CH_2-NR^7R^8$, $-OCH_2CH_2OR^9$, $-OCH_2CH_2OCOR^9$, $-OCONR^7R^8$, halogène, 1,3-dioxolanne ou 1,3-dioxanne le cas échéant substitué par un méthyle, ou

$R^1$ peut représenter un reste norbornane, norbornène, di(cycloalkyl en $C_3$-$C_6$)méthyle, de préférence dicylopropylméthyle, adamantane ou noradamantane, qui peut être le cas échéant, substitué par alkyle en $C_1$-$C_4$, de préférence méthyle, ou

$R^1$ peut représenter un reste de formule A-alkyle en $C_1$-$C_6$, A-CONH-alkyle en $C_1$-$C_6$, A-CONH-alcényle en $C_2$-$C_6$, A-CONH-alcynyle en $C_2$-$C_6$, A-NH-CO-alkyle en $C_1$-$C_6$, A-NHCO-alcényle en $C_2$-$C_6$, A-NHCO-alcynyle en $C_2$-$C_6$, A-alcényle en $C_2$-$C_6$, A-alcynyle en $C_2$-$C_6$ ou A, où A est un hétérocycle à 5, 6 ou 7 membres lié en C ou N, qui contient un ou plusieurs hétéroatomes du groupe de l'azote, de l'oxygène ou du soufre et le cas échéant, peut être substitué une ou plusieurs fois, de préférence une fois, par benzyle, le cas échéant benzyle substitué par méthoxy, alkyle en $C_1$-$C_4$, -CN, $-CH_2NR^7R^8$, $-COOR^9$, $-CONR^7R^8$, $-COR^{10}$, $-NO_2$, $-NH_2$, $-OR^9$, =O, un cétal, un éthylènecétal, $-SO_3H$, $-SO_2R^9$ ou halogène ;

$R^2$ ou $R^3$ peuvent représenter alkyle en $C_1$-$C_8$, alcényle en $C_2$-$C_8$, alcynyle en $C_2$-$C_8$, qui peuvent être le cas échéant, substitués une ou plusieurs fois par -CN, $-CH_2NR^7R^8$, $-COOR^9$, $-CONR^7R^8$, -CHO, $-COR^{10}$, -CH(OH) $R^{10}$, $-CH(OR^9)_2$, $-CH=CHR^{11}$, -CH=NOH, $-CH=NOR^9$, $-NR^7R^8$, $-NHCOR^9$, $-NHCONR^7R^8$, $-NHCOOR^9$, -NHCON-Hphényle, $-OR^9$, $-OCOR^9$, -OCOpyridyle, $-OCH_2COOR^9$, $-OCH_2-CONR^7R^8$, $-OCH_2-CH_2-NR^7R^8$, $-OCH_2CH_2OR^9$,

$-OCH_2CH_2OCOR^9$, $OCONR^7R^8$, halogène, 1,3-dioxolanne ou 1,3-dioxanne le cas échéant substitué par méthyle, ou

$R^2$ ou $R^3$ peuvent représenter phényl(alkle en $C_1$-$C_6$), de préférence phényl(alkyle en $C_1$-$C_4$), phényl(alcényle en $C_2$-$C_6$) ou phényl(alcynyle en $C_2$-$C_6$), où le cycle phényle peut être substitué le cas échéant, soit directement, soit par un pont alcylène ayant 1 à 4 atomes C, par un ou plusieurs des radicaux alkyle en $C_1$-$C_3$, -CN, $-CH_2OCOR^9$, $-COOR^9$, $-CF_3$, $-CONR^7R^8$, $-CH_2OR^9$, -CHO, $-CH=NOR^9$, $-COR^{10}$, $-CH(OH)R^{10}$, $-CH(OR^9)_2$, $-CH=CH-R^{10}$, $-CH_2-OCONR^7R^8$, $-CH_2-CH_2-OCONR^7R^8$, $-COR^9$, -CO-(alkyl en $C_1$-$C_4$)$NR^7R^8$, $NR^7R^8$, $-NO_2$, $-NHCOR^9$, $-NHCONR^7R^8$, $-NHCOOR^9$, $-OR^9$, $-OCOR^9$, -OCOpyridyle, $-OCH_2COOR^9$, $-OCH_2-CONR^7R^8$, $-OCH_2-CH_2-NR^7R^8$, $-OCH_2-CH_2-OR^9$, $-OCH_2-CH_2-OCOR^9$, $-OCONR^7R^8$, halogène, 1,3-dioxolanne ou 1,3-dioxanne le cas échéant substitué par méthyle, ou

$R^2$ ou $R^3$ peuvent représenter un reste de formule A-alkyle en $C_1$-$C_6$, A-CONH-alkyle en $C_1$-$C_6$, A-CONH-alcényle en $C_2$-$C_6$, A-CONH-alcynyle en $C_2$-$C_6$, A-NH-CO-alkyle en $C_1$-$C_6$, A-NHCO-alcényle en $C_2$-$C_6$, A-NHCO-alcynyle en $C_2$-$C_6$, A-alcényle en $C_2$-$C_6$, A-alcynyle en $C_2$-$C_6$, où A est un hétérocycle à 5, 6 ou 7 membres lié en C ou N, qui contient un ou plusieurs hétéroatomes du groupe de l'azote, de l'oxygène ou du soufre et le cas échéant, peut être substitué une ou plusieurs fois, de préférence une fois, par benzyle, le cas échéant benzyle substitué par méthoxy, alkyle en $C_1$-$C_4$, -CN, $-CH_2NR^7R^8$, $-COOR^9$, $-CONR^7R^8$, $-COR^{10}$, $-NO_2$, $-NH_2$, $-OR^9$, =O, un cétal, un éthylènecétal, $-SO_3H$, $-SO_2R^9$ ou halogène ;

$R^4$ ou $R^6$ peuvent représenter hydrogène, alkyle en $C_1$-$C_4$, qui peut être le cas échéant, substitué par $-NR^7R^8$, benzyle, de préférence hydrogène,

$R^5$ peut représenter hydrogène, un radical alkyle en $C_1$-$C_8$, alcényle en $C_2$-$C_8$, alcynyle en $C_2$-$C_8$, qui peut être le cas échéant, substitué une ou plusieurs fois par -CN, $-CH_2NR^7R^8$, $-COOR^9$, $-CONR^7R^8$, -CHO, $-COR^{10}$, -CH(OH) $R^{10}$, $-CH(OR^9)_2$, $-CH=CHR^{11}$, -CH=NOH, $-CH=NOR^9$, $-NR^7R^8$, $-NHCOR^9$, $-NHCONR^7R^8$, $-NHCOOR^9$, -NHCON-Hphényle, $-OR^9$, $-OCOR^9$, -OCOpyridyle, $-OCH_2COOR^9$, $-OCH_2-CONR^7R^8$, $-OCH_2-CH_2-NR^7R^8$, $-OCH_2CH_2OR^9$, $-OCH_2CH_2OCOR^9$, $OCONR^7R^8$, halogène, 1,3-dioxolanne ou 1,3-dioxanne le cas échéant substitué par méthyle, ou

$R^5$ peut représenter cycloalkyle en $C_3$-$C_7$, de préférence cyclopentyle ou cyclohexyle, qui peut être le cas échéant, substitué par =O, -OH, $-OR^9$, $-OCOR^9$ ou -OCOpyridyle, ou

$R^5$ peut représenter phényle, qui peut être substitué le cas échéant par -OH, halogène, $-OR^9$, alkyle en $C_1$-$C_4$, de préférence $-CH_3$, $-NH_2$, -COOH, $-SO_3H$, $-COOR^9$, $-OCH_2COOR^9$, -CN ou $-OCH_2-CONR^7R^8$, ou

$R^5$ peut représenter phényl(alkyle en $C_1$-$C_6$), de préférence phényl(alkyle en $C_1$-$C_4$), phényl(alcényle en $C_2$-$C_6$) ou phényl(alcynyle en $C_2$-$C_6$), où le cycle phényle peut être substitué le cas échéant, soit directement, soit par un pont alkylène ayant 1 à 4 atomes C, par un ou plusieurs des radicaux alkyle en $C_1$-$C_3$, -CN, $-CH_2OCOR^9$, $-COOR^9$, $-CF_3$, $-CONR^7R^8$, $-CH_2OR^9$, -CHO, $-CH=NOR^9$, $-COR^{10}$, $-CH(OH)R^{10}$, $-CH(OR^9)_2$, $-CH=CH-R^{11}$, $-CH_2-OCONR^7R^8$, $-CH_2-CH_2-OCONR^7R^8$, $-COR^9$, -CO-(alkyl en $C_1$-$C_4$)$NR^7R^8$, $NR^7R^8$, $-NO_2$, $-NHCOR^9$, $-NHCONR^7R^8$, $-NHCOOR^9$, $-OR^9$, $-OCOR^9$, -OCOpyridyle, $-OCH_2COOR^9$, $-OCH_2-CONR^7R^8$, $-OCH_2-CH_2-NR^7R^8$, $-OCH_2-CH_2-OR^9$, $-OCH_2-CH_2-OCOR^9$, $-OCONR^7R^8$, halogène, 1,3-dioxolanne ou 1,3-dioxanne le cas échéant substitué par méthyle, ou

$R^5$ peut représenter un reste norbornane, norbornène, di(cycloalkyl en $C_3$-$C_6$)méthyle, de préférence dicylopropylméthyle, adamantane ou noradamantane, qui peut être le cas échéant, substitué par alkyle en $C_1$-$C_4$, de préférence méthyle, ou

$R^5$ peut représenter -CHO, $-COOR^9$, $-CONR^7R^8$ ou $NR^7R^8$, ou

$R^5$ peut représenter (cycloalkyle en $C_3$-$C_7$)alkyle en $C_1$-$C_6$, (cycloalkyle en $C_3$-$C_7$)alcényle en $C_2$-$C_6$, (cycloalkyle en $C_3$-$C_7$)alcynyle en $C_2$-$C_6$, où le reste cycloalkyle peut être le cas échéant, substitué de manière directe ou par un pont alkylène ayant 1 à 4 atomes C, par un ou plusieurs des restes alkyle en $C_1$-$C_3$, -CN, $-CH_2COOR^9$, $-COOR^9$, $-CF_3$, $-CONR^7R^8$, $-CH_2OR^9$, -CHO, $-CH=NOR^9$, $-COR^{10}$, $-CH(OH)R^{10}$, $-CH(OR^9)_2$, $-CH=CHR^{11}$, $-CH_2-OCONR^7R^8$, $-CH_2-CH_2OCONR^7R^8$, $-COR^9$, -CO-(alkyl en $C_1$-$C_4$)$NR^7R^8$, $-NR^7R^8$, $-NO_2$, $-NHCOR^9$, $-NHCONR^7R^8$, $-NHCOOR^9$, $-OR^9$, $-OCOR^9$, -OCOpyridyle, $-OCH_2COOR^9$, $-OCH_2-CONR^7R^8$, $-OCH_2-CH_2-NR^7R^8$, $-OCH_2CH_2OR^9$, $-OCH_2CH_2OCOR^9$, $-OCONR^7R^8$, halogène, 1,3-dioxolanne ou 1,3-dioxanne le cas échéant substitué par méthyle, ou

$R^5$ peut représenter un reste de formule A-alkyle en $C_1$-$C_6$, A-CONH-alkyle en $C_1$-$C_6$, A-CONH-alcényle en $C_2$-$C_6$, A-CONH-alcynyle en $C_2$-$C_6$, A-NH-CO-alkyle en $C_1$-$C_6$, A-NHCO-alcényle en $C_2$-$C_6$, A-NHCO-alcynyle en $C_2$-$C_6$, A-alcényle en $C_2$-$C_6$, A-alcynyle en $C_2$-$C_6$ ou A, où A est un hétérocycle à 5, 6 ou 7 membres lié en C ou N, qui contient un ou plusieurs hétéroatomes du groupe de l'azote, de l'oxygène ou du soufre et le cas échéant, peut être substitué une ou plusieurs fois, de préférence une fois, par benzyle, le cas échéant benzyle substitué par méthoxy, alkyle en $C_1$-$C_4$, -CN, $-CH_2NR^7R^8$, $-COOR^9$, $-CONR^7R^8$, $-COR^{10}$, $-NO_2$, $-NH_2$, $-OR^9$, =O, un cétal, un éthylènecétal, $-SO_3H$, $-SO_2R^9$ ou halogène ;

$R^7$ peut représenter hydrogène, alkyle en $C_1$-$C_8$, de préférence alkyle en $C_1$-$C_4$, qui peut être substitué le cas échéant, par $-COOR^9$, $-COR^{10}$, $-OR^9$, $-OCOR^9$, amino, phényle, phényle ou amino substitué par méthoxy, ou

cycloalkyle en $C_3$-$C_6$, ou
$R^7$ peut représenter un hétérocycle à 5, 6 ou 7 membres lié en C, directement ou par une chaine alkyle ayant 1 à 4 atomes C, qui contient un, deux ou trois hétéroatomes du groupe de l'azote, de l'oxygène ou du soufre et le cas échéant, peut être substitué une ou plusieurs fois, de préférence une fois, par benzyle, benzyle substitué par méthoxy, alkyle en $C_1$-$C_4$, halogène, -$OR^9$, -CN, -$NO_2$, -$NH_2$, =O, -$SO_3H$ ou -$COOR^9$ ;
$R^8$ peut représenter hydrogène, alkyle en $C_1$-$C_8$, de préférence alkyle en $C_1$-$C_4$, qui peut être substitué le cas échéant, par -$COOR^9$, -$COR^{10}$, -$OR^9$, -$OCOR^9$, amino, phényle, phényle ou amino substitué par méthoxy, ou un cycloalkyle en $C_3$-$C_6$, ou
$R^8$ peut représenter un hétérocycle à 5, 6 ou 7 membres lié en C, directement ou par une chaine alkyle ayant 1 à 4 atomes C, qui contient un, deux ou trois hétéroatomes du groupe de l'azote, de l'oxygène ou du soufre et le cas échéant, peut être substitué une ou plusieurs fois, de préférence une fois, par benzyle, benzyle substitué par méthoxy, alkyle en $C_1$-$C_4$, halogène, -$OR^9$, -CN, -$NO_2$, -$NH_2$, =O,-$SO_3H$ ou -$COOR^9$, ou
$R^7$ et $R^8$ forment ensemble avec l'atome d'azote, un cycle à 5 ou 6 membres, saturé ou insaturé, qui peut contenir comme autres hétéroatomes, un azote, un oxygène ou un soufre, où l'hétérocycle peut être substitué par un radical alkyle linéaire ou ramifié ayant 1 à 4 atomes de carbone, de préférence méthyle, ou peut porter un des restes -CN, -$COOR^9$, -$CONH_2$, -$NO_2$, -$NH_2$, -$OR^9$, -$SO_3H$, -$SO_2R^9$, halogène ou -$(CH_2)_n$-phényle, -$(CH_2)_n$-$NH_2$, =O, -O-$CH_2$-$CH_2$-O-, -O-$CH_2$-$CH_2$-$CH_2$-O-, -$(CH_2)_n$-NH-(alkyle en $C_1$-$C_4$), -$(CH_2)_n$-N(alkyle en $C_1$-$C_4$)$_2$, -$(CH_2)_n$-$NHCOOR^9$, où n = 1, 2, 3 ou 4 ;
$R^9$ peut représenter hydrogène, alkyle en $C_1$-$C_4$, alcényle en $C_2$-$C_4$, alcynyle en $C_2$-$C_4$, un reste benzyle ou phényle, qui peut être le cas échéant, une ou plusieurs fois, substitué par $OCH_3$ ;
$R^{10}$ peut représenter alkyle en $C_1$-$C_4$, alcényle en $C_2$-$C_4$, alcynyle en $C_2$-$C_4$, phényle le cas échéant substitué par méthoxy, benzyle le cas échéant substitué par méthoxy, cycloalkyle en $C_3$-$C_6$ ;
$R^{11}$ peut représenter hydrogène, alkyle en $C_1$-$C_3$, -$COOR^9$, -$CH_2OR^9$, -$CH_2NR^7R^8$, -$CONR^7R^8$, ou phényle le cas échéant substitué par méthoxy,
le cas échéant sous la forme de leurs racémates, de leurs énantiomères, de leurs diastéréoisomères et de leurs mélanges, ainsi que le cas échéant, leurs sels d'addition d'acide pharmacologiquement sans inconvénient.

**2.** Composés de formule générale (I), selon la revendication 1,
où
$R^1$ peut représenter hydrogène, alkyle en $C_1$-$C_8$, qui peut être le cas échéant, substitué par -COOH, -COO-alkyle en $C_1$-$C_4$, -COO-phényle, -COO-benzyle, -$CONR^7R^8$, -CO-alkyle en $C_1$-$C_4$, -CHO, $NR^7R^8$, -NHCO-alkyle en $C_1$-$C_4$, -NHCO-phényle, hydroxy, alkyloxy en $C_1$-$C_4$, phényloxy, -O-phénylméthoxy, benzyloxy, -O-benzylméthoxy, -OCO-alkyle en $C_1$-$C_4$, -OCO-phényle, -OCO-benzyle, -OCO-pyridyle, -O-alcényle en $C_2$-$C_4$, ou halogène, ou
$R^1$ peut représenter -CHO, -COOH, -COO-alkyle en $C_1$-$C_4$, -COO-phényle, -COO-benzyle, -$CONR^7R^8$, ou une amine de formule générale $NR^7R^8$, ou
$R^1$ peut représenter phényle, qui peut être substitué le cas échéant, par alkyle en $C_1$-$C_4$, -CH=NOH, -COOH, -COO-alkyle en $C_1$-$C_4$, -COO-phényle, -COO-benzyle, $NR^7R^8$, hydroxy, alcoxy en $C_1$-$C_4$, benzyloxy, phényloxy, -OCO-alkyle en $C_1$-$C_4$, -OCO-phényle, -OCO-benzyle, -OCO-pyridyle, -O-alcényle en $C_2$-$C_4$, ou halogène; de préférence chlore ou fluor, ou
$R^1$ peut représenter un reste cycloalkyle en $C_3$-$C_6$ ou (cycloalkyl en $C_3$-$C_6$)alkyle en $C_1$-$C_4$, qui peut être, le cas échéant, substitué par hydroxy, alkyle en $C_1$-$C_3$ ou alkyloxy en $C_1$-$C_3$, ou
$R^1$ peut représenter un reste norbornane, norbornène, adamantane ou noradamantane, qui peut être le cas échéant, substitué par alkyle en $C_1$-$C_4$, de préférence méthyle, ou
$R^1$ peut représenter phényl(alkyle en $C_1$-$C_4$), de préférence benzyle, phényl(alcényle en $C_2$-$C_6$) ou phényl(alcynyle en $C_2$-$C_6$), où le cycle phényle peut être substitué le cas échéant, par alkyle en $C_1$-$C_3$, -CO-alkyle en $C_1$-$C_4$, (alkyl en $C_1$-$C_4$)$NR^7R^8$, -COOH, -COO(alkyle en $C_1$-$C_4$), -COO-phényle, -COO-benzyle, -$CONR^7R^8$, -CO-(alkyl en $C_1$-$C_4$)$NR^7R^8$, $NR^7R^8$, hydroxy, (alkyloxy en $C_1$-$C_4$), benzyloxy, phényloxy, -OCO(alkyle en $C_1$-$C_4$), -OCO-phényle, -OCO-benzyle, -OCO-pyridyle, -O-alcényle en $C_2$-$C_4$ ou halogène, de préférence chlore ou fluor, ou
$R^1$ peut représenter un hétérocycle à 5 ou 6 membres lié en C ou N, directement ou par un pont alkylène ayant 1 à 4 atomes C, qui contient un, deux ou trois hétéroatomes du groupe de l'azote, de l'oxygène ou du soufre et le cas échéant, peut être substitué une ou plusieurs fois, par benzyle ou alkyle en $C_1$-$C_4$ ;
$R^2$ ou $R^3$ peuvent représenter un radical alkyle en $C_1$-$C_8$, qui peut être le cas échéant, substitué par -$NR^7R^8$, OH, alkyloxy en $C_1$-$C_4$, ou COOH, ou alcényle en $C_2$-$C_8$, phényl-alkyle en $C_1$-$C_4$, de préférence benzyle, phényl-alcényle en $C_2$-$C_6$ ou phényl-alcynyle en $C_2$-$C_6$, où le cycle phényle peut être substitué le cas échéant, par hydroxy, alkyle en $C_1$-$C_3$, alkyloxy en $C_1$-$C_4$, $NR^7R^8$ ou halogène, de préférence chlore ou fluor, ou
$R^2$ ou $R^3$ peuvent représenter un hétérocycle à 5 ou 6 membres lié en C ou N, directement ou par un pont alkylène ayant 1 à 4 atomes C, qui contient un, deux ou trois hétéroatomes du groupe de l'azote, de l'oxygène ou du soufre et le cas échéant, peut être substitué par benzyle, ou alkyle en $C_1$-$C_4$ ;

$R^4$ ou $R^6$ peuvent représenter hydrogène, alkyle en $C_1$-$C_4$, qui peut être le cas échéant, substitué par -$NR^7R^8$, benzyle ;
$R^5$ peut représenter hydrogène, alkyle en $C_1$-$C_8$, de préférence alkyle en $C_1$-$C_6$, qui peut être le cas échéant, substitué par halogène, ou
$R^5$ peut représenter phényle, qui peut être substitué le cas échéant, par alkyle en $C_1$-$C_4$, alcoxy en $C_1$-$C_4$, hydroxy ou halogène, de préférence fluor ou chlore, ou
$R^5$ peut représenter phényl(alkyle en $C_1$-$C_6$), de préférence benzyle, où le cycle phényle peut être substitué le cas échéant, par alkyle en $C_1$-$C_3$, alkyloxy en $C_1$-$C_4$, hydroxy, $NR^7R^8$ ou halogène, de préférence fluor ou chlore, ou
$R^5$ peut représenter une amine de formule $NR^7R^8$, ou
$R^5$ peut représenter un hétérocycle à 5 ou 6 membres lié en C ou N, directement ou par un pont alkylène ayant 1 à 4 atomes C, qui contient un ou plusieurs hétéroatomes du groupe de l'azote ou de l'oxygène et le cas échéant, peut être substitué par benzyle, ou alkyle en $C_1$-$C_4$ ;
$R^5$ peut représenter cycloalkyle en $C_3$-$C_6$, qui peut être le cas échéant, substitué par =O, hydroxy ou alkyloxy en $C_1$-$C_4$, ou
$R^5$ peut représenter norbornane, norbornène, adamantane ou noradamantane, le cas échéant substitué par alkyle en $C_1$-$C_4$, de préférence méthyle ;
$R^7$ peut représenter hydrogène, un radical alkyle linéaire ou ramifié ayant 1 à 4 atomes de carbone, ou
$R^7$ peut représenter un hétérocycle à 5 ou 6 membres lié en C, qui contient un, deux ou trois hétéroatomes du groupe de l'azote, de l'oxygène ou du soufre et le cas échéant, peut être substitué par benzyle, alkyle en $C_1$-$C_4$, alkyloxy en $C_1$-$C_4$, halogène, -CN, -$NO_2$, -$NH_2$, -OH ou =O ;
$R^8$ peut représenter hydrogène, un radical alkyle linéaire ou ramifié ayant 1 à 4 atomes de carbone, ou
$R^8$ peut représenter un hétérocyclé à 5 ou 6 membres lié en C, qui contient un, deux ou trois hétéroatomes du groupe de l'azote, de l'oxygène ou du soufre et le cas échéant, peut être substitué par benzyle, alkyle en $C_1$-$C_4$, alkyloxy en $C_1$-$C_4$, halogène, -CN, -$NO_2$, -$NH_2$, -OH ou =O, ou
$R^7$ et $R^8$ forment ensemble avec l'atome d'azote, un cycle à 5 ou 6 membres, saturé ou insaturé, qui peut contenir comme autres hétéroatomes, un azote ou un oxygène, où l'hétérocyle peut être substitué par un radical alkyle linéaire ou ramifié ayant 1 à 4 atomes de carbone, de préférence méthyle, ou par un reste -$(CH_2)_{1-4}$-phényle, de préférence benzyle,
le cas échéant sous la forme de leurs racémates, de leurs énantiomères, de leurs diastéréoisomères et de leurs mélanges, ainsi que le cas échéant, leurs sels d'addition d'acide pharmacologiquement sans inconvénient.

**3.** Composés de formule générale (I), selon la revendication 1 ou 2,
où
$R^1$ peut représenter hydrogène, alkyle en $C_1$-$C_6$, de préférence alkyle en $C_1$-$C_4$, qui peut être le cas échéant, substitué par -CO-alkyle en $C_1$-$C_4$, -CHO, -COOH, -COO-alkyle en $C_1$-$C_4$, -COO-phényle, -COO-benzyle, -$CONR^7R^8$, $NR^7R^8$, -NHCO-alkyle en $C_1$-$C_4$, -NHCO-phényle, hydroxy, =O, alkyloxy en $C_1$-$C_4$, phényloxy, -O-phényl-O-méthoxy, benzyloxy, -O-benzyl-O-méthoxy, -OCO-alkyle en $C_1$-$C_4$, -OCO-phényle, -OCO-pyridyle, -OCO-benzyle, -O-alcényle en $C_2$-$C_4$, ou halogène, ou
$R^1$ peut représenter phényle, où le cycle phényle peut être substitué par alkyle en $C_1$-$C_4$, hydroxy, alcoxy en $C_1$-$C_4$, $NR^7R^8$, halogène, de préférence chlore ou fluor, ou
$R^1$ peut représenter phényl(alkyle en $C_1$-$C_3$), de préférence benzyle, où le cycle phényle peut être substitué par alkyle en $C_1$-$C_3$, -CO-alkyle en $C_1$-$C_4$, (alkyl en $C_1$-$C_4$)$NR^7R^8$, -COOH, -COO(alkyle en $C_1$-$C_4$), -COO-phényle, -COO-benzyle, -$CONR^7R^8$, -CO-(alkyl en $C_1$-$C_4$)$NR^7R^8$, $NR^7R^8$, hydroxy, (alkyloxy en $C_1$-$C_4$), benzyloxy, phényloxy, -OCO(alkyle en $C_1$-$C_4$), -OCO-phényle, -OCO-benzyle, -OCO-pyridyle, -O-alcényle en $C_2$-$C_4$ ou halogène, de préférence chlore ou fluor, ou
$R^1$ peut représenter cyclopentyle, cyclohexyle, hydroxycyclopentane ou hydroxycyclohexane, relié par une simple liaison ou par une chaine alkylène ayant 1 à 4 atomes C, ou
$R^1$ peut représenter -CHO, -COOH, -COO(alkyle en $C_1$-$C_4$), -COOphényle, -COObenzyle, -CONH(alkyle en $C_1$-$C_4$), -CON(alkyle en $C_1$-$C_4$)$_2$, ou -CONHphényle, ou
$R^1$ peut représenter une amine de formule $NR^7R^8$, ou
$R^1$ peut représenter un furanne, tétrahydrofuranne, α-pyranne, γ-pyranne, dioxolanne, tétrahydropyranne, dioxanne, thiophène, thiolane, dithiolane, pyrrole, pyrroline, pyrrolidine, pyrazole, pyrazoline, imidazole, imidazoline, imidazolidine, triazole, pyridine, pipéridine, pyridazine, pyrimidine, pyrazine, pipérazine, triazine, morpholine, thiomorpholine, oxazole, isoxazole, oxazine, thiazole, isothiazole, thiadiazole, oxadiazole ou pyrazolidine, lié par une simple liaison ou par une chaine alkylène ayant 1 à 4 atomes C ;
$R^2$ ou $R^3$ peuvent représenter un radical alkyle en $C_1$-$C_7$, de préférence alkyle en $C_1$-$C_5$, qui peut être le cas échéant, substitué par -$NR^7R^8$, OH, alkyloxy en $C_1$-$C_4$, ou COOH, alcényle en $C_2$-$C_5$, alcynyle en $C_2$-$C_5$, phénylalkyle en $C_1$-$C_4$, de préférence benzyle, où le cycle phényle peut être substitué par alkyle en $C_1$-$C_3$, $NR^7R^8$, hy-

droxy, alkyloxy en $C_1$-$C_4$ ou halogène, de préférence chlore ou fluor, ou

$R^2$ ou $R^3$ peuvent représenter un furanne, tétrahydrofuranne, $\alpha$-pyranne, $\gamma$-pyranne, dioxolanne, tétrahydropyranne, dioxanne, thiophène, thiolane, dithiolane, pyrrole, pyrroline, pyrrolidine, pyrazole, pyrazoline, imidazole, imidazoline, imidazolidine, triazole, pyridine, pipéridine, pyridazine, pyrimidine, pyrazine, pipérazine, triazine, morpholine, thiomorpholine, oxazole, isoxazole, oxazine, thiazole, isothiazole, thiadiazole, oxadiazole ou pyrazolidine, lié par une simple liaison ou par une chaine alkylène ayant 1 à 4 atomes C ;

$R^4$ ou $R^6$ peuvent représenter hydrogène, alkyle en $C_1$-$C_4$, de préférence alkyle en $C_1$-$C_3$, (alkyle en $C_1$-$C_4$)$NR^7R^8$, benzyle ;

$R^5$ peut représenter hydrogène, alkyle en $C_1$-$C_6$, de préférence alkyle en $C_1$-$C_4$, qui peut être le cas échéant, substitué par halogène, ou

$R^5$ peut représenter cyclopentyle, cyclohexyle, cyclopentanone, cyclohexanone, hydroxycyclopentane ou hydroxycyclohexane, ou

$R^5$ peut représenter pyridyle, tétrahydrofurannyle, tétrahydropyrannyle, furyle ou un reste morpholine, pipéridine ou pipérazine, le cas échéant substitué par alkyle en $C_1$-$C_4$ ou benzyle, ou

$R^5$ peut représenter un reste phényle, qui peut être substitué le cas échéant, par alkyle en $C_1$-$C_4$, halogène ou hydroxy, ou

$R^5$ peut représenter phényl(alkyle en $C_1$-$C_3$), de préférence benzyle, où le cycle phényle peut être substitué le cas échéant, par alkyle en $C_1$-$C_3$, $NR^7R^8$, hydroxy, alkyloxy en $C_1$-$C_4$, ou halogène, de préférence fluor ou chlore, ou

$R^5$ peut représenter une amine de formule $NR^7R^8$, ou

$R^5$ peut représenter un reste norbornane, norbornène, adamantane ou noradamantane, qui peut être le cas échéant, substitué par alkyle en $C_1$-$C_4$, de préférence méthyle ;

$R^7$ représente hydrogène, un radical alkyle linéaire ou ramifié ayant 1 à 4 atomes de carbone, ou

$R^7$ peut représenter un pyrrole, pyrrolidine, pyrazole, imidazole, imidazolidine, triazole, pyridine, pipéridine, pyrimidine, pyrazine, pipérazine, morpholine, oxazole, isoxazole, thiazole, isothiazole ou thiadiazole le cas échéant substitué par alkyle en $C_1$-$C_4$, $-NO_2$, $-NH_2$, hydroxy, alkyloxy en $C_1$-$C_4$, chlore ou brome ;

$R^8$ peut représenter hydrogène, un radical alkyle linéaire ou ramifié ayant 1 à 4 atomes de carbone, ou

$R^8$ peut représenter un pyrrole, pyrrolidine, pyrazole, imidazole, imidazolidine, triazole, pyridine, pipéridine, pyrimidine, pyrazine, pipérazine, morpholine, oxazole, isoxazole, thiazole, isothiazole ou thiadiazole le cas échéant substitué par alkyle en $C_1$-$C_4$, $-NO_2$, $-NH_2$, hydroxy, alkyloxy en $C_1$-$C_4$, chlore ou brome, ou

$R^7$ et $R^8$ forment ensemble avec l'atome d'azote, un cycle à 5 ou 6 membres, saturé ou insaturé, qui peut contenir comme autres hétéroatomes, un azote ou un oxygène, où l'hétérocycle peut être substitué par un radical alkyle linéaire ou ramifié ayant 1 à 4 atomes de carbone, de préférence méthyle, ou par un reste $-(CH_2)_{1-4}$-phényle, de préférence benzyle,

le cas échéant sous la forme de leurs racémates, de leurs énantiomères, de leurs diastéréoisomères et de leurs mélanges, ainsi que le cas échéant, leurs sels d'addition d'acide pharmacologiquement sans inconvénient.

**4.** Composés de formule générale (I), selon l'une des revendications 1 à 3, où

$R^1$ peut représenter hydrogène, alkyle en $C_1$-$C_4$, qui peut être le cas échéant, substitué par -NHCO-alkyle en $C_1$-$C_4$, $NR^7R^8$, hydroxy, alkyloxy en $C_1$-$C_4$, fluor, chlore ou brome, ou -CHO, -COOH, -COO-alkyle en $C_1$-$C_4$, phényle, phénylalcoyle en $C_1$-$C_3$, de préférence benzyle, qui peut être substitué par alkyle en $C_1$-$C_4$, alkyloxy en $C_1$-$C_4$, hydroxy, benzyloxy, ou fluor, ou phényloxyalkyle en $C_1$-$C_3$, de préférence phényloxyméthyle, qui peut être le cas échéant substitué par méthoxy, ou benzyloxyalkyle en $C_1$-$C_3$, de préférence benzyloxyméthyle, qui peut être le cas échéant substitué par méthoxy, ou benzoyloxyméthyle, pyridylcarbonyloxyméthyle, cyclohexylméthyle, pyridylméthyle, pyrrolylméthyle, morpholinométhyle, cyclopentyle ou furyle ;

$R^2$ ou $R^3$ peuvent représenter un radical alkyle en $C_1$-$C_5$, qui peut être le cas échéant, substitué par hydroxy, méthoxy ou cyclopropyle ; alcényle en $C_2$-$C_4$, alcynyle en $C_2$-$C_4$, ou benzyle ;

$R^4$ ou $R^6$ peuvent représenter hydrogène, alkyle en $C_1$-$C_3$, (alkyle en $C_1$-$C_3$)$NR^7R^8$, N-morpholinoéthyle ou benzyle ;

$R^5$ représente hydrogène, alkyle en $C_1$-$C_4$, qui peut être le cas échéant, substitué par fluor, ou phényle, benzyle, où le cycle phényle peut être le cas échéant substitué par fluor, ou pyridyle, pipéridinyle, morpholinyle, pipérazinyle, 4-benzylpipérazinyle, furyle, tétrahydrofurannyle, tétrahydropyrannyle, $NR^7R^8$, cyclopentyle, cyclohexyle, adamantyle, noradamantyle, norbornyle ou norbornényle ;

$R^7$ représente hydrogène, un radical alkyle en $C_1$-$C_4$ ou pyridyle ;

$R^8$ peut représenter hydrogène, un radical alkyle en $C_1$-$C_4$ ou pyridyle,

le cas échéant sous la forme de leurs racémates, de leurs énantiomères, de leurs diastéréoisomères et de leurs mélanges, ainsi que le cas échéant, leurs sels d'addition d'acide pharmacologiquement sans inconvénient.

**5.** Composés de formule générale (I), selon l'une des revendications 1 à 4, où
$R^1$ peut représenter hydrogène, méthyle, qui peut être le cas échéant; substitué par $-NH_2$, -NHMe, $-NMe_2$, -N(i-propyle)$_2$, -NHacétyle, hydroxy, méthoxy, éthoxy, phényloxy, méthoxyphényloxy, méthoxybenzyloxy, morpholine, benzoyloxy, pyridylcarbonyloxy, pyridine, pyridylamino, méthylpipérazine ou pyrrole, ou éthyle qui peut être le cas échéant, substitué par $-NH_2$, ou hydroxy, ou n-propyle, i-propyle, n-butyle, tert.-butyle, cyclopentyle, cyclohexylméthyle, benzyle, qui peut être le cas échéant, substitué par hydroxy, méthoxy, benzyloxy, diméthylaminométhoxy, N-morpholinoéthoxy ou fluor, ou phényle, phényléthyle, -COOH, -COOméthyle, -COOpropyle ou -COObutyle ;
$R^2$ ou $R^3$ peuvent représenter méthyle, qui peut être le cas échéant substitué par cyclopropyle, ou éthyle qui peut être le cas échéant substitué par hydroxy ou méthoxy, ou n-propyle, i-propyle, n-butyle, i-butyle, tert.-butyle, butényle, n-pentényle, allyle ou propargyle ;
$R^4$ ou $R^6$ peuvent représenter hydrogène, méthyle ou éthyle, qui peut être le cas échéant, substitué par morpholine ;
$R^5$ représente hydrogène, méthyle, éthyle, n-propyle, i-propyle, n-butyle, tert.-butyle, phényle, benzyle, pyridine, pipéridine, morpholine, pipérazine, 4-benzylpipérazinyle, tétrahydrofurannyle, tétrahydropyrannyle, $NMe_2$, cyclopentyle, cyclohexyle, adamantyle, noradamantyle, norbornanyle ou norbornényle ;
le cas échéant sous la forme de leurs racémates, de leurs énantiomères, de leurs diastéréoisomères et de leurs mélanges, ainsi que le cas échéant, leurs sels d'addition d'acide pharmacologiquement sans inconvénient.

**6.** Composés de formule générale (I), selon l'une des revendications 1 à 5, où
$R^1$ peut représenter méthyle, qui peut être le cas échéant, substitué par $-NH_2$, -NHMe, $-NMe_2$, -N(i-propyle)$_2$, -NHacétyle, hydroxy, phényloxy, méthylpipérazine ou pyrrole, ou éthyle qui peut être le cas échéant, substitué par $-NH_2$, ou hydroxy,ou cyclopentyle, benzyle, qui peut être le cas échéant, substitué par hydroxy, méthoxy, benzyloxy, diméthylaminoéthoxy, N-morpholinoéthoxy ou fluor, ou phényléthyle, -COOH, -COOpropyle ou -COObutyle ;
$R^2$ ou $R^3$ peuvent représenter méthyle, qui peut être le cas échéant, substitué par cyclopropyle, ou éthyle, qui peut être le cas échéant, substitué par hydroxy ou méthoxy, ou n-propyle, i-propyle, n-butyle, i-butyle, butényle, n-pentyle, allyle ou propargyle ;
$R^4$ ou $R^6$ peuvent représenter hydrogène, méthyle ou éthyle, qui peut être le cas échéant, substitué par morpholine ;
$R^5$ représente méthyle, éthyle, n-propyle, tert.-butyle, cyclopentyle ou norbornényle ;
le cas échéant sous la forme de leurs racémates, de leurs énantiomères, de leurs diastéréoisomères et de leurs mélanges, ainsi que le cas échéant, leurs sels d'addition d'acide pharmacologiquement sans inconvénient.

**7.** Composés de formule générale (I), selon l'une des revendications 1 à 6, où
$R^1$ peut représenter méthyle, qui peut être le cas échéant, substitué par phényloxy ou pyrrole, ou benzyle, qui peut être le cas échéant, substitué par hydroxy, méthoxy, diméthylaminoéthoxy ou fluor, ou cyclopentyle, phényléthyle ;
$R^2$ ou $R^3$ peuvent représenter éthyle, n-propyle, allyle ou propargyle ;
$R^4$ ou $R^6$ peuvent représenter hydrogène ;
$R^5$ peut représenter méthyle, éthyle, n-propyle, tert.-butyle, cyclopentyle ou norbornényle,
le cas échéant sous la forme de leurs racémates, de leurs énantiomères, de leurs diastéréoisomères et de leurs mélanges, ainsi que le cas échéant, leurs sels d'addition d'acide pharmacologiquement sans inconvénient.

**8.** Composés de formule générale (I), selon l'une des revendications 1 à 7, **caractérisés en ce qu'**ils se présentent sous la forme d'isomères des formules (Ia) ou (Ib) :

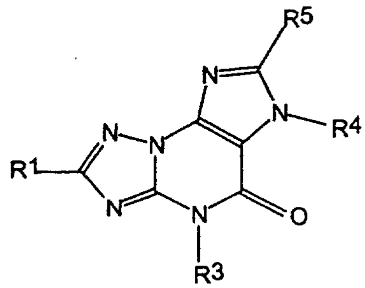

(Ia)

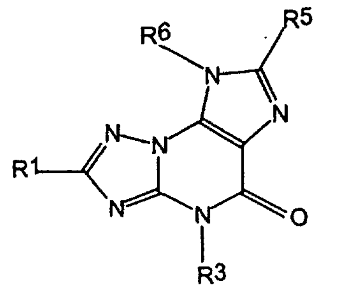

(Ib)

**9.** Composés de formule générale (I), selon l'une des revendications 1 à 8, **caractérisés en ce qu'**ils se présentent sous la forme d'isomères, de formule (Ib) :

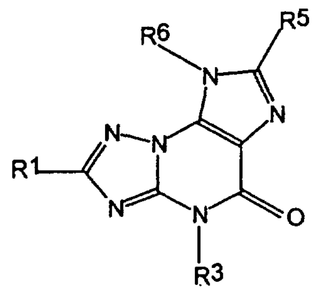

(Ib)

**10.** Composés de formule générale (I), selon l'une des revendications 1 à 8, **caractérisés en ce qu'**ils se présentent sous la forme d'isomères, de formule (Ia) :

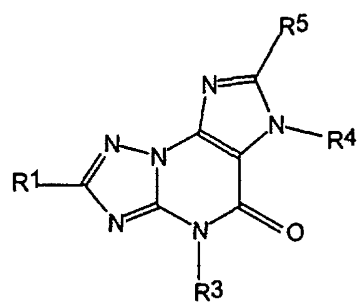

(Ia)

**11.** Composés de formule générale (I), selon l'une des revendications 1 à 7, choisis parmi le groupe constitué de :

la 2-cyclopentyl-4,7-di-n-propylimidazo[4.5-e]-s-triazolo[1.5-a]pyrimidin-5-one ;
la 2-benzyl-7-méthyl-4-n-propylimidazo[4.5-e]-s-triazolo[1.5-a]pyrimidin-5-one ;
la 4-éthyl-7-méthyl-2-(2-phényléthyl)imidazo[4.5-e]-s-triazolo[1.5-a]pyrimidin-5-one ;
la 4-éthyl-2-(2-phényléthyl)-7-n-propylimidazo[4.5-e]-s-triazolo[1.5-a]pyrimidin-5-one ;
la 7-t-butyl-4-éthyl-2-(4-hydroxybenzyl)imidazo[4.5-e]-s-triazolo[1.5-a]pyrimidin-5-one ;

la 7-t-butyl-4-éthyl-2- (4-méthoxybenzyl)imidazo[4.5-e]-s-triazolo[1.5-a]pyrimidin-5-one ;
la 7-cyclopentyl-4-éthyl-2-phénoxyméthylimidazo[4.5-e]-s-triazolo[1.5-a]pyrimidin-5-one ;
la 7-cyclopentyl-4-éthyl-2-(N-pyrrolylméthyl)imidazo[4.5-e]-s-triazolo[1.5-a]pyrimidin-5-one ;
la 2-(2-furannyl)-7-méthyl-4-n-propylimidazo[4.5-e]-s-triazolo[1.5-a]pyrimidin-5-one ;
la 2-(2-furannyl)-4,7-di-n-propylimidazo[4.5-e]-s-triazolo[1.5-a]pyrimidin-5-one ;
la 2-cyclopentyl-7-éthyl-4-(1-propèn-3-yl) imidazo[4.5-e]-s-triazolo[1.5-a]pyrimidin-5-one, ou
la 2-cyclopentyl-7-éthyl-4-(1-propyn-3-yl) imidazo[4.5-e]-s-triazolo[1.5-a]pyrimidin-5-one,

le cas échéant sous la forme de leurs racémates, de leurs énantiomères, de leurs diastéréoisomères et de leurs mélanges, ainsi que le cas échéant, leurs sels d'addition d'acide pharmacologiquement sans inconvénient.

**12.** Utilisation d'un composé selon l'une des revendications 1 à 11, pour la préparation d'un médicament à action antagoniste de l'adénosine.

**13.** Compositions pharmaceutiques, contenant comme substance active, un ou plusieurs composés selon l'une quelconque des revendications 1 à 11 ou leurs sels d'addition d'acide physiologiquement compatibles, en combinaison avec les adjuants et/ou supports usuels.